# EUROPEAN PATENT APPLICATION

(11) **EP 4 105 232 A1**
(43) Date of publication of application: **21.12.2022**
(21) Application number: 21753514.5
(22) Date of filing: 15.02.2021
(51) Int. Cl.: C07K 16/28, C07K 14/705, C07K 14/725, C12N 15/86, C12N 15/85

(54) **IMMUNE CELLS OVEREXPRESSING CELL SIGNALING REGULATORY FACTOR INTRODUCED FROM OUTSIDE AND USE THEREOF**

(30) Priority: 14.02.2020 KR 20200018169
(71) Applicant: Beijing Yongtai Ruike Biotechnology Company Ltd, Beijing 100176 (CN); Pharos Vaccine Inc., Gyeonggi-do 13201 (KR)
(72) Inventor: KIM, Hoeon, Yongin-si Gyeonggi-do 16827 (KR); JEON, Choonju, Yongin-si Gyeonggi-do 16827 (KR); WANG, Yu, Beijing 100086 (CN); JUNG, Namchul, Seongnam-si Gyeonggi-do 13201 (KR)
(74) Representative: HGF
(86) International application number: PCT/KR2021/001904
(87) International publication number: WO 2021/162521

(57) **Abstract**

The present invention relates to an immune cell that are engineered to overexpress cell signaling pathway modulator(s) and a use thereof. As a specific example, an immune cell expressing a fusion protein comprising a chimeric antigen receptor and a cell signaling pathway modulator(s) performs an immune response by selecting a target cancer cell by a chimeric antigen receptor expressed on a cell membrane. In this case, the cell signaling pathway modulator is overexpressed in the cytoplasm, thereby being capable of regulating the activity of an immune cell. Therefore, the fusion protein comprising a chimeric antigen receptor and cell signaling pathway modulator(s), and the immune cell engineered to overexpress the cell signaling pathway modulator(s) of the present invention can be usefully used in the treatment of cancer.

## Description

### Technical Field

The present invention relates to immune cells engineered to overexpress cell signaling pathway modulator(s) and use thereof.

### Background Art

Cancer is the second leading cause of morbidity and mortality worldwide. Cancers with a high morbidity are breast cancer, lung and bronchial cancer, prostate cancer, colorectal cancer, bladder cancer, skin melanoma, non-Hodgkin's lymphoma, thyroid cancer, kidney and renal pelvic cancer, endometrial cancer, leukemia and pancreatic cancer. In order to treat cancer, various methods such as surgical operation, radiation therapy, and chemotherapy have been attempted. However, as several side effects have been reported, an immunotherapy using the immune function of patients has been recently developed. Studies of treatment methods in which a chimeric antigen receptor (CAR) or T cell receptor capable of recognizing specific cancer cells is expressed in immune cells through a viral vector and then transplanted into patients with cancer (CAR-T-based/TCR T-based Adoptive cell therapy) are being actively conducted.

Specifically, a chimeric antigen receptor is composed of a fragment of an antibody, a hinge region, a transmembrane domain, and an intracellular signaling domain. An immune cell such as a T cell (CAR-T cell) in which the chimeric antigen receptor is expressed or a natural killer cell (CAR-NK cell) in which the chimeric antigen receptor is expressed, specifically recognizes a cell (cancer cell and the like) in which the fragment of an antibody recognizes the cells expressing a target molecule to activate cytotoxicity of the immune cell, thereby inducing cell death of a target cell. Therefore, an immune cell in which the chimeric antigen receptor is expressed, is used in genetically engineered cell therapy. In particular, it has been reported that a chimeric antigen receptor T cell exhibits a very high therapeutic effect against a hematologic cancer in which CD19 is expressed.

However, unlike hematologic cancer, in the case of solid cancer, it is not easy for an immune cell such as a CAR-T cell to which genes are introduced to migrate to tumor tissues where cancer cells grow. In addition, even if an immune cell infiltrates into the tumor tissue, an immune response of the CAR-T cell is lowered due to several types of immune checkpoint proteins and various cytokines expressed in cancer cells or cells surrounding cancer cells, and thus therapeutic efficacy is not exhibited in solid cancer so far.

Among them, TGF-β is known as a representative cytokine that suppresses the immune response of CAR-T cells. The signaling pathway of TGF-β is mediated by two types of receptors, TGFβR1 and TGFβR2, which exist on a cell membrane. First, when TGF-β binds to TGFβR2, TGFβR1 is phosphorylated and activated, and an activated TGFβR1 phosphorylates a SMAD protein. The phosphorylated SMAD protein forms a trimer and translocates to the nucleus to induce the expression of a specific gene (Andres Rojas et al., Biochimica et Biophysica Acta 1793 (2009) 1165-1173). Therefore, many studies are being conducted to overcome the problem of suppressing the immune response of CAR-T cells and to produce immune cells with better activity.

### Detailed Description of the Invention

### Technical Problem

Accordingly, the present inventors have studied to develop a method that can not only prevent the mechanism through which the activity of an immune cell into which a gene is introduced is inhibited, but also increase the cellular activity of an immune cell into which a gene is introduced. The experiment was conducted on CAR-T cells, which are representative examples of an immune cell into which a gene is introduced. As a result, a method was designed that can not only prevent the mechanism through which CAR-T cells are inhibited, but also increase the cellular activity of CAR-T cells, and thus the present inventors completed the present invention.

### Solution to Problem

In order to solve the above problems, one aspect of the present invention provides a polynucleotide encoding a fusion protein that includes (i) an antigen binding domain; (ii) a transmembrane domain; (iii) an intracellular signaling domain including at least one co-stimulatory domain; (iv) a self-cleavage peptide; and (v) a signaling pathway modulator(s).

Another aspect of the present invention provides a polynucleotide including (i) a polynucleotide encoding an antigen binding domain; (ii) a polynucleotide encoding a transmembrane domain; (iii) a polynucleotide encoding an intracellular signaling domain including at least one co-stimulatory domain; (iv) a polynucleotide encoding an IRES (Internal Ribosome Entry Site); and (v) a polynucleotide encoding a signaling pathway modulator(s).

Another aspect of the present invention provides a vector including a polynucleotide encoding a fusion protein that includes (i) an antigen binding domain; (ii) a transmembrane domain; (iii) an intracellular signaling domain including at least one co-stimulatory domain; and (iv) a signaling pathway modulator(s).

Another aspect of the present invention provides an immune cell expressing a chimeric antigen receptor, characterized in that the immune cell expresses a fusion protein including (i) an antigen binding domain; (ii) a transmembrane domain; (iii) an intracellular signaling domain including at least one costimulatory domain, and overexpresses a signaling pathway modulator(s) that is externally introduced.

Another aspect of the present invention provides an immune cell expressing a signaling pathway modulator(s) that is externally introduced.

### Effects of the Invention

The immune cell engineered to overexpressing a chimeric antigen receptor and a signaling pathway modulator(s) that is externally introduced of the present invention specifically elicits an immune reaction against specific cancer cells through by the chimeric antigen receptor expressed on the cell membrane. In addition, since a signaling pathway modulator(s) that is externally introduced is overexpressed in the cytoplasm of the immune cell, it is possible to attenuate any specific immune suppressive signaling pathway(s). In addition, it is possible to escalate the overall ability to kill tumor cells by regulating the activity of the immune cell. Therefore, the immune cells engineered to overexpress a chimeric antigen receptor and a signaling pathway modulator(s) that is externally introduced of the present invention can be usefully used in the treatment of cancer.

### Brief Description of Drawings

Figure 1 is a view showing the domain structure of the 19bbz CAR experimental group constructed in one embodiment.
Figure 2 is a view showing the domain structure of the 19bbz CAR experimental group constructed in one embodiment.
Figure 3 is a view showing the domain structure of the Hbbz CAR experimental group constructed in one embodiment.

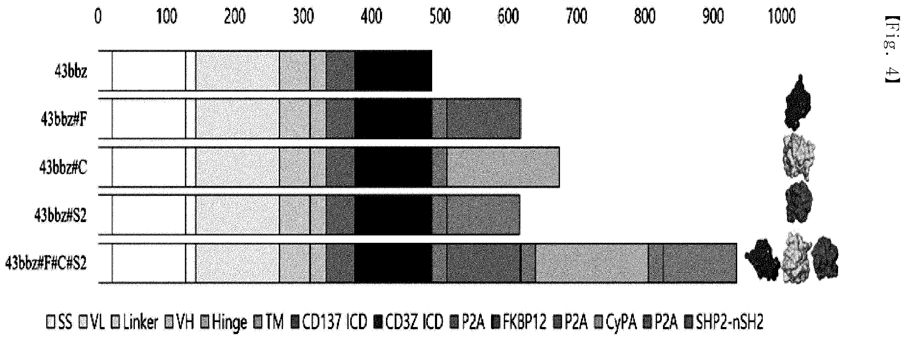

Figure 4 is a view showing the domain structure of the 43bbz CAR experimental group constructed in one embodiment.

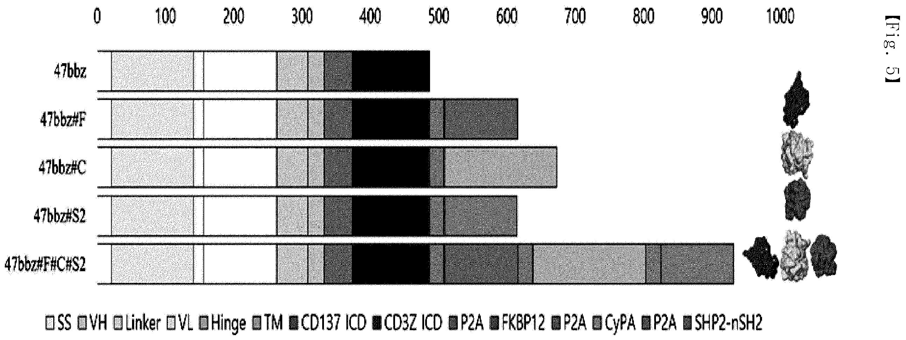

Figure 5 is a view showing the domain structure of the 47bbz CAR experimental group constructed in one embodiment.
Figure 6 is a view showing the domain structure of the Pbbz CAR experimental group constructed in one embodiment.
Figure 7 is an image comparing anti-tumor potency of the CAR-T cell experimental group constructed in one embodiment to evaluate anti-tumor therapeutic efficacy in an animal model of hematological malignancy (II).
Figure 8 is a view showing a map of a lentiviral vector expressing 19bbz used in one embodiment.
Figure 9 is a view showing a map of a lentiviral vector expressing 19bbz#F used in one embodiment.
Figure 10 is a view showing a map of a lentiviral vector expressing 19bbz#M used in one embodiment.
Figure 11 is a view showing a map of a lentiviral vector expressing 19bbz#N used in one embodiment.
Figure 12 is a view showing a map of a lentiviral vector expressing 19bbz#C used in one embodiment.
Figure 13 is a view showing a map of a lentiviral vector expressing 19bbz#S1 used in one embodiment.
Figure 14 is a view showing a map of a lentiviral vector expressing 19bbz#S2 used in one embodiment.
Figure 15 is a view showing a map of a lentiviral vector expressing 19bbz#TC used in one embodiment.
Figure 16 is a view showing a map of a lentiviral vector expressing 19bbz#RG used in one embodiment.
Figure 17 is a view showing a map of a lentiviral vector expressing 19bbzT used in one embodiment.
Figure 18 is a view showing a map of a lentiviral vector expressing 19bbz#FCS2 used in one embodiment.
Figure 19 is a view showing a map of a lentiviral vector expressing Hbbz used in one embodiment.
Figure 20 is a view showing a map of a lentiviral vector expressing Hbbz#F used in one embodiment.
Figure 21 is a view showing a map of a lentiviral vector expressing Hbbz#C used in one embodiment.
Figure 22 is a view showing a map of a lentiviral vector expressing Hbbz#S2 used in one embodiment.
Figure 23 is a view showing a map of a lentiviral vector expressing Hbbz#FCS2 used in one embodiment.
Figure 24 is a view showing a map of a lentiviral vector expressing 43bbz used in one embodiment.
Figure 25 is a view showing a map of a lentiviral vector expressing 43bbz#F used in one embodiment.
Figure 26 is a view showing a map of a lentiviral vector expressing 43bbz#C used in one embodiment.
Figure 27 is a view showing a map of a lentiviral vector expressing 43bbz#S2 used in one embodiment.
Figure 28 is a view showing a map of a lentiviral vector expressing 43bbz#FCS2 used in one embodiment.
Figure 29 is a view showing a map of a lentiviral vector expressing 47bbz used in one embodiment.
Figure 30 is a view showing a map of a lentiviral vector expressing 47bbz#F used in one embodiment.
Figure 31 is a view showing a map of a lentiviral vector expressing 47bbz#C used in one embodiment.
Figure 32 is a view showing a map of a lentiviral vector expressing 47bbz#S2 used in one embodiment.
Figure 33 is a view showing a map of a lentiviral vector expressing 47bbz#FCS2 used in one embodiment.
Figure 34 is a view showing a map of a lentiviral vector expressing Pbbz used in one embodiment.
Figure 35 is a view showing a map of a lentiviral vector expressing Pbbz#F used in one embodiment.
Figure 36 is a view showing a map of a lentiviral vector expressing Pbbz#C used in one embodiment.
Figure 37 is a view showing a map of a lentiviral vector expressing Pbbz#S2 used in one embodiment.
Figure 38 is a view showing a map of a lentiviral vector expressing Pbbz#FCS2 used in one embodiment.
Figure 39 is a view showing a map of a lentiviral vector expressing HERV-E specific TCR used in one embodiment.
Figure 40 is a view showing a map of a lentiviral vector expressing HERV-E specific TCR#F used in one embodiment.
Figure 41 is a view showing a map of a lentiviral vector expressing HERV-E specific TCR#C used in one embodiment.
Figure 42 is a view showing a map of a lentiviral vector expressing HERV-E specific TCR#S2 used in one embodiment.
Figure 43 is a view showing a map of a lentiviral vector expressing HERV-E specific TCR#FCS2 used in one embodiment.
Figure 44 is a view showing a map of a lentiviral vector expressing NY-ESO-1 specific TCR used in one embodiment.
Figure 45 is a view showing a map of a lentiviral vector expressing NY-ESO-1 specific TCR#F used in one embodiment.
Figure 46 is a view showing a map of a lentiviral vector expressing NY-ESO-1 specific TCR#C used in one embodiment.
Figure 47 is a view showing a map of a lentiviral vector expressing NY-ESO-1 specific TCR#S2 used in one embodiment.
Figure 48 is a view showing a map of a lentiviral vector expressing NY-ESO-1 specific TCR#FCS2 used in one embodiment.
Figure 49 is a table summarizing the CAR-T experimental group used in one embodiment to evalute anti-tumor therapeutic efficacy in an animal model of hematological malignancy (I).
Figure 50 is an image comparing anti-tumor potency of the CAR-T cell experimental group constructed in one embodiment to evaluate anti-tumor therapeutic efficacy in an animal model of hematological malignancy (I).
Figure 51 is a view showing the cocrytal structure of TGF-β type 1 receptor kinase domain bound to FKBP12.
Figure 52 is a view showing the tetrad aromatic amino acids (Aromatic Residues) of FKBP12, which are pivotally involved in FKBP12 binding to TGF-β type 1 receptor.
Figure 53 is a view indicating the aromatic amino acids of FKBP12 pivotally involved in FKBP12 binding to TGF-β type 1 receptor
Figure 54 is a view of flow cytometric analysis confirming the expression of CD19-specific CAR in the CAR-T cells constructed in one embodiment.
Figure 55 is an image of immunofluorescence microscopy revealing the feature of surface CAR expression in the CD19-specific CAR-T cells constructed in one embodiment.
Figure 56 is an image of immunofluorescence microscopy showing CAR-mediated pairing of CAR T cells with the target tumor cells in one embodiment.
Figure 57 is a view of flow cytometric analysis in the gate of CAR positive cells to evaluate the T cell subsets of ex *vivo* expanded CD19-specific CAR-T cells in one embodiment.
Figure 58 is a view of flow cytometric analysis on CD19 expression in K562, K562-CD19 and Daudi cells.
Figure 59 is a blot image showing the protein expression of FKBP12, cyclophilin A, and N-terminal SH2 domain of SHP2 protein in the CAR-T cells constructed in one embodiment.
Figure 60 is a table showing the RNA expression of FKBP12, cyclophilin A, and N-terminal SH2 domain of SHP2 protein in the CAR-T cells constructed in one embodiment. RNA expression was quantitated by real-time polymerase chain reaction using the cDNA derived from the same amount of total RNA.
Figure 61 is a graph showing the amount of IFNγ released by the CD19-specific CAR-T cells upon antigenic stimulation.
Figure 62 is a graph showing the amount of TNFα released by the CD19-specific CAR-T cells upon antigenic stimulation.
Figure 63 is a graph showing the amount of IL-2 released by the CD19-specific CAR-T cells upon antigenic stimulation.
Figure 64 is a graph showing the amount of IFNy released by the CD19-specific CAR-T cells upon antigenic stimulation in the presence of TGF-β1 in one embodiment.
Figure 65 is a graph showing the amount of TNFα released by the CD19-specific CAR-T cells upon antigenic stimulation in the presence of TGF-β1 in one embodiment.
Figure 66 is a graph showing the results *of in vitro* cell migration assay performed after activation of the CD19-specific CAR-T cells with anti-CD3/anti-CD28 beads.
Figure 67 is an image showing the intrinsic motility of the CAR-T cells cultured in the presence of antigenic stimulation in one embodiment.
Figure 68 is a graph showing the assay results of CAR T-mediated tumor cell lysis measured against K562-CD19 cells (Bottom) and Daui Fluc-eGFP cells (Top), as the target cells of the CD19-specific CAR-T cells, respectively.
Figure 69 is a view of flow cytometric analysis confirming the expression of CAR in the Her2-specific CAR-T cells constructed in one embodiment. Specifically, the expression of Her2-specific CAR was analyzed by flow cytometry for the T cells transduced with Hbbz, Hbbz#F, Hbbz#C, Hbbz#S2 or Hbbz#FCS2 lentivirus, respectively.
Figure 70 is an image of immunofluorescence microscopy revealing the feature of surface CAR expression in the Her2-specific CAR-T cells constructed in one embodiment.
Figure 71 is a graph showing the amount of IFNγ released by the Her2-specific CAR-T cells upon antigenic stimulation.
Figure 72 is a graph showing the amount of TNFα released by the Her2-specific CAR-T cells upon antigenic stimulation.
Figure 73 is a graph showing the amount of IL-2 released by the Her2-specific CAR-T cells upon antigenic stimulation.
Figure 74 is a graph showing the results *of in vitro* cell migration assay performed after activation of the Her2-specific CAR-T cells with anti-CD3/anti-CD28 beads.
Figure 75 is a graph showing the assay results of CAR T-mediated tumor cell lysis measured against SKBR3-Luc cells, as the target cells of the Her2-specific CAR-T cells.
Figure 76 is an image comparing anti-tumor potency of of the Her2-specific CAR-T cells used in one embodiment.
Figure 77 is a view of flow cytometric analysis confirming the expression of PSMA-specific CAR in the CAR-T cells constructed in one embodiment.
Figure 78 is an image of immunofluorescence microscopy revealing the feature of surface CAR expression in the PSMA-specific CAR-T cells constructed in one embodiment.
Figure 79 is a view of flow cytometric analysis in the gate of CAR positive cells to evaluate the T cell subsets of ex *vivo* expanded PSMA-specific CAR-T cells.
Figure 80 is a view of flow cytometric analysis in the gate of CAR positive cells to evaluate the T cell exhaustion of ex *vivo* expanded PSMA-specific CAR-T cells
Figure 81 is a graph showing the results *of in vitro* cell migration assay performed after activation of the PSMA-specific CAR-T cells with anti-CD3/anti-CD28 beads.
Figures 82 and 83 are graphs showing the assay results of CAR T-mediated tumor cell lysis measured against the target tumor cells of the PSMA-specific CAR-T cells.

### Best Mode for Carrying out the Invention

Hereinafter, the present invention will be described in detail.

### Polynucleotide including a self-cleavage peptide

One aspect of the present invention provides a polynucleotide encoding a fusion protein that includes (i) an antigen binding domain; (ii) a transmembrane domain; (iii) an intracellular signaling domain including at least one co-stimulatory domain; (iv) a self-cleavage peptide; and (v) a signaling pathway modulator(s).

In this case, additionally a spacer may be placed between (i) the antigen binding domain and (ii) the transmembrane domain.

In this case, the polynucleotide may be specifically in the following form:
(a) a polynucleotide including a polynucleotide encoding a fusion protein containing (i) an antigen binding domain, (ii) a spacer and transmembrane domain, and (iii) an intracellular signaling domain including at least one co-stimulatory domain; (iv) a polynucleotide encoding a self-cleavage peptide; and (v) a polynucleotide encoding a signaling pathway modulator(s); or
(b) a polynucleotide including (i) a polynucleotide encoding an antigen binding domain; (ii) a polynucleotide encoding a spacer and transmembrane domain; (iii) a polynucleotide encoding an intracellular signaling domain including at least one co-stimulatory domain; (iv) a polynucleotide encoding a self-cleavage peptide; and (v) a polynucleotide encoding a signaling pathway modulator(s).

In this case, (i) the polynucleotide encoding an antigen binding domain; (ii) the polynucleotide encoding a transmembrane domain; (iii) the polynucleotide encoding an intracellular signaling domain including at least one co-stimulatory domain; (iv) the polynucleotide encoding a self-cleavage peptide; and (v) the polynucleotide encoding a signaling pathway modulator(s) may be linked sequentially in the order from 5' to 3'. However, as long as a signaling pathway modulator(s) may be expressed in the cytoplasm, the sequence arrangement may be appropriately modified. For example, a signaling pathway modulator(s) may be located upstream of an antigen binding domain. In this case, a self-cleavage sequence may be located downstream of a signaling pathway modulator(s).

### Signaling pathway modulator that is externally introduced

In addition, the signaling pathway modulator may be any one selected from the group consisting of a) a protein located in the immunosuppressive signaling pathway, b) an immunophilin or a fragment thereof, c) a protein involved in the antigen loss-mediated relapse, d) a protein located in the T cell stimulatory signaling pathway, e) a protein involved in the inhibition of negative feedback, and f) a combination thereof.

In this case, the signaling pathway modulator is characterized in that it operates in the cytoplasm.

### Protein located in the immunosuppressive signaling pathway: TGF-β/SMAD signaling pathway

The protein located in the immunosuppressive signaling pathway may be a protein located in the TGF-β/SMAD (FKBP12-FK506/rapamycin) signaling pathway or a fragment thereof. Specifically, the protein located in the TGF-β/SMAD (FKBP12-FK506/rapamycin) signaling pathway may be any one selected from the group consisting of FKBP12 (FK506-binding protein 12, hereinafter, FKBP12 coming after a self-cleavage peptide is referred to as #F, SEQ ID NO: 13), a C-terminal MH2 domain of a SMAD4 protein (hereinafter, a C-terminal MH2 domain of a SMAD4 protein coming after a self-cleavage peptide is referred to as #M, SEQ ID NO: 20) and the N-terminal domain of SKI protein (N-SKI, hereinafter, N-SKI coming after a self-cleavage peptide is referred to as #N, SEQ ID NO: 22).

As used herein, the term "transforming growth factor β (TGF-β)" is a member of the transforming growth factor β superfamily, and refers to a cytokine that performs various cellular functions including cell growth, differentiation, cell death, development and the like.

As used herein, the term "TGF-β/SMAD signaling pathway" refers to a signaling pathway activated by TGF-β. In the process of TGF-β signal transduction, ligand binding promotes the formation of a heterotetramer consisting of TGF-β type 1/type 2 receptors, and the TGF-β type 1 receptor is phosphorylated by the TGF-β type 2 receptor. Once the TGF-β type 1 receptor is phosphorylated, SMAD, a signal transducing protein present in the cytoplasm, is phosphorylated, thereby enabling subsequent signal transduction. The phosphorylated SMAD forms SMAD trimer via the C-terminal domain, and then the SMAD trimer translocates into the nucleus, where it binds transcriptional factors/ cofactors and cause up-/ downregulation of specific target genes. TGF-β/SMAD signaling in T cells results in a significant reduction in cytokine production, cell proliferation and a variety of immune-associated functions. Specifically, the protein located in the TGF-β/SMAD signaling pathway may be FKBP12, a C-terminal MH2 domain of a SMAD4 protein and N-SKI and the like.

As used herein, the term "FKBP12" is one of the FK506 binding protein family, with a molecular mass of12 kDa, and is involved in the regulation of various cellular activities such as protein folding and cell trafficking and immune modulation. Specifically, FKBP12 binds to the TGF-β type 1 receptor, and sterically blocks TGF-β type 2 receptor-mediated phosphorylation of TGF-β type 1 receptor, thereby impeding TGF-β-medaiated signaling. Interestingly, it is known that even in the absence of TGF-β, TGF-β type 1 receptor is able to form a complex with TGF-β type 2 receptor via their innate affinity for each other to some extent. Therefore, regardless of the presence or absence of TGF-β, such a leaky signaling can occur all the time. FKBP12, which can inhibit the phosphorylation of the TGF-β type 1 receptor, is known consequently to function as a "molecular guardian impeding TGF-β leaky signaling." On the other hand, the two well-known immunosuppressive drugs, rapamycin and FK506, are revealed to bind to FKBP12 so that they competitively interfere with FKBP12 binding to TGF-β type 1 receptor. Thus, rapamycin and FK506 promote immunosuppressive signaling which is dependent on the phosphorylation of TGF-β type 1 receptor.

In addition, the complex of FKBP12 and FK506, an immunosuppressive drug, is able to inhibit the activity of calcineurin by its binding to calcineurin. The "calcineurin" is a calcium (Ca²⁺)-dependent protein phosphatase, which activates an inflammatory immune response by mediating dephosphorylation of NFAT (nuclear factor of activated T-cells). The NFAT (Nuclear factor of activated T-cell) is a transcription factor, and is expressed in most immune cells. In particular, the NFAT functions to enhance T cell-mediated immune reactions because it upregulates the transcription of interleukin-2 (IL-2) in T cells.

The FKBP12 may be derived from human, and may include the amino acid sequence disclosed in NCBI Reference Sequence: NP_000792.1 or NP_001186715.1. Alternatively, the FKBP12 may include the amino acid sequence disclosed in NP_004107 (FKBP12.6).

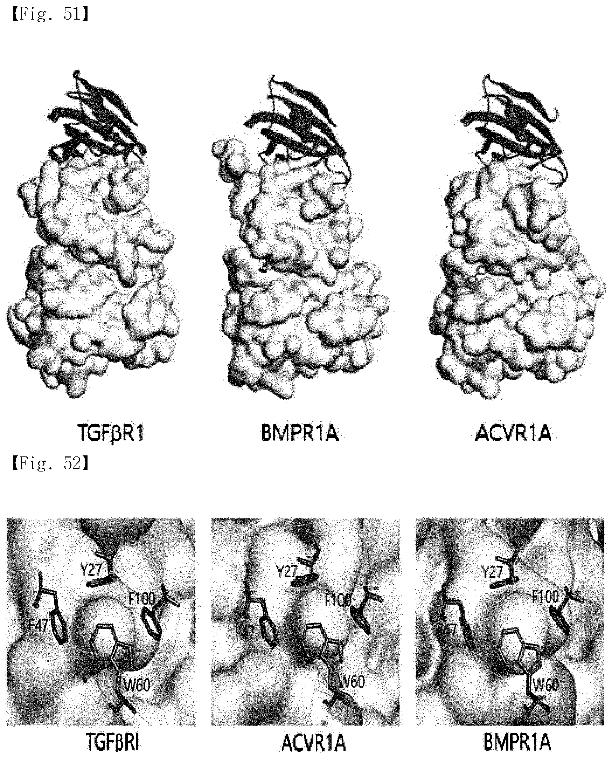

In addition, the FKBP12 may include the amino acid sequence represented by SEQ ID NO: 13 or 19. In addition, the FKBP12 may have about 80%, 90%, 95% or 99% or more homology to the amino acid sequence of SEQ ID NO: 13 or 19. In addition, the nucleotide sequence encoding the FKBP12 may be the nucleotide sequence represented by SEQ ID NO: 14. In addition, the nucleotide sequence encoding the FKBP12 may have about 95%, 97% or 99% or more homology to the nucleotide sequence represented by SEQ ID NO: 14. In addition, the fragment of FKBP12 may include amino acids from amino acid 27 to amino acid 100 of SEQ ID NO: 13. As shown in Figure 52, amino acid 27, amino acid 47, amino acid 60, amino acid 100 of SEQ ID NO: 13 are the amino acids involved in the binding of the TGF-β receptor.

As used herein, the term "SMAD" is a key protein transducing signals coming from the receptors of TGF-β superfamily, and is involved in cell growth, differentiation, cell death, development and the like. There are three types of SMADs: receptor-regulated SMADs (R-SMADs), common partner SMAD (Co-SMAD) and inhibitory SMADs (I-SMADs). A trimer of two receptor-regulated SMADs and a common partner SMAD acts as a transcription factor that regulates the expression of specific genes. The receptor-regulated SMAD includes SMAD1, SMAD2, SMAD3, SMAD5 and SMAD8/9, and the common partner SMAD includes SMAD4, and the inhibitory SMAD includes SMAD6 and SMAD7. The receptor-regulated/common partner SMAD complex is mainly present in the cytoplasm, but after receiving the TGF-β signal, it accumulates and acts in the nucleus, and I-SMADs are mainly present in the nucleus, and serve as a transcriptional regulatory factor.

As used herein, the term "SMAD4" is a common partner receptor as described above, and forms a complex with receptor-regulated SMADs to support the action of the receptor-regulated SMAD. On the other hand, SMAD4 mediates TGF-β-dependent reduction of the c-myc expression in T cells, T cell proliferation and immune response of T cells. The "c-myc" is a proto-oncogene, and is a transcription factor that regulates the cell proliferation and growth. Specifically, SMAD4 may be a protein having the amino acid sequence disclosed in NP_005350. The fragment of SMAD4 (the C-terminal MH2 domain of the SMAD4 protein) used in one embodiment of the present invention is a domain involved in the "SMAD Trimerization." Therefore, upon the overexpression of this fragment in immune cells, nonfunctional SMAD complex, incapable of acting as a transcription factor due to the lack of DNA binding activtiy, is formed and thus the immunosuppressive TGF-β signaling is blocked, thereby increasing the overall immune reactivity. This SMAD4 fragment may consist of the amino acid sequence of SEQ ID NO: 20. In addition, the SMAD4 fragment may have about 80%, 90%, 95% or 99% or more homology to the amino acid sequence of SEQ ID NO: 20.

As used herein, the term "SKI" is a proto-oncogene product that acts as a transcriptional regulatory protein enhancing the transcription of specific genes in the nucleus. One of several known activities of the SKI protein is to block TGF-β signal by inhibiting the formation of the functional SMAD trimer. The N-SKI used in one embodiment is involved in the function of the SKI protein that blocks the TGF-β signal. Specifically, the N-terminal region of SKI protein directly binds to SMAD and inhibits the action of TGF-β by interrupting SMAD-mediated transcriptional regulation on the target genes. In this case, the N-terminal region of SKI protein that binds to SMAD may be referred to as N-SKI. This N-SKI binds to the trimerization domain of the SMAD protein and interferes with the formation of the functional SMAD trimer. Therefore, upon the overexpression of this fragment in immune cells, the immunosuppressive TGF-β signaling is blocked, thereby increasing the overall immune reactivity. Specifically, the SKI may be a protein including the amino acid sequence disclosed as NP_003027. The fragment of N-SKI used in one embodiment may be the amino acid sequence of SEQ ID NO: 22. In addition, the fragment of N-SKI may have about 80%, 90%, 95% or 99% or more homology to the amino acid sequence of SEQ ID NO: 22.

### Protein located in the immunosuppressive signaling pathway: protein located in the immune checkpoint pathway

The protein located in the immunosuppressive signaling pathway may be a protein located in the inhibitory immune checkpoint pathway, or a fragment thereof. Specifically, the protein located in the inhibitory immune checkpoint pathway may be an N-terminal SH2 (N-SH2) domain of a SHP-1 protein (Src homology 2 domain-containing phosphatase 1, hereinafter, the SHP-1 coming after a self-cleavage peptide is referred to as #S1, SEQ ID NO: 26) or an N-SH2 domain of a SHP-2 protein (hereinafter, the SHP-2 coming after a self-cleavage peptide is referred to as #S2, SEQ ID NO: 28).

As used herein, the term "inhibitory immune checkpoint pathway" refers to an intracellular signaling pathway that induces immune tolerance or inhibits immune stimulatory signaling pathway. Proteins involved in the inhibitory immune checkpoint pathway in T cells include programmed cell death protein 1 (PD-1), cytotoxic T-lymphocyte-associated antigen 4 (CTLA-4) and the like.

Specifically, the binding of PD-1 by its ligand PD-L1 or PD-L2, leads to the phosphorylation of ITIM (immunoreceptor tyrosine-based inhibitory motif) and ITSM (immunoreceptor tyrosine-based switch motif) in the cytoplasmic domain of PD-1 and the subsequent activation of SHP-2/SHP-1 and deactivation of immune stimulatory signals in T cells. CTLA-4 binds to CD80/86 to inhibit CD28 signaling, and activates SHP-2 through the YVKM motif present in the cytoplasmic domain to inhibit RAS, thereby inhibiting the immune stimulatory signaling in T cells.

As used herein, the term "SHP-1" is one of the PTP (protein tyrosine phosphatase) family, and is known as PTPN6 (tyrosine-protein phosphatase non-receptor type 6). The "PTP" is a signal transducing molecule that regulates various cellular processes including cell growth, differentiation, mitotic cycle and oncogenic transformation. SHP-1 contains two tandem Src homology (SH2, N-SH2 and C-SH2) domains involved in phosphotyrosine binding and a PTP domain (catalytic domain) that has a protein tyrosine phosphatase activity near C-terminus. The N-terminal SH2 (N-SH2) domain regulates the activity of SHP-1, which binds to the PTP domain in an inactive state and inhibits the activity of the enzyme. However, when the Tyr residue of the N-SH2 domain is phosphorylated, the N-SH2 domain is released from the PTP domain, and the free PTP domain interacts with the substrate, thereby converting SHP-1 into a state having dephosphorylation activity. Therefore, upon overexpression of an N-SH2 domain of a SHP-1 protein in immune cells, the inhibitory immune checkpoint signal is blocked by binding to the PTP domain of the SHP-1 protein and maintaining an inactive state. Therefore, the overall immune activity of the corresponding immune cells is increased. The SHP-1 may be a protein including the amino acid sequence disclosed in NP_002822. The fragment of SHP-1 used in one embodiment may be the amino acid sequence of SEQ ID NO: 26. In addition, the fragment of SHP-1 may have about 80%, 90%, 95% or 99% or more homology to the amino acid sequence of SEQ ID NO: 26.

As used herein, the term "SHP-2" is one of the PTP family, and is also known as PTPN11 (tyrosine-protein phosphatase non-receptor type 11), PTP-1D (protein-tyrosine phosphatase 1D) or PTP-2C (protein-tyrosine phosphatase 2C). Similar to the SHP-1, SHP-2 also consists of two tandem Src homologue (SH2, N-SH2 and C-SH2) domains that act as a protein phosphotyrosine binding domain at the N-terminal region and the PTP domain (catalytic domain) that has phosphatase activity at the C-terminal region. When SHP-2 is in an inactivated state, the N-SH2 domain occludes the active site of the PTP domain of SHP-2, and when N-SH2 domain binds a phosphotyrosine-containing peptide region, the PTP domain is released from N-SH2 domin, and become catalytically active. Therefore, upon overexpression of an N-SH2 domain of a SHP-2 protein in immune cells, the inhibitory immune checkpoint signal is blocked by binding to the PDP domain of the SHP-2 protein and maintaining an inactive state. Therefore, the overall immune activity of the corresponding immune cells is increased by ectopical expression of the N-SH2 domain. The SHP-2 may be a protein that has the amino acid sequence disclosed as NP_002825. The fragment of SHP-2 used in one embodiment may be the amino acid sequence of SEQ ID NO: 28. In addition, the fragment of SHP-2 may have about 80%, 90%, 95% or 99% or more homology to the amino acid sequence of SEQ ID NO: 28.

### Immunophilin: FKBP12 and cyclophilin A

As used herein, the term "immunophilin" refers to a target protein of a well-known immunosuppressive agent such as FK506, Rapamycin, and cyclosporine. One class of immunophilins known so far is a protein that binds to FK506, and there are 16 immunophilins, one of which is FKBP12. Another class of immunophilins known so far is a protein that binds to cyclosporine, and there are 16 immunophilins, one of which is cyclophilin A. They all commonly have a peptidyl prolyl isomerase (PPI) activity. This activity promotes peptide bond isomerization and mediates protein folding. This regulates various intracellular signaling pathways. When they bind to an immunosuppressive agent and their original functions are interfered, immunosuppression is induced. Therefore, these immunophilin proteins are understood to have important functions for immune activity. As an example of such immune-related functions, one of the known functions that FKBP12 and cyclophilin A (CYPA) perform is to regulate the T cell adhesion and migration.

An immunophilin in T cells is known to bind to Crkll (CT10 regulator of kinase II) to promote the activation of Crkll. The "Crk" is one of the adaptor proteins that mediate signal transduction by transmitting a signal formed by a T cell antigen receptor (TCR) that responds to an external stimulus to a downstream protein of the receptor, and includes Crkl and Crkll.

In T cells, "Crkll" binds to ZAP70, a protein located downstream of the signaling pathway of TCR, and transmits a signal to C3G (Crk SH3 domain-binding guanine-nucleotide releasing factor), thereby inducing the activity of RAP1 (Ras-related protein 1). The TCR is a receptor located on the surface of T cells, and recognizes an antigen presented by a major histocompatibility complex (MHC) of an antigen presenting cell, thereby activating an immune response of T cells.

The "ZAP70" is a constituent protein of TCR, and transmits an activation signal of TCR to a downstream protein to induce the activity of T cells. The "RAP1" is a small GTPase and belongs to the Ras superfamily. GTPase is activated when bound to GTP and inactivated when bound to GDP. The activity of such GTPase is regulated by GTPase activity proteins (GAPs) and GEF (guanine nucleotide exchange factor), and GAP promotes the formation of GTPase to which GDP is bound, and GEF promotes the formation of GTPase to which GTP is bound.

The "C3G" is a type of GEF, and increases RAP1 to which GTP is bound in T cells, thereby inducing the activity of LFA-1 (lymphocyte function-associated antigen 1) to increase T cell adhesion. The "LFA-1" is one of integrins expressed in T cells, and binds to ICAM-1 (intercellular adhesion molecule 1), a ligand expressed in a target cell in the process of migrating from blood into body tissue, to mediate the adhesion of T cells to a target cell. An immunophilin such as FKBP12 and CYPA is known to bind to Crkll and to increase the binding of Crkll and C3G by binding to Crkll, thereby increasing the activity of the downstream signaling pathway of C3G, thereby increasing T cell adhesion regulated by LFA-1.

In this case, one embodiment of the FKBP family may be FKBP12. In addition, the sequence of the FKBP12 protein and fragment thereof is as described above in "Protein located in the immunosuppressive signaling pathway." In addition, one embodiment of cyclophilin may be cyclophilin A. A protein located in the cyclophilin A-mediated signaling pathway may be CYPA (cyclophilin A, hereinafter, CYPA bound after a self-cleavage peptide is referred to as #C, SEQ ID NO: 24).

As used herein, the term "cyclosporine A (CsA)" is an immunosuppressive agent derived from a natural product. It is taken orally or administered through intravenous injection, and is used as a drug to prevent rheumatoid arthritis, psoriasis, Crohn's disease, nephrotic syndrome and organ transplant rejection. The cyclophilin A is a cytoplasmic binding protein that binds to CsA, and the CsA/CYPA complex inhibits the phosphatase activity of calcineurin, thereby inhibiting the immune response of lymphocytes. In addition, CYPA is involved in protein folding through the activity of peptidyl prolyl isomerase (PPI), and through this activity, it regulates biological processes such as intracellular signaling, transcription, inflammation and cell death and the like. In addition to its intracellular role, it is known that CYPA can be secreted in response to inflammatory stimulation, hypoxia, infection and oxidative stress, and acts as a chemoattractant in viral infection, periodontitis, and atherosclerosis, thereby promoting the inflammatory response. Specifically, cyclophilin A used in one embodiment is a protein including the amino acid sequence disclosed as NP_066953. Specifically, cyclophilin A may include the amino acid sequence of SEQ ID NO: 24. In this case, the cyclophilin A may have about 80%, 90%, 95% or 99% or more homology to the amino acid sequence of SEQ ID NO: 24.

### Protein involved in the antigen loss-mediated relapse

The protein involved in the antigen loss-mediated relapse may be a protein involved in trogocytosis or a fragment thereof. Specifically, the protein involved in trogocytosis may be TC21 (teratocarcinoma oncogene 21, hereinafter, TC21 bound after a self-cleavage peptide is referred to as #TC, SEQ ID NO: 32) or RhoG (Ras homology growth-related, hereinafter, RhoG bound after a self-cleavage peptide is referred to as #RG, SEQ ID NO: 34).

As used herein, the term "TC21" is also known as R-Ras2, and is one of the Ras GTPases superfamily. It binds to a cell membrane and mediates signal transduction related to the cell proliferation. In addition, it is known to bind to TCR and activate PI3K (phosphoinositide 3-kinase), a downstream protein on the signaling pathway, thereby inducing the internalization of immunological synapse and mediating membrane molecule transport. The TC21 may be a protein that has the amino acid sequence disclosed in NP_036382. In addition, the TC21 may be a varient, and the TC21 used in one embodiment may include the amino acid sequence of SEQ ID NO: 32. The TC21 may have about 80%, 90%, 95% or 99% or more homology to the amino acid sequence of SEQ ID NO: 32.

As used herein, the term "RhoG" is a monomeric GTP binding protein (G protein), and is involved in the regulation of cell motility, transcription, endocytosis, dendrite growth and the like. In addition, it is also known to mediate membrane molecule transport induced by TCR by being activated by TC21 and PI3K. The RhoG may be a protein that has the amino acid sequence disclosed in NP_001656. In addition, the RhoG may be a varient, and a fragment of RhoG used in one embodiment may include the amino acid sequence of SEQ ID NO: 34. The fragment of RhoG may have about 80%, 90%, 95% or 99% or more homology to the amino acid sequence of SEQ ID NO: 34.

As used herein, the term "membrane molecule transport" refers to a phenomenon in which T cells and antigen presenting cells are bound through an immunological synapse, and in this state, the surface molecules of one cell are separated and transported to other cells. It is known as a phenomenon that occurs in order to regulate the inhibition or amplification of the immune response. The "immunological synapse" is a molecule structure formed in the process of adhesion and recognition of T cells and antigen presenting cells.

When the TC21 and RhoG proteins are overexpressed in immune cells, trogocytosis is interfered in the reaction with cancer cells, and thus immune-evasive process mediated by antigen loss can be inhibited.

### Protein located in the T cell stimulatory signaling pathway: adaptor protein of the TCR/ZAP70 pathway

The protein located in the T cell stimulatory signaling pathway may be an adaptor protein of the TCR/ZAP70 pathway or a fragment thereof. Specifically, the adaptor protein of the TCR/ZAP70 pathway may be any one selected from the group consisting of NCK1 (hereinafter, NCK1 bound after a self-cleavage peptide is referred to as #K), LAT (Linker for activation of T cells, hereinafter, LAT bound after a self-cleavage peptide is referred to as #L) and NEMO (NF-κB essential modulator, hereinafter, NEMO bound after a self-cleavage peptide is referred to as #I).

As used herein, the term "TCR/ZAP70 pathway" is a signaling pathway that occurs when a T cell antigen receptor of a naive T cells binds to the MHC/antigen complex of an antigen presenting cell, and activates an immune response of T cells. Specifically, when the TCR of naive T cells binds to the MHC/antigen complex, the gamma (γ), delta (δ), epsilon (ε), and zeta (ζ) chains of the accessory protein CD3 of TCR are phosphorylated, and ZAP70 binds to the phosphorylated CD3ζ chain. Then, LCK that is bound to CD4 or CD8, an accessory receptor of T cells, phosphorylates and activates ZAP70.

The activated ZAP70 phosphorylates LAT and SLP-76 (SH2 domain-containing leukocyte protein 76 kDa) to induce a signal response that activates transcription factors such as NF-κB (nuclear factor kappa-light-chain-enhancer of activated B cells), AP-1 (activator protein 1) and NFAT and the like. The NF-κB is a transcription factorthat regulates inflammatory response regulation, immune modulation, cell death, cell proliferation and differentiation and the like, and consists of p50, p52, RelA (p65), ReIB, c-Rel and v-Rel. AP-1 is a transcription factor that regulates inflammatory response regulation, cell death, cell proliferation and differentiation and the like, and forms a dimer with other transcription factors such as c-Fos, c-Jun, ATF (activating transcription factor) and JDP and the like, thereby acting to regulate transcription. The transcription factors such as NF-κB, AP-1 and NFAT promote the expression of interleukin-2, thereby activating T cell division, differentiation and immune response.

As used herein, the term "NCK1" is a signaling-mediated protein including SH2 and SH3 domains, and mediates the signaling of tyrosine kinase receptor. In addition, NCK1 can regulate cytoskeleton rearrangement by binding to a WASP/Arp2/3 complex. The "WASP/Arp2/3 complex" is a protein that induces the formation of actin filament, which is a cytoskeleton. In T cells, NCK promotes the formation of an immunological synapse that is induced by the activity of TCR. The NCK1 may be a protein including the amino acid sequence disclosed in NP_006144.

As used herein, the term "LAT" is a 34 kDa transmembrane protein, and is phosphorylated by ZAP70/Syk (spleen-associated tyrosine kinase) upon activation of the signaling pathway of TCR. Adaptor proteins including the SH2 domain involved in the signaling pathway of TCR directly/indirectly bind to the phosphorylated LAT to mediate signal transduction. The adaptor protein may include PLCγ1 (phospholipase C γ1), Grb2 (growth factor receptor-bound protein 2), Gads (Grb2-related adapter protein downstream of Shc), Grap (Grb2-related adaptor protein), SH3BP2 (SH3 domain-binding protein 2), Shb (SH2 domain-containing adapter protein B), SOS1 (son of sevenless homolog 1), c-Cbl (casitas B lymphoma), VAV, SLP-76 (SH2 domain-containing leukocyte protein of 76 kDa), and Itk (IL-2 inducible T cell kinase) and the like. The LAT may be a protein including the amino acid sequence disclosed as NP_055202.

As used herein, the term "NEMO" is known as IKKγ (IκB kinase γ), and forms a complex with IKKα/IKKβ to induce phosphorylation and degradation of IκB (inhibitor of nuclear factor κ-B kinase), thereby promoting the activity of NF-κB. The NEMO may be a protein including the amino acid sequence disclosed as NP_001093326.

### Protein located in the T cell stimulation pathway: protein located in the TNFR/TLR receptor pathway

The protein located in the T cell stimulation pathway may be a protein located in the TNFR/TLR receptor (TRAF/NF-kB) pathway or a fragment thereof. Specifically, the TNFR/TLR receptor (TRAF/NF-kB) pathway protein may be TLR4, and preferably, may be an intracellular signaling domain of the TLR4 protein (hereinafter, an intracellular signaling domain of TLR4 protein bound after a self-cleavage peptide is referred to as #T, SEQ ID NO: 30).

As used herein, the term "TNFR (tumor necrosis factor receptor)" is a receptor of tumor necrosis factor α (TNFα). The TNFα is an inflammatory cytokine mainly produced in activated macrophages, helper T cells, natural killer cells and the like, and regulates various biological activities such as cell growth, differentiation and cell death, inflammatory response and the like.

As used herein, the term "TNFR signaling pathway" refers to an intracellular signaling pathway induced by binding of TNFR to a ligand. When TNFα binds to TNFR1, TRADD (TNF receptor-associated death domain), RIP1 (receptor-interacting protein kinase 1), TRAF (tumor necrosis factor receptor (TNFR)-associated factors) 2 or TRAF5 (TRAF2/5), clAP1 (apoptosis Inhibitor 1), and clAP2 form a complex to promote the activation of TAK1. The activated TAK1 (transforming growth factor-β-activated kinase 1) may phosphorylate IKKβ to induce phosphorylation and proteolysis of IκB, thereby inducing the activity of NF-κB.

As used herein, the term "TLR (Toll-like receptor)" is a protein that plays an important role in innate immunity. TLR is a non-catalytic single protein receptor embedded in a cell membrane, and is mainly expressed on the surface of macrophages, dendritic cells and the like or mucosal epithelial cells, neutrophils and the like in the process of innate immunity. There are 13 types of TLRs: TLR1, TLR2, TLR3, TLR4, TLR5, TLR6, TLR7, TLR8, TLR9, TLR10, TLR11, TLR12 and TLR13. Among them, TLR11, TLR12 and TLR13 are not expressed in humans.

As used herein, the term "TLR signaling pathway" is an intracellular signaling pathway induced by binding of TLRs to a ligand, and signaling for TLRs is generally activated through MyD88 (myeloid differential factor 88) and TRIF (Toll/IL-1R domain-containing adaptor inducing IFN-γ). MyD88 binds to TIR (Toll/IL-1R) domain of TLRs to induce the activity of IRAK-4 (IL-1 receptor-associate kinase 4), and the activated IRAK-4 phosphorylates IRAK-1 to induce the phosphorylation of TRAF6. The phosphorylated TRAF6 activates IKK to induce phosphorylation and proteolysis of IκB, thereby inducing the activity of NF-κB.

The "TLR4" is a receptor belonging to the TLR family, and is activated by recognizing LPS (lipopolysaccharide). "LPS" is also referred to as endotoxin, and is a molecule composed of lipids and polysaccharides, and is a component of the outer membrane of Gram-negative bacteria. The TLR4 may be a protein having the amino acid sequence disclosed as NP_003257. The fragment of TLR4 used in one embodiment may be the amino acid sequence of SEQ ID NO: 30. The fragment of TLR4 may have about 80%, 90%, 95% or 99% or more homology to the amino acid sequence of SEQ ID NO: 30.

### Protein located in the T cell stimulation pathway: protein located in the cytokine receptor (JAK-STAT) pathway

The protein located in the T cell stimulation pathway may be a protein located in the cytokine receptor (JAK-STAT) pathway or a fragment thereof.

As used herein, the term "cytokine" is a polypeptide or glycoprotein that is secreted by several types of cells in a living body and is involved in cell proliferation, differentiation, activation and the like, and plays an important role in immune and inflammatory response. As used herein, the term "cytokine receptor signaling pathway" refers to an intracellular signaling pathway induced by cytokines. Compared to the various types of cytokines that are present, the intracellular signaling process goes through a similar pathway. When a cytokine binds to each specific receptor, the oligomerization of the receptor is induced, and JAKs (janus kinases) located downstream in the receptor are phosphorylated and activated. A tyrosine residue in the cytoplasmic domain of the receptor is phosphorylated by the activated JAKs to bind to STAT (signal transducers and activators of transcription). In addition, STAT bound to the receptor is activated through phosphorylation by JAKs and separated from the receptor. The activated STAT forms a homodimer or heterodimer in the cytoplasm, and then translocates the nucleus to induce the expression of target genes.

### Protein located in the T cell stimulation pathway: protein located in the MAP kinase pathway

The protein located in the T cell stimulation pathway may be a protein located in the MAP kinase (mitogen-activated protein kinase) pathway or a fragment thereof. Specifically, the protein located in the MAP kinase pathway may be any one selected from the group consisting of GADD45α (growth arrest and DNA-damage-inducible gene 45α, hereinafter, GADD45α bound after a self-cleavage peptide is referred to as #A), CDC42 (cell division cycle 42, hereinafter, CDC42 bound after a self-cleavage peptide is referred to as #B), and HRAS (hereinafter, HRAS bound after a self-cleavage peptide is referred to as #H).

As used herein, the term "MAPK signaling pathway" refers to a signaling pathway that is activated by various mitogens such as growth factor, cytokine or hormone and the like, and is involved in various cellular functions including cell proliferation, differentiation and migration. This pathway includes many proteins including MAPK, which is called ERK (extracellular signal regulated kinase), and transmits a signal by phosphorylation of adjacent protein. The MAPK signaling pathway regulates cell responses such as inflammation, cell stress response, cell differentiation, cell division, cell proliferation, metabolism, motility and cell death and the like. In mammals, MAPK is divided into 4 groups: ERK-1/2, JNK1/2/3 (jun aminoterminal kinases 1, 2, 3), p38 protein (p38α, β, γ, δ), and ERK5.

As used herein, the term "GADD45" is a protein present in the nucleus, and there are three types: GADD45α, β, and γ. It acts as a sensor for environment and physiological stress, and binds to proteins related to cell cycle, cell survival, cell death, maintenance of genomic stability and DNA repair or regulates the activity thereof. GADD45α inhibits cell cycle G2/M conversion, and induces cell death, and stabilizes the genome through DNA-demethylation. In addition, it is known to bind to p38MAPK in type 1 helper T cells (Th1) and dendritic cells to inhibit phosphorylation (Tyr323) of p38MAPK, thereby inhibiting the activity of T cells. The GADD45 may be a protein having the amino acid sequence disclosed in NP_001915.

As used herein, the term "CDC42" is a GTPase belonging to the Rho family that regulates cell shape, migration, endocytosis and cell cycle and the like. The activity of CDC42 promotes cytoskeleton rearrangement to induce cell adhesion and migration. In addition, it is activated by the signaling protein downstream of TCR to promote the activation of JNK and p38MAPK, thereby inducing the production of inflammatory cytokines and the proliferation of T cells. The CDC42 may be a protein including the amino acid sequence disclosed as NP_001782.

As used herein, the term "HRAS" is a GTPase, also known as transforming protein 21, and regulates cell division by growth factors. The activated HRAS activates signaling proteins such as c-Raf and PI3K, which are the downstream proteins on the signaling pathway. HRAS binds to GTP in the active state, and is inactivated by cleaving the phosphate at the end of nucleotide to convert GTP into GDP. The HRAS may be a protein including the amino acid sequence disclosed in NP_005334.

### Protein involved in the inhibition of negative feedback

In addition, the protein involved in the inhibition of negative feedback may be a protein involved in the inhibition of negative feedback of cytokine signaling pathway or a fragment thereof. Specifically, the signal modulator involved in the inhibition of negative feedback of cytokine signaling pathway may be a C-terminal domain of a SOCS1 protein (suppressor of cytokine signaling 1, hereinafter, a C-terminal domain of a SOCS1 protein bound after a self-cleavage peptide is referred to as #Q, NP_003736).

As used herein, the term "cytokine signaling pathway" is as described for the cytokine receptor signaling pathway. The JAK-STAT signaling pathway includes a negative feedback regulation mechanism, and which is made by three types of regulatory protein groups: PIAS (protein inhibitors of activated STAT), PTPs, and SOCS. PIAS inhibits the activity of STAT by binding SUMO (small ubiquitin-like modifier) to STAT or inhibiting the binding of STAT and DNA. PIAS may be PIAS1, PIAS3, PIASx, and PIASγ. The "SUMO" is a protein that is covalently attached or detached to a protein, and binds to a target protein to regulate the function of the target protein. It mainly performs nucleus-cytoplasm transport, transcriptional regulation, protein stability regulation function. PTPs bind to cytokine receptor, JAK and STAT, and remove their phosphate groups, thereby inhibiting the cytokine signaling pathway. PTPs may be SHP-1, SHP-2, and CD45.

As used herein, the term "SOCS" is a protein family including a SOCS box motif having a SH2 domain and a 40 amino acid region at the C-terminus. The family includes eight proteins: CISH (cytokine-inducible SH2 domain-containing protein), SOCS1, SOCS2, SOCS3, SOCS4, SOCS5, SOCS6, and SOCS7. The SOCS and JAB (JAK-binding protein), CIS (cytokine-inducible STAT inhibitor), and SSI (STAT-induced STAT inhibitor) are the same protein family. The activity of the JAK-STAT signaling pathway by cytokines induces the expression of CISH, SOCS1 and SOCS3, and these proteins interfere with the activation of JAK or inhibit the activation of STAT in a manner of blocking the binding of STAT by binding to the phosphorylation domain of the receptor. In particular, "SOCS-1" induces the proteolysis of the phosphorylated JAK by binding to the phosphorylated JAK.

The C-terminal domain of the SOCS1 protein may bind to Elongin B/C, which mediates ubiquitin ligase, competitively with intact SOCS1. Therefore, when the C-terminal domain of the SOCS1 protein is overexpression, even if the intact SOCS1 protein binds to the phosphorylated JAK, the phosphorylated JAK protein is rescued without being degraded because it does not accompany ubiquitination. Therefore, this mechanism can increase the immune activity of immune cells. The signal modulator involved in the inhibition of negative feedback is a C-terminal domain of a SOCS1 protein, and may be a protein having the amino acid sequence disclosed as NP_003736.

### Combination of signaling pathway modulators

The signaling pathway modulator may include one or more signaling pathway modulators. In this case, a self-cleavage peptide may be included between each signaling pathway modulator. Specifically, the signaling pathway modulator may include two signaling pathway modulators. In addition, the signaling pathway modulator may include three signaling pathway modulators.

### Combination of two signaling pathway modulators

The signaling pathway modulator may have the structure of the following structural formula (I): $N ′ - X - L 1 - Y - C ′$ wherein, in the structural formula (I),
N' is an N-terminal of a fusion protein,
C' is a C-terminal of a fusion protein, and
Each of X and Y may be any one selected from the group consisting of a) a protein located in the immunosuppressive signaling pathway, or a fragment thereof; b) an immunophilin or a fragment thereof; c) a protein involved in the antigen loss-mediated relapse, or a fragment thereof; d) a protein located in the T cell stimulation pathway, or a fragment thereof; and e) a protein involved in the inhibition of negative feedback, or a fragment thereof.

L1 may be a self-cleavage peptide or an IRES (Internal Ribosome Entry Site).

Specifically, X may be selected from a protein located in the immunosuppressive signaling pathway. In one embodiment, X may be any one selected from the group consisting of a FKBP12 protein or a fragment thereof; a C-terminal MH2 domain of SMAD4, or a fragment thereof; N-SKI or a fragment thereof; an N-SH2 domain of a SHP-1 protein, or a fragment thereof; and an N-SH2 domain of a SHP-2 protein, or a fragment thereof. In addition, X may be an immunophilin. In one embodiment, X may be an FKBP12 protein or a fragment thereof; or cyclophilin A (CYPA) or a fragment thereof. In addition, X may be selected from a protein involved in the antigen loss-mediated relapse. In one embodiment, X may be any one selected from the group consisting of TC21 or a fragment thereof; RHOG or a fragment thereof. In addition, X may be selected from a protein located in the T cell stimulation pathway. In one embodiment, X may be any one selected from the group consisting of NCK1 or a fragment thereof; LAT or a fragment thereof; NEMO or a fragment thereof; a TLR4 intracellular domain or a fragment thereof; GADD45α or a fragment thereof; CDC42 or a fragment thereof; and HRAS or a fragment thereof. In addition, X may be selected from a protein involved in the inhibition of negative feedback. In one embodiment, X may be a C-terminal domain of a SOCS1 protein or a fragment thereof.

Specifically, Y may be selected from a protein located in the immunosuppressive signaling pathway. In one embodiment, Y may be any one selected from the group consisting of an FKBP12 protein or a fragment thereof; a C-terminal MH2 domain of an SMAD4 protein, or a fragment thereof; N-SKI or a fragment thereof; an N-SH2 domain of a SHP-1 protein, or a fragment thereof; and an N-SH2 domain of a SHP-2 protein, or a fragment thereof. In addition, Y may be an immunophilin. In one embodiment, Y may be an FKBP12 protein or a fragment thereof; or cyclophilin A (CYPA) or a fragment thereof. In addition, Y may be selected from a protein involved in the antigen loss-mediated relapse. In one embodiment, Y may be any one selected from the group consisting of TC21 or a fragment thereof; and RhoG or a fragment thereof. In addition, Y may be selected from a protein located in the T cell stimulation pathway. In one embodiment, Y may be any one selected from the group consisting of NCK1 or a fragment thereof; LAT or a fragment thereof; NEMO or a fragment thereof; an intracellular domain of TLR4, or a fragment thereof; GADD45α or a fragment thereof; CDC42 or a fragment thereof; HRAS or a fragment thereof. In addition, Y may be selected from a protein involved in the inhibition of negative feedback. In one embodiment, Y may be a C-terminal domain of a SOCS1 protein, or a fragment thereof.

The two signaling pathway modulators may include an immunophilin. Specifically, the FKBP12 protein or a fragment thereof; or cyclophilin A may be included.

Specific examples of the combination of the two signaling pathway modulators may be a FKBP12 protein or a fragment thereof, and cyclophilin A. In addition, in one embodiment, it may be a FKBP12 protein or a fragment thereof, and an N-SH2 domain of a SHP-1 protein or a fragment thereof. In addition, in one embodiment, it may be a FKBP12 protein or a fragment thereof, and an N-SH2 domain of a SHP-2 protein or a fragment thereof. In addition, in one embodiment, it may be a FKBP12 protein or a fragment thereof, and a C-terminal domain of a SOCS1 protein or a fragment thereof. In addition, in one embodiment, it may be cyclophilin A and an N-SH2 domain of a SHP-1 protein, or a fragment thereof. In addition, in one embodiment, it may be cyclophilin A and an N-SH2 domain of a SHP-2 protein, or a fragment thereof.

### Combination of three signaling pathway modulators

The signaling pathway modulator may have the structure of the following structural formula (II): $N ′ - X - L 1 - Y - L 2 - Z - C ′$ wherein, in the structural formula (II),
N' is an N-terminal of a fusion protein,
C' is a C-terminal of a fusion protein, and
each of X, Y and Z may be any one selected from the group consisting of a) a protein located in the immunosuppressive signaling pathway, or a fragment thereof; b) an immunophilin or a fragment thereof; c) a protein involved in the antigen loss-mediated relapse, or a fragment thereof; d) a protein located in the T cell stimulation pathway, or a fragment thereof; and e) a protein involved in the inhibition of negative feedback, or a fragment thereof.

Each of L1 and L2 may be a self-cleavage peptide or an IRES.

Specifically, X may be selected from a protein located in the immunosuppressive signaling pathway. In one embodiment, X may be any one selected from the group consisting of a FKBP12 protein or a fragment thereof; a C-terminal MH2 domain of SMAD4, or a fragment thereof; N-SKI or a fragment thereof; an N-SH2 domain of a SHP-1 protein, or a fragment thereof; and an N-SH2 domain of a SHP-2 protein, or a fragment thereof. In addition, X may be an immunophilin. In one embodiment, X may be a FKBP12 protein or a fragment thereof; or cyclophilin A (CYPA) or a fragment thereof. In addition, X may be selected from a protein involved in the antigen loss-mediated relapse. In one embodiment, X may be any one selected from the group consisting of TC21 or a fragment thereof; and RhoG or a fragment thereof. In addition, X may be selected from a protein located in the T cell stimulation pathway. In one embodiment, X may be any one selected from the group consisting of NCK1 or a fragment thereof; LAT or a fragment thereof; NEMO or a fragment thereof; a TLR4 intracellular domain or a fragment thereof; GADD45α or a fragment thereof; CDC42 or a fragment thereof; HRAS or a fragment thereof. In addition, X may be selected from a protein involved in the inhibition of negative feedback. In one embodiment, X may be a C-terminal domain of a SOCS1 protein or a fragment thereof.

Specifically, Y may be selected from a protein located in the immunosuppressive signaling pathway. In one embodiment, Y may be any one selected from the group consisting of a FKBP12 protein or a fragment thereof; a C-terminal MH2 domain of a SMAD4 protein, or a fragment thereof; N-SKI or a fragment thereof; an N-SH2 domain of a SHP-1 protein, or a fragment thereof; and an N-SH2 domain of a SHP-2 protein, or a fragment thereof. In addition, Y may be an immunophilin. In one embodiment, Y may be a FKBP12 protein or a fragment thereof; or cyclophilin A (CYPA) or a fragment thereof. In addition, Y may be selected from a protein involved in the antigen loss-mediated relapse. In one embodiment, Y may be any one selected from the group consisting of TC21 or a fragment thereof; and RhoG or a fragment thereof. In addition, Y may be selected from a protein located in the T cell stimulation pathway. In one embodiment, Y may be any one selected from the group consisting of NCK1 or a fragment thereof; LAT or a fragment thereof; NEMO or a fragment thereof; an intracellular domain of TLR4, or a fragment thereof; GADD45α or a fragment thereof; CDC42 or a fragment thereof; HRAS or a fragment thereof. In addition, Y may be selected from a protein involved in the inhibition of negative feedback. In one embodiment, Y may be a C-terminal domain of a SOCS1 protein, or a fragment thereof.

Specifically, Z may be selected from a protein located in the immunosuppressive signaling pathway. In one embodiment, Z may be any one selected from the group consisting of a FKBP12 protein or a fragment thereof; a C-terminal MH2 domain of a SMAD4 protein, or a fragment thereof; N-SKI or a fragment thereof; an N-SH2 domain of a SHP-1 protein, or a fragment thereof; and an N-SH2 domain of a SHP-2 protein, or a fragment thereof. In addition, Z may be an immunophilin. In one embodiment, Z may be a FKBP12 protein or a fragment thereof; or cyclophilin A (CYPA) or a fragment thereof. In addition, Z may be selected from a protein involved in the antigen loss-mediated relapse. In one embodiment, Z may be any one selected from the group consisting of TC21 or a fragment thereof; and RhoG or a fragment thereof. In addition, Z may be selected from a protein located in the T cell stimulation pathway. In one embodiment, Z may be any one selected from the group consisting of NCK1 or a fragment thereof; LAT or a fragment thereof; NEMO or a fragment thereof; an intracellular domain of TLR4 or a fragment thereof; GADD45α or a fragment thereof; CDC42 or a fragment thereof; HRAS or a fragment thereof. In addition, Z may be selected from a protein involved in the inhibition of negative feedback. In one embodiment, Z may be a C-terminal domain of a SOCS1 protein or a fragment thereof.

The three signaling pathway modulators can be in various combinations. Preferably, at least one of the three signaling pathway modulators may be an immunophilin.

Specific examples of the combination of the three signaling pathway modulators may be a FKBP12 protein or a fragment thereof, cyclophilin A, and an N-SH2 domain of a SHP-2 protein or a fragment thereof. In addition, in one embodiment, it may be a FKBP12 protein or a fragment thereof, cyclophilin A, and an N-SH2 domain of a SHP-1 protein or a fragment thereof.

### Chimeric antigen receptor

The term "antigen binding domain" used in the present invention refers to a site of an antibody that binds to an antigen. The antigen binding domain may be an antibody or an antigen binding fragment thereof. Preferably, the antigen binding domain may be an antigen binding fragment. In addition, the antigen binding fragment may be a fragment having one antigen binding site by linking one heavy chain and one light chain in an antibody by a disulfide bond. The antigen binding fragment may be any one selected from the group consisting of scFv, Fab and Fab'. Preferably, the antigen binding fragment may be scFv. In one embodiment, scFvwas used as an antigen binding domain.

The antigen binding domain may specifically bind to any one antigen selected from the group consisting of alpha folate receptor (examples of target tumors: ovarian cancer, gastric cancer and the like), 5T4 (kidney cancer, prostate cancer and the like), αvβ6 integrin (ovarian cancer, pancreatic cancer and the like), BCMA (multiple myeloma), B7-H3 (brain cancer, osteosarcoma and the like), B7-H6 (lymphoma, melanoma and the like), CAIX (kidney cancer, glioblastoma and the like), CD16 (IgG-opsonized tumor), CD19 (leukemia, lymphoma), CD20 (leukemia, lymphoma), CD22 (leukemia, lymphoma), CD30 (leukemia, lymphoma), CD33 (leukemia), CD43 (leukemia), CD44 (liver cancer), CD44v6 (leukemia, multiple myeloma, ovarian cancer and the like), CD44v7/8 (uterine cancer), CD47 (leukemia, lymphoma, ovarian cancer, pancreatic cancer and the like), CD70 (leukemia, lymphoma, neuroma), CD79a (leukemia, lymphoma), CD79b (leukemia, lymphoma), CD123 (leukemia), CD138 (multiple myeloma), CD171 (neuroblastoma), CEA (colorectal cancer, gastric cancer, lung cancer), CSPG4 (leukemia, glioblastoma), EGFR (breast cancer, lung cancer), EGFR family including ErbB2 (HER2) (glioblastoma, sarcoma), EGFRvIII (glioblastoma), EGP2 (colorectal cancer), EGP40 (colorectal cancer), EPCAM (colorectal cancer), EphA2 (esophageal cancer), FAP (mesothelioma, lung cancer, colorectal cancer), fetal AchR (rhabdomyosarcoma), FRα (ovarian cancer, breast cancer), GD2 (neuroblastoma), GD3 (melanoma, neuroma and the like), glypican-3 (GPC3) (liver cancer), HLA-A1+MAGE1 (melanoma), HLA-A2+MAGE1 (melanoma), HLA-A3+MAGE1 (melanoma), HLA-A1+NY-ESO-1 (synovial sarcoma, melanoma), HLA-A2+NY-ESO-1 (synovial sarcoma, melanoma), HLA-A3+NY-ESO-1 (synovial sarcoma, melanoma), IL-11Rα (osteosarcoma), IL-13Rα2 (neuroma), lambda, Lewis-Y, kappa (lymphoma), mesothelin (mesothelioma, pancreatic cancer and the like), Muc1 (breast cancer), Muc16 (ovarian cancer), NCAM (neuroblastoma, lung cancer), NKG2D ligand (leukemia, multiple myeloma), NY-ESO-1 (multiple myeloma), PRAME (medullary cell tumor), PSCA (prostate cancer), PSMA (prostate cancer), ROR1 (lung cancer, breast cancer), SSX (synovial sarcoma), survivin (lung cancer), TAG72 (ovarian cancer), TEMs, VEGFR2 (melanoma, kidney cancer), and WT-1 (leukemia). Specifically, the antigen binding domain may specifically bind to CD19, HER2, PSMA, CD43, and CD47.

### Chimeric antigen receptor: CD19

An antigen binding domain specific for CD19 may include a fragment of an antibody that specifically binds to CD19. In one embodiment of the present invention, the antigen binding domain specific for CD19 may include a light chain variable region including L-CDR1 (SEQ ID NO: 73), L-CDR2 (SEQ ID NO: 74) and L-CDR3 (SEQ ID NO: 75). In addition, the antigen binding domain specific for CD19 may include a heavy chain variable region including H-CDR1 (SEQ ID NO: 76), H-CDR2 (SEQ ID NO: 77) and H-CDR3 (SEQ ID NO: 78). In addition, the antigen binding domain specific for CD19 may include the amino acid sequence represented by SEQ ID NO: 3. In addition, a nucleotide sequence encoding the antigen binding domain specific for CD19 may be the nucleotide sequence represented by SEQ ID NO: 4.

### Chimeric antigen receptor: HER2

An antigen binding domain specific for Her2 may include a fragment of an antibody that specifically binds to Her2. In one embodiment of the present invention, the antigen binding domain specific for Her2 may include a light chain variable region including L-CDR1 (SEQ ID NO: 79), L-CDR2 (SEQ ID NO: 80) and L-CDR3 (SEQ ID NO: 81). In addition, the antigen binding domain specific for Her2 may include a heavy chain variable region including H-CDR1 (SEQ ID NO: 82), H-CDR2 (SEQ ID NO: 83) and H-CDR3 (SEQ ID NO: 84). In addition, the antigen binding domain specific for Her2 may include the amino acid sequence represented by SEQ ID NO: 45. In addition, a nucleotide sequence encoding the antigen binding domain specific for Her2 may be the nucleotide sequence represented by SEQ ID NO: 46.

### Chimeric antigen receptor: PSMA

An antigen binding domain specific for PSMA may include a fragment of an antibody that specifically binds to PSMA. In one embodiment of the present invention, the antigen binding domain specific for PSMA may include a light chain variable region including L-CDR1 (SEQ ID NO: 85), L-CDR2 (SEQ ID NO: 86) and L-CDR3 (SEQ ID NO: 87). In addition, the antigen binding domain specific for PSMA may include a heavy chain variable region including H-CDR1 (SEQ ID NO: 88), H-CDR2 (SEQ ID NO: 89) and H-CDR3 (SEQ ID NO: 90). In addition, the antigen binding domain specific for PSMA may include the amino acid sequence represented by SEQ ID NO: 54. In addition, a nucleotide sequence encoding the antigen binding domain specific for PSMA may be the nucleotide sequence represented by SEQ ID NO: 55.

### Chimeric antigen receptor: CD43

An antigen binding domain specific for CD43 may include a fragment of an antibody that specifically binds to CD43. In one embodiment of the present invention, the antigen binding domain specific for CD43 may include a light chain variable region including L-CDR1 (SEQ ID NO: 91), L-CDR2 (SEQ ID NO: 92) and L-CDR3 (SEQ ID NO: 93). In addition, the antigen binding domain specific for CD43 may include a heavy chain variable region including H-CDR1 (SEQ ID NO: 94), H-CDR2 (SEQ ID NO: 95) and H-CDR3 (SEQ ID NO: 96). In addition, the antigen binding domain specific for CD43 may include the amino acid sequence represented by SEQ ID NO: 47. In addition, a nucleotide sequence encoding the antigen binding domain specific for CD43 may be the nucleotide sequence represented by SEQ ID NO: 48.

### Chimeric antigen receptor: CD47

An antigen binding domain specific for CD47 may include a fragment of an antibody that specifically binds to CD47. In one embodiment of the present invention, the antigen binding domain specific for CD47 may include a light chain variable region including L-CDR1 (SEQ ID NO: 100), L-CDR2 (SEQ ID NO: 101) and L-CDR3 (SEQ ID NO: 102). In addition, the antigen binding domain specific for CD47 may include a heavy chain variable region including H-CDR1 (SEQ ID NO: 97), H-CDR2 (SEQ ID NO: 98) and H-CDR3 (SEQ ID NO: 99). In addition, the antigen binding domain specific for CD47 may include the amino acid sequence represented by SEQ ID NO: 50. In addition, a nucleotide sequence encoding the antigen binding domain specific for CD47 may be the nucleotide sequence represented by SEQ ID NO: 51.

### Transmembrane domain

The term "transmembrane domain" used in the present invention refers to a site that connects the domain to which an antigen binds and the domain that transmits an intracellular signal,region and penetrates a cell membrane among a structure of a protein located in a cell membrane, and allows the protein located in a cell membrane to be immobilized on the cell membrane. The transmembrane domain may be derived from any one selected from the group consisting of T-cell receptor, CD3δ, CD3ε, CD3γ, CD3ζ, CD4, CD5, CD8α, CD9, CD16, CD22, CD27, CD28, CD33, CD37, CD45, CD64, CD80, CD86, CD134, CD137, CD152, CD154, AMN and PD-1. Specifically, the transmembrane domain may be derived from CD8α.

In one embodiment of the present invention, a spacer and transmembrane domain consisting of the amino acid sequence represented by SEQ ID NO: 5 derived from CD8α was used. In addition, a nucleotide sequence encoding the spacer and transmembrane domain may be the nucleotide sequence represented by SEQ ID NO: 6.

### Spacer

As described above, an antigen binding domain and a transmembrane domain may be connected through a spacer. The spacer refers to a linker, and may be a protein or peptide. In addition, it may be composed of 1 to 1,000 amino acids, and may be composed of 10 to 300 amino acids. In addition, it may be composed of 15 to 100 amino acids, and may be composed of 15 to 60 amino acids. In addition, the spacer may be composed of 15 to 45 amino acids. In addition, the linker may be a fragment of a protein in human body such as an Fc region. In addition, the linker may be derived from any one selected from the group consisting of CD35, CD3ε, CD3γ, CD3ζ, CD4, CD5, CD8α, CD9, CD16, CD22, CD27, CD28, CD33, CD37, CD45, CD64, CD80, CD86, CD134, CD137, CD152, CD154, AMN and PD-1. In addition, the spacer may include an amino acid sequence derived from a hinge of CD8α.

### Intracellular signaling domain

The term "an intracellular signaling domain" used in the present invention refers to a region that transmits a signal inside of a cell in order to induce a response such as activation of cells, release of cytotoxic factors, cytokine production, proliferation and the like when an antigen receptor (antigen binding domain) present on the cell surface recognizes an extracellular antigen. In addition, in general, since a signal transmitted through one antigen receptor (antigen binding domain) is insufficient for activation of cells, a secondary or co-stimulatory signal is required. Therefore, the intracellular signaling domain may include a primary signaling domain and a secondary signaling domain and/or a co-stimulatory domain. Specifically, the intracellular signaling domain may include a co-stimulatory domain and a primary signaling domain.

The co-stimulatory domain may be derived from at least one molecule selected from the group consisting of TLR1, TLR2, TLR3, TLR4, TLR5, TLR6, TLR7, TLR8, TLR9, TLR10, CARD11, CD2, CD7, CD27, CD28, CD30, CD40, CD54 (ICAM), CD83, CD134 (OX40), CD137 (4-1BB), CD278 (ICOS), DAP10, LAT, NKD2C, SLP76, TRIM and ZAP70. Specifically, the co-stimulatory domain may be derived from CD137 (4-1BB).

In one embodiment of the present invention, a co-stimulatory domain consisting of the amino acid sequence represented by SEQ ID NO: 7 derived from CD137 (4-1BB) was used. In addition, a nucleotide sequence encoding the co-stimulatory domain may be the nucleotide sequence represented by SEQ ID NO: 8.

The primary signaling domain may be derived from FcRγ, FcRβ, CD3γ, CD3δ, CD3ε, CD3ζ, CD22, CD79a, CD79b or CD66d. In particular, T cells transmit a signal inside of a cell through the γ, δ, ε, ζ chains of CD3, and when CAR-T cells (Chimeric antigen receptor T cells) are constructed, the γ, δ, ε, ζ chains of CD3 may be used as a primary signaling domain. Specifically, the primary signaling domain may be derived from CD3ζ.

In one embodiment of the present invention, a primary signaling domain consisting of the amino acid sequence represented by SEQ ID NO: 9 derived from CD3ζ was used. In addition, a nucleotide sequence encoding the primary signaling domain may be the nucleotide sequence represented by SEQ ID NO: 10.

The intracellular signaling domain may be appropriately combined. In one embodiment, the intracellular signaling domain may include CD137 (4-1BB) and CD3ζ.

### Self-cleavage peptide

The term "self-cleavage peptide" used in the present invention refers to a peptide consisting of 10 to 50, 12 to 42, 14 to 34, 16 to 26, or 18 to 22 amino acids capable of inducing cleavage of proteins synthesized in the cell. The self-cleavage peptide may be derived from 2A region of a viral gene. The self-cleavage peptide may be derived from P2A, E2A, F2A, or T2A. Specifically, the self-cleavage peptide may be derived from P2A. In addition, instead of the self-cleavage peptide, a peptide cleaved by a protease present in the cytoplasm may be used.

In one embodiment of the present invention, a self-cleavage peptide consisting of the amino acid sequence represented by SEQ ID NO: 11 or 56 derived from P2A was used. In addition, a nucleotide sequence encoding the self-cleavage peptide may be the nucleotide sequence represented by SEQ ID NO: 12.

### Specific examples of fusion proteins

Specific examples of the fusion proteins may be as follows:
(1) N-terminus - signal peptide - antigen binding domain - hinge - transmembrane domain - costimulatory domain - primary signaling domain - self-cleavage peptide - signaling pathway modulator (FKBP12 or fragment thereof) - C-terminus;
(2) N-terminus - signal peptide - antigen binding domain - hinge - transmembrane domain - costimulatory domain - primary signaling domain- co-stimulatory domain - self-cleavage peptide - signaling pathway modulator (cyclophilin A) - C-terminus;
(3) N-terminus - signal peptide - antigen binding domain - hinge - transmembrane domain - costimulatory domain - primary signaling domain - self-cleavage peptide - signaling pathway modulator (N-SH2 domain of SHP-1 protein) - C-terminus;
(4) N-terminus - signal peptide - antigen binding domain - hinge - transmembrane domain - costimulatory domain - primary signaling domain - self-cleavage peptide - signaling pathway modulator (N-SH2 domain of SHP-2 protein) - C-terminus;
(5) N-terminus - signal peptide - antigen binding domain - hinge - transmembrane domain - costimulatory domain - primary signaling domain - self-cleavage peptide - signaling pathway modulator (C-terminal MH2 domain of SMAD4 protein or fragment thereof) - C-terminus;
(6) N-terminus - signal peptide - antigen binding domain - hinge - transmembrane domain - costimulatory domain - primary signaling domain - self-cleavage peptide - signaling pathway modulator (N-SKI or fragment thereof) - C-terminus;
(7) N-terminus - signal peptide - antigen binding domain - hinge - transmembrane domain - costimulatory domain - primary signaling domain - self-cleavage peptide - signaling pathway modulator (FKBP12 or fragment thereof) - self-cleavage peptide - signaling pathway modulator (cyclophilin A) - C-terminus;
(8) N-terminus - signal peptide - antigen binding domain - hinge - transmembrane domain - costimulatory domain - primary signaling domain - self-cleavage peptide - signaling pathway modulator (fragment of FKBP12) - self-cleavage peptide - signaling pathway modulator (N-SH2 domain of SHP-2 protein)- C-terminus;
(9) N-terminus - signal peptide - antigen binding domain - hinge - transmembrane domain - costimulatory domain - primary signaling domain - self-cleavage peptide - signaling pathway modulator (cyclophilin A) - self-cleavage peptide - signaling pathway modulator (N-SH2 domain of SHP-2 protein)- C-terminus;
(10) N-terminus - signal peptide - antigen binding domain - hinge - transmembrane domain - costimulatory domain - primary signaling domain - self-cleavage peptide - signaling pathway modulator (FKBP12 or fragment thereof) - self-cleavage peptide - signaling pathway modulator (cyclophilin A) - self-cleavage peptide - signaling pathway modulator (N-SH2 domain of SHP-2 protein) - C-terminus;
(11) N-terminus - signaling pathway modulator (FKBP12 or fragment thereof) - self-cleavage peptide - signal peptide - antigen binding domain - hinge - transmembrane domain - co-stimulatory domain - primary signaling domain - C-terminus;
(12) N-terminus - signaling pathway modulator (cyclophilin A) - self-cleavage peptide - signal peptide - antigen binding domain - hinge - transmembrane domain - co-stimulatory domain - primary signaling domain - co-stimulatory domain - C-terminus;
(13) N-terminus - signaling pathway modulator (N-SH2 domain of SHP-1 protein) - self-cleavage peptide - signal peptide - antigen binding domain - hinge - transmembrane domain - co-stimulatory domain - primary signaling domain - C-terminus;
(14) N-terminus - signaling pathway modulator (N-SH2 domain of SHP-2 protein) - self-cleavage peptide - signal peptide - antigen binding domain - hinge - transmembrane domain - co-stimulatory domain - primary signaling domain - C-terminus;
(15) N-terminus - signaling pathway modulator (C-terminal MH2 domain of SMAD4 protein or fragment thereof) - self-cleavage peptide - signal peptide - antigen binding domain - hinge - transmembrane domain - co-stimulatory domain - primary signaling domain - C-terminus;
(16) N-terminus - signaling pathway modulator (N-SKI or fragment thereof) - self-cleavage peptide - signal peptide - antigen binding domain - hinge - transmembrane domain - co-stimulatory domain - primary signaling domain - C-terminus;
(17) N-terminus - signaling pathway modulator (N-SKI or fragment thereof) - self-cleavage peptide - signaling pathway modulator (cyclophilin A) - self-cleavage peptide - signal peptide - antigen binding domain - hinge - transmembrane domain - co-stimulatory domain - primary signaling domain - C-terminus;
(18) N-terminus - signaling pathway modulator (FKBP12 or fragment thereof) - self-cleavage peptide - signaling pathway modulator (N-SH2 domain of SHP-2 protein) - self-cleavage peptide - signal peptide - antigen binding domain - hinge - transmembrane domain - co-stimulatory domain - primary signaling domain - C-terminus;
(19) N-terminus - signaling pathway modulator (cyclophilin A) - self-cleavage peptide - signaling pathway modulator (N-SH2 domain of SHP-2 protein) - self-cleavage peptide - signal peptide - antigen binding domain - hinge - transmembrane domain - co-stimulatory domain - primary signaling domain - C-terminus;
(20) N-terminus - signaling pathway modulator (FKBP12 or fragment thereof) - self-cleavage peptide - signaling pathway modulator (cyclophilin A) - self-cleavage peptide - signaling pathway modulator (N-SH2 domain of SHP-2 protein) - self-cleavage peptide - signal peptide - antigen binding domain - hinge - transmembrane domain - co-stimulatory domain - primary signaling domain - C-terminal.

In this case, in the formulas (1) to (20) above, an antigen binding domain, a hinge, a transmembrane domain, a co-stimulatory domain, a primary signaling domain, a self-cleavage peptide and a signaling pathway modulator are as described above. Specifically, in the formulas (1) to (20) above, the antigen binding domain may be a scFv specific for CD19, a scFv specific for Her2, a scFv specific for PSMA, a scFv specific for CD43, a scFv specific for CD47, and the transmembrane domain may be derived from CD8α, and the co-stimulatory domain may be derived from CD137 (4-1BB), and the primary signaling domain may be derived from CD3ζ, and the self-cleavage peptide may be derived from P2A.

In one embodiment of the present invention, it was designed to include an antigen binding domain (CD19 scFv), a transmembrane domain (CD8α), an intracellular signaling domain (4-1BB and CD3Q, a self-cleavage peptide (P2A) and FKBP12, and it was referred to as "19bbz#F". In addition to the above, a signaling pathway modulators and notation are as described above.

### Polynucleotide encoding a fusion protein

As used herein, the term "polynucleotide" refers to deoxyribonucleic acid (DNA), ribonucleic acid (RNA), and a DNA/RNA hybrid. A polynucleotide is single-stranded or double-stranded, and may be recombined, synthesized, or isolated. The polynucleotide includes pre-messenger RNA (pre-mRNA), messenger RNA (mRNA), RNA, genomic RNA (gRNA), plus strand RNA (RNA (+)), minus strand RNA (RNA (-)), synthetic RNA, synthetic mRNA, genomic DNA (gDNA), PCR-amplified DNA, complementary DNA (cDNA), synthetic DNA or recombinant DNA, but is not limited thereto.

The polynucleotide may be codon-optimized. As used herein, the term "codon-optimized" refers to substituting a codon in a polynucleotide encoding a polypeptide in order to increase the expression, stability and/or activity of the polypeptide. Factors influencing codon optimization include (i) modification of the degree of codon bias within an organism, gene or set of genes, (ii) systematic modification of a codon including context, (iii) modification of codons according to their decoding tRNA, (iv) modification of a codon according to GC% overall or at one of the three positions, (v) modification of the degree of similarity with a reference sequence, for example a naturally derived sequence, (vi) modification in the codon frequency cutoff, (vii) structural properties of the transcribed mRNA from the DNA sequence, (viii) prior knowledge of the function of the DNA sequence based on the design of the codon substitution set, (ix) systematic modification of the codon set for each amino acid, and/or (x) isolated removal of a nonlogical translation initiation site, but are not limited thereto.

### Vector including a polynucleotide encoding a fusion protein

Another aspect of the present invention provides a vector including the polynucleotide.

The polynucleotide is as described above. The polynucleotide may be prepared, engineered, expressed and delivered using any of a variety of established techniques known and available in the art. In order to express the desired fusion protein, a polynucleotide encoding a fusion protein may be inserted into an appropriate vector.

The vector can be used in a variety of vectors known in the art, and expression regulatory sequences such as a promoter, a terminator, an enhancer and the like, sequences for membrane targeting or secretion and the like can be appropriately selected depending on the type of host cell in which the antigen receptor is to be produced, and variously combined according to the purpose. The vector of the present invention includes a plasmid vector, a cosmid vector, a bacteriophage vector and a viral vector and the like, but is not limited thereto. Suitable vectors include expression regulatory elements such as a promoter, an operator, a start codon, a stop codon, a polyadenylation signal, and an enhancer, as well as signal peptides or leader sequences for membrane targeting or secretion, and can be variously prepared according to the purpose.

Preferably, the vector may be a viral vector, and the viral vector may be derived from a retrovirus, a lentivirus, an adenovirus, an adeno-associated virus, a herpesvirus, a poxvirus, a baculovirus, a papillomavirus, and a parvovirus. In one embodiment of the present invention, a lentiviral vector was used.

The vector may further include a sequence encoding a signal peptide in order to expose the antigen binding domain to the outside of the cell membrane, and in this case, the sequence encoding a signal peptide may be inserted prior to a sequence encoding an antigen binding domain. The signal peptide may consist of the amino acid sequence represented by SEQ ID NO: 1, and the nucleotide sequence encoding the same may be the sequence represented by SEQ ID NO: 2.

### Virus including a polynucleotide encoding a fusion protein

Another aspect of the present invention provides a virus including the polynucleotide. In this case, the polynucleotide is as described above. The virus may be a retrovirus, a lentivirus, an adenovirus, an adeno-associated virus, a herpesvirus, a poxvirus, a baculovirus, a papillomavirus, or a parvovirus.

### Cell expressing a polynucleotide encoding a fusion protein

Another aspect of the present invention provides an immune cell into which the vector is introduced. In this case, the vector is as described above. The immune cell may be a T cell or a natural killer cell.

A method for introducing the vector into an immune cell may use methods known in the art, and the vector can be introduced into a cell by, for example, transient transfection, microinjection, transduction, cell fusion, calcium phosphate precipitation, liposome-mediated transfection, DEAE Dextran-mediated transfection, polybrene-mediated transfection, electroporation, gene gun or other known methods for introducing nucleic acids into a cell (Wu et al., J. Bio. Chem., 267:963-967, 1992; Wu and Wu, J. Bio. Chem., 263:14621-14624, 1988). However, it is not limited thereto.

The transduced or transfected immune cells are proliferated ex *vivo* after a vector is introduced. In one embodiment, the transfected immune cells may be cultured for at least about 1 day, 2 days, 3 days, 4 days, 5 days, 6 days, 7 days, 8 days, 9 days, 10 days, 11 days, 12 days, 13 days or 14 days so as to proliferate, and may be preferably cultured for 12 days to 14 days.

Methods for confirming whether the vector has been well introduced into the immune cells include, for example, molecular biological assays well known to those skilled in the art, for example, Southern and Northern blotting, RT-PCR and PCR; biochemical assay, for example, as an example, detection of the presence or absence of specific peptides by immunological method (for example, ELISAs and Western blots).

When the fusion protein is expressed in an immune cell, due to a self-cleavage peptide of (iv), a signaling pathway modulator (e.g., FKBP12 or a fragment thereof) in (i) to (iii) corresponding to the chimeric antigen receptor (CAR) in the cytoplasm may be separated, and in the case of a chimeric antigen receptor, it may be immobilized on a cell membrane to recognize an extracellular antigen and transmit a signal into the cell. In addition, a signaling pathway modulator, for example, FKBP12 can block the signaling pathway of TGF-β by binding to TGFβR1.

### Polynucleotide including IRES

Another aspect of the present invention provides a polynucleotide including (i) a polynucleotide encoding an antigen binding domain; (ii) a polynucleotide encoding a transmembrane domain; (iii) a polynucleotide encoding an intracellular signaling domain including at least one co-stimulatory domain; (iv) a polynucleotide encoding an IRES (Internal Ribosome Entry Site); and (v) a polynucleotide encoding a signaling pathway modulator.

In this case, a polynucleotide encoding a spacer may be further included between the polynucleotide encoding an antigen binding domain and the polynucleotide encoding a transmembrane domain.

The antigen binding domain, the transmembrane domain, the intracellular signaling domain and the signaling pathway modulator are as described above.

As used herein, the term "IRES" is an abbreviation of internal ribosome entry site. The IRES is a nucleic acid used to simultaneously express two genes.

Another aspect of the present invention provides an expression vector including the polynucleotide. In this case, the vector and the components included therein are as described above.

Another aspect of the present invention provides a virus including the polynucleotide. In this case, the virus and the components included therein are as described above.

Another aspect of the present invention provides an immune cell into which the polynucleotide is introduced. In this case, a method of introducing an immune cell and a polynucleotide is as described above.

### Polynucleotide that does not include a self-cleavage sequence or an IRES

Another aspect of the present invention provides a polynucleotide encoding a fusion protein that includes (i) an antigen binding domain; (ii) a transmembrane domain; (iii) an intracellular signaling domain including at least one co-stimulatory domain; (iv) a signaling pathway modulator.

In this case, the fusion protein may further include a spacer between (i) the antigen binding domain and (ii) the transmembrane domain.

In this case, the signaling pathway modulator may be a protein located in the T cell stimulation pathway. In this case, the protein located in the T cell stimulation pathway may be a protein located in the TNFR/TLR receptor pathway, or a fragment thereof. Specifically, the protein located in the TNFR/TLR receptor pathway may be a TLR4 intracellular domain (hereinafter, a TLR4 intracellular domain is referred to as T).

In this case, the protein located in the T cell stimulation pathway may be a protein located in the cytokine receptor (JAK-STAT) pathway or a fragment thereof. Specifically, the protein located in the cytokine receptor (JAK-STAT) pathway may be γc (hereinafter, γc is referred to as γ).

In this case, when a self-cleavage peptide is not included, the intracellular signal may be regulated while a signaling pathway modulator is bound to CAR.

Another aspect of the present invention provides an expression vector including the polynucleotide. The expression vector is as described above. The vector may further include a sequence encoding a signal peptide in order to expose an antigen binding domain to the outside of the cell membrane, and in this case, the sequence encoding a signal peptide may be inserted prior to a sequence encoding an antigen binding domain. The signal peptide is as described above.

Another aspect of the present invention provides a virus including the polynucleotide. The virus is as described above.

Another aspect of the present invention provides an immune cell into which the polynucleotide is introduced. In this case, the immune cell is as described above.

### Immune cells engineered to overexpress signaling pathway modulator(s)

Another aspect of the present invention provides a transformed immune cell, characterized in that the transformed immune cells engineered to overexpress signaling pathway modulator(s).

In this case, the immune cell may be a T cell or an NK cell. In addition, the immune cell may be a transformed T cell or NK cell. In this case, the transformed T cell may be a CAR-T cell or a TCR-T cell.

The signaling pathway modulator is as described above. Specifically, the signaling pathway modulator may be any one selected from the group consisting of a FKBP12 protein or a fragment thereof; a C-MH2 domain of a SMAD4 protein or a fragment thereof; N-SKI or a fragment thereof; cyclophilin A (CYPA) or a fragment thereof; an N-SH2 domain of a SHP-1 protein, or a fragment thereof; and an N-SH2 domain of a SHP-2 protein, or a fragment thereof; TC21 or a fragment thereof; and RhoG or a fragment thereof; NCK1 or a fragment thereof; LAT or a fragment thereof; NEMO or a fragment thereof; a TLR4 intracellular domain or a fragment thereof; GADD45α or a fragment thereof; CDC42 or a fragment thereof; HRAS or a fragment thereof; and a C-terminal domain of a SOCS1 protein, or a fragment thereof.

In this case, the signaling pathway modulator is characterized in that it operates in the cytoplasm. In addition, a polynucleotide encoding the signaling pathway modulator is expressed in the cytoplasm, and the signaling pathway modulator is not secreted outside the cell. Therefore, the signaling pathway modulator may be located in an immune cell, and it may regulate the intracellular signaling pathway.

In addition, it may express two or three signaling pathway modulators.

### CAR-T immune cell

The chimeric antigen receptor (CAR) is made based on the scFv (single chain variable fragment) nucleotide sequence of a monoclonal antibody that recognizes a characteristic antigen expressed on the surface of cancer cells. Therefore, the T cells expressing the same specifically bind to a tumor antigen, and thus only the tumor cells can be removed. The structure of the CAR consists of a receptor region that binds to a tumor antigen, a co-stimulatory domain that activates T cells after the receptor binds to the antigen, and a spacer and transmembrane domain that connects the two regions.

In this case, the CAR-T cell may be characterized in that the CAR-T cell expresses a fusion protein including (i) an antigen binding domain; (ii) a transmembrane domain; and (iii) an intracellular signaling domain including at least one co-stimulatory domain.

In one embodiment, the transformed CAR-T immune cell may be prepared by transducing a polynucleotide including (i) a polynucleotide encoding an antigen binding domain; (ii) a polynucleotide encoding a spacer and transmembrane domain; (iii) a polynucleotide encoding an intracellular signaling domain including at least one co-stimulatory domain; (iv) a polynucleotide encoding a self-cleavage peptide; and (v) a polynucleotide encoding a signaling pathway modulator, as described above.

In addition, the transformed immune cell may be prepared in a manner of transducing a first polynucleotide including (i) an antigen binding domain; (ii) a spacer and transmembrane domain; (iii) an intracellular signaling domain including at least one co-stimulatory domain, and a second polynucleotide including a signaling pathway modulator, respectively. In this case, the first polynucleotide and the second polynucleotide may be introduced through one virus, but may also be introduced into immune cells through different viruses.

In this case, the signaling pathway modulator may be any one selected from the group consisting of a) a protein located in the immunosuppressive signaling pathway, b) an immunophilin, c) a protein involved in the antigen loss-mediated relapse, d) a protein located in the T cell stimulation pathway, e) a protein involved in the inhibition of negative feedback, and f) a combination thereof. Details on the signaling pathway modulators and a combination thereof are as described above.

In one embodiment, the immune cell may be a cell that overexpresses an immunophilin. Preferably, it may be a CAR-T cell that overexpresses FKBP12 or a fragment thereof and/or cyclophilin A.

In this case, the polynucleotide is as described above. In addition, a vector loaded with the polynucleotide may be introduced using various methods described above.

### TCR-T immune cell

The TCR-T cell refers to a T cell receptor-engineered T cell (TCR-T). TCR-T is an immune cell therapeutics that is prepared by introducing a gene of T cell receptor that recognizes a tumor-specific antigen peptide presented on the tumor cell surface through MHC molecules, thereby allowing the expressed TCR to recognize a specific tumor antigen and select an attack target. TCR-T is administered to the body after its ex vivo expansion.

TCR generally includes two polypeptide chains, for example the α-chain of TCR, the β-chain of TCR, the γ-chain of TCR, the δ-chain of TCR, or a combination thereof. The above-mentioned polypeptide chains of TCR are known in the art. Specifically, TCR-T may be one that expresses an antigen recognizable α-chain and β-chain.

For example, the α-chain or β-chain may include any amino acid sequence as long as it can specifically bind to a disease-related antigen or an epitope thereof to immunologically recognize the same. In addition, a viral vector may be used as a method for introducing the TCR gene into T cells. In this case, the viral vector is as described above.

In one embodiment, the transformed TCR-T immune cell may be prepared by transducing a polynucleotide including (i) a polynucleotide encoding the α-chain of TCR; (ii) a polynucleotide encoding a self-cleavage peptide; (iii) a polynucleotide encoding the β-chain of TCR; (iv) a polynucleotide encoding a self-cleavage peptide; and (v) a polynucleotide encoding a signaling pathway modulator, as described above.

In addition, the transformed immune cell may be prepared in a manner of transducing a first polynucleotide including (i) a polynucleotide encoding the α-chain of TCR; (ii) a polynucleotide encoding a self-cleavage peptide; (iii) a polynucleotide encoding the β-chain of TCR and a second polynucleotide including a signaling pathway modulator, respectively. In this case, the first polynucleotide and the second polynucleotide may be introduced into immune cells through different viruses.

In this case, the signaling pathway modulator may be any one selected from the group consisting of a) a protein located in the immunosuppressive signaling pathway, b) an immunophilin, c) a protein involved in the antigen loss-mediated relapse, d) a protein located in the T cell stimulation pathway, e) a protein involved in the inhibition of negative feedback, and f) a combination thereof. Details on the signaling pathway modulators and a combination thereof are as described above.

In one embodiment, the immune cell may be a cell that overexpresses an immunophilin. Preferably, it may be a TCR-T cell that overexpresses FKBP12 or a fragment thereof and/or cyclophilin A.

In this case, a method of transducing the polynucleotide may introduce a vector loaded with the polynucleotide as described above by using various methods described above.

### Pharmaceutical composition including the immune cells engineered to overexpress signaling pathway modulator(s)

Another aspect of the present invention provides a pharmaceutical composition for treating cancer, including the immune cells engineered to overexpress signaling pathway modulator(s) as an active ingredient.

In this case, the immune cells may be T cells, NK cells, and preferably CD8+ T cells or CD4+ T cells, or cells in which they are mixed in a certain ratio. In addition, the immune cells may be prepared by transducing a vector including the above-mentioned signaling pathway modulator that is externally introduced.

In this case, the cancer may be any one selected from the group consisting of gastric cancer, liver cancer, lung cancer, colorectal cancer, breast cancer, prostate cancer, ovarian cancer, pancreatic cancer, cervical cancer, thyroid cancer, laryngeal cancer, leukemia, acute myeloid leukemia, brain tumor, neuroblastoma, retinoblastoma, head and neck cancer, salivary gland cancer, lymphoma, kidney cancer, melanoma, multiple myeloma, brain cancer, osteosarcoma, glioblastoma, IgG-opsonized tumor, lymphoma, neuroma, mesothelioma, and esophageal cancer.

The type of cancer may be determined depending to an antigen binding domain. In one embodiment, CD19-specific immune cells may be used to treat hematologic cancer. Preferably, the hematologic cancer may be leukemia.

When the antigen binding domain specifically binds to a protein that is specifically overexpressed in cancer, the cancer may be targeted. In particular, when a protein that is specifically overexpressed in solid cancer is specifically recognized, it may be applied for the treatment of various solid cancers. In one embodiment, Her2-specific CAR-T cells may be utilized for the treatment of Her2 overexpressed cancer, and in particular, may be effectively utilized for Her2 (+) breast cancer. In addition, PSMA-specific CAR-T cells may be effectively utilized for the treatment of prostate cancer.

The "pharmaceutical composition" for immunotherapy in human patients described herein includes immune cells. In addition to the cells, other pharmaceutically acceptable salts, carriers, excipients, vehicles, and other additives and the like that can further improve the immune response may be added to the pharmaceutical composition.

"Effective amount" or "therapeutically effective amount" is used interchangeably herein, and refers to an amount of a compound, a preparation, a material or a composition, such as immune cells, as described herein that is effective to achieve a particular biological result. In one embodiment, CAR-T cells may include 1×10² to 1×10¹⁰, 1×10³ to 1×10⁸, and 1×10⁴ to 1×10⁶ cells when administered once, but are not limited thereto.

The cell therapy composition of the present invention can be administered in a conventional manner through rectal, intravenous, intraarterial, intraperitoneal, intramuscular, intrasternal, transdermal, topical, intracranial, intraocular or intradermal route.

### Method for treating or preventing cancer by using the immune cells expressing a signaling pathway modulator(s) that is externally introduced

Another aspect of the present invention provides a method for treating or preventing cancer, including a step of administering the immune cells engineered to overexpress a signaling pathway modulator(s) to a subject.

In this case, the signaling pathway modulator is as described above. In addition, the immune cells are as described above. In particular, the immune cells may include CAR-T cells and TCR-T cells.

### Use of immune cells expressing a signaling pathway modulator(s) that is externally introduced

Another aspect of the present invention provides use of immune cells overexpressing a signaling pathway modulator(s) that is externally introduced, for the treatment or prevention of cancer.

In this case, the signaling pathway modulator is as described above. In addition, the immune cells are as described above. In particular, the immune cells may include CAR-T cells and TCR-T cells.

Hereinafter, the characteristics of the CAR-T cells engineered to overexpress a signaling pathway modulator(s) prepared in one embodiment will be described in detail.

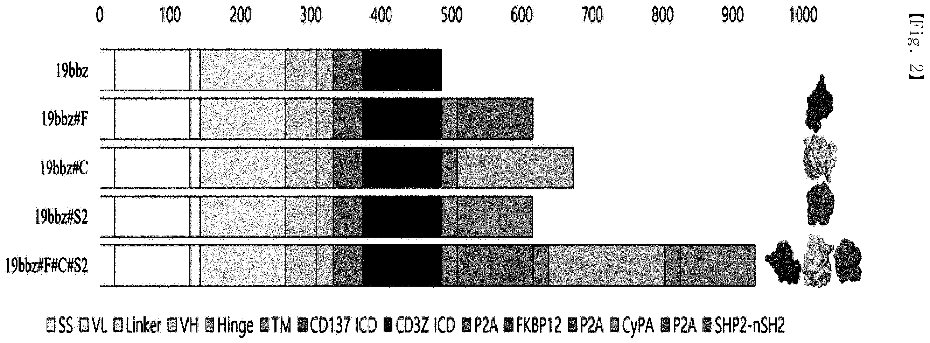

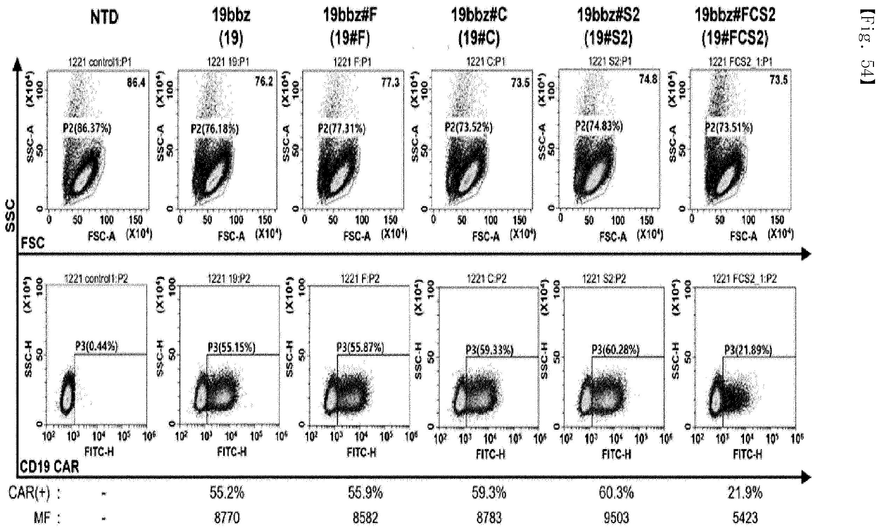

### Preparation of CD19-specific CAR-T cells in which a signaling pathway modulator(s) is expressed (Figures 2 and 54)

In general, "CD19-specific CAR-T (19bbz)" representing the conventional 2nd generation CAR-T cells, which has been confirmed to be useful in clinical practice and is currently most commonly used, was prepared and used in the experiment. In addition, an immune-regulatory cytoplasmic small-sized protein (hereinafter, a signaling pathway modulator), which is predicted to be capable of enhancing the anticancer function of CAR-T cells when overexpressed in CAR-T cells, was expressed through a lentivirus together with the CAR gene. As a specific example, two types of CAR-T cells (19bbz#F and 19bbz#C) expressing two immunophilins, FKBP12 (12kDa) and cyclophilin A (18kDa), respectively, were prepared, respectively.

The present inventor designed a signaling pathway modulator, which acts as a useful small-sized immune modulator, capable of inhibiting the signaling pathway of various inhibitory immune checkpoint molecules. In one embodiment, a small protein predicted to effectively inhibit the dephosphorylation of pTyr, which occurs very early and in common in the signaling pathways of various inhibitory immune checkpoint molecules, was designed. Specifically, CAR-T cells (19bbz#S2) overexpressing the N-terminal SH2 domain (12kDa) of SHP-2 as a small-sized modulator in the cytoplasm of CAT-T cellswere prepared.

The SHP2 protein (Src homology-2 domain-containing protein tyrosine phosphatase-2), together with the SHP1 protein, plays a very important role as a common factor in mediating about 100 kinds of various inhibitory immune checkpoint signals. From the crystal structural data of the SHP2 protein already published in 1998, taking notice of that the N-terminal SH2 domain of the SHP2 protein, which is a tyrosine dephosphorylation enzyme, actually plays a role as "a kind of lid that blocks the accessibility of the catalytic active site," the present inventor prepared the CAR-T cells (19bbz#S2) that overexpress the small-sized N-terminal SH2 domain in the cytoplasm.

In addition, it was confirmed that when the CAR-T cells (19bbz#FCS2) that simultaneously overexpress small-sized signaling pathway modulators having these other immune activating mechanisms were prepared, it was possible to prepare Super-armed CAR-T cells capable of exerting an anticancer activity even in immunosuppressive environment through the complementary synergy of these other signaling pathway modulators.

As shown in the FACS analysis, when the expression of CAR on the cell surface was confirmed through the binding of FITC-labeled recombinant CD19 protein to CAR-T cells on the 4th day after lentiviral transduction, it was confirmed that CAR may be made in all CAR-T cells as intended. In general, it is known that the efficiency of genome transmission through a lentiviral vector decreases as the size of a transgene to be transmitted increases. Therefore, in the case of lentiviral transduction that transmits three types of signaling pathway modulator genes together with the CAR gene, it was observed to show a relatively low ratio of a transduction rate compared to transduction that transmits one type of modulator with the CAR gene. However, then, through cell culture, in the case of 19bbz#FCS2, the ratio of CAR(+)-T cells was observed to reach a level of 40-50%.

As observed in this Example, in order to correct the difference in the transduction rate in the preparation of each CAR-T cell, non-transduced T cells (non-transduction mock T cells) immediately before performing *in vitro* function analysis (i.e., CAR (-)-T cells obtained through the same culture process without treatment with only lentivirus as T cells derived from peripheral blood cells of the same donor) were appropriately added, and in all CAR-T cell samples, "the number of T cells" and "the total number of T cells" were adjusted equally, and then a reaction with cancer cells was performed.

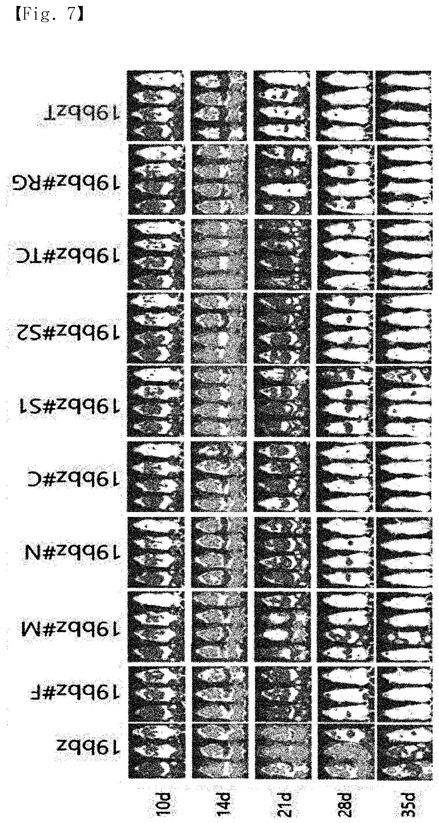

### Anti-tumor potency assessed in vivo for CD19-specific CAR-T cells (II) (Figure 7)

The anti-tumor potency of CAR-T cell_19bbz#F, CAR-T cell_19bbz#C, CAR-T cell_19bbz#M, CAR-T cell_19bbz#N, CAR-T cell_19bbz#S1, CAR-T cell_19bbz#S2, CAR-T cell_19bbz#TC, CAR-T cell_19bbz#RG and CAR-T cell_19bbzT was evaluated.

As a result, as shown in Figure 7, it was found that the control group treated with the conventional 2nd generation CAR-T cells (19bbz) did not effectively inhibit the growth of tumor so that rapid tumor growth was observed in all the three subjects of this control CAR T group.

On the other hand, it was observed that the group treated with CAR-T cells (19bbz#F) that overexpressed FKBP12, an immunophilin, and the group treated with CAR-T cells (19bbz#C) that overexpressed cyclophilin A, another immunophilin, showed an excellent efficacy in inhibiting the tumor progression. These results indicate that overexpressing an immunophilin is a very useful technique for augmenting the anticancer activity of immune cells.

In addition, an excellent efficacy in inhibiting tumor progression was also observed in the group treated with CAR-T cells (19bbz#F) that overexpressed FKBP12, the group treated with CAR-T cells (19bbz#M) that overexpressed the C-terminal MH2 domain of the SMAD4 protein, and the group treated with CAR-T cells (19bbz#N) that overexpressed N-SKI, as a cytoplasmic immune modulator capable of inhibiting TGF-β signaling. Therefore, these results indicate that overexpressing a modulator capable of blocking the signaling pathway of an immunosuppressive cytokine is a very useful technique for augmenting the anticancer activity of immune cells.

An excellent efficacy in inhibiting tumor progression was also observed in the group treated with CAR-T cells (19bbz#S1) that overexpressed the N-SH2 domain of the SHP1 protein, and the group treated with CAR-T cells (19bbz#S2) that overexpressed an N-SH2 domain of a SHP2 protein, as a cytoplasmic immune modulator capable of blocking various immunosuppressive checkpoint signaling pathways. Therefore, these results indicate that the overexpression of such specific modulators capable of impeding the activity of a pTyr phosphatase involved in the pathway of various immunosuppressive checkpoint signals, is a very useful technique for augmenting the anticancer activity of immune cells.

An excellent efficacy in inhibiting tumor progression was also observed in the group treated with CAR-T cells (19bbz#TC) that overexpressed TC21, and the group treated with CAR-T cells (19bbz#RG) that overexpressed RG, as a cytoplasmic immune modulator capable of inhibiting trogocytosis which is a process leading to target antigen loss in cancer cells. Therefore, these results indicate that the overexpression of such specific modulators capable of inhibiting trogocytosis is a very useful technique for augmenting the anticancer activity of immune cells.

An excellent efficacy in inhibiting tumor progression was also observed in the group treated with CAR-T cells (19bbzT) that overexpressed the intracellular domain of the TLR4 protein having the TIR domain (Toll/IL-1 Receptor homology domain), as an example of specific modulators belonging to the immune enhancing signaling system. Therefore, these results indicate that the technique of overexpressing a modulator that mediates immune enhancing signaling is a very useful technique for augmenting the anticancer activity of immune cells.

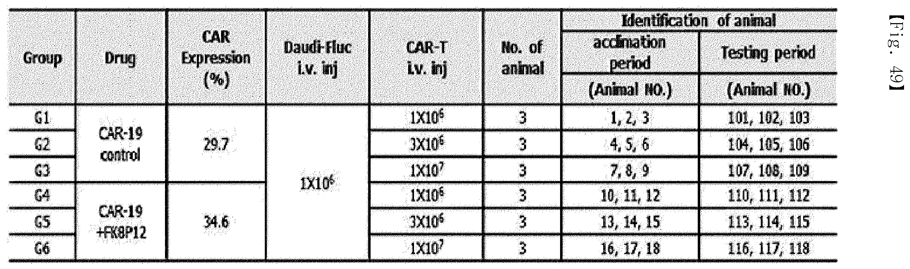

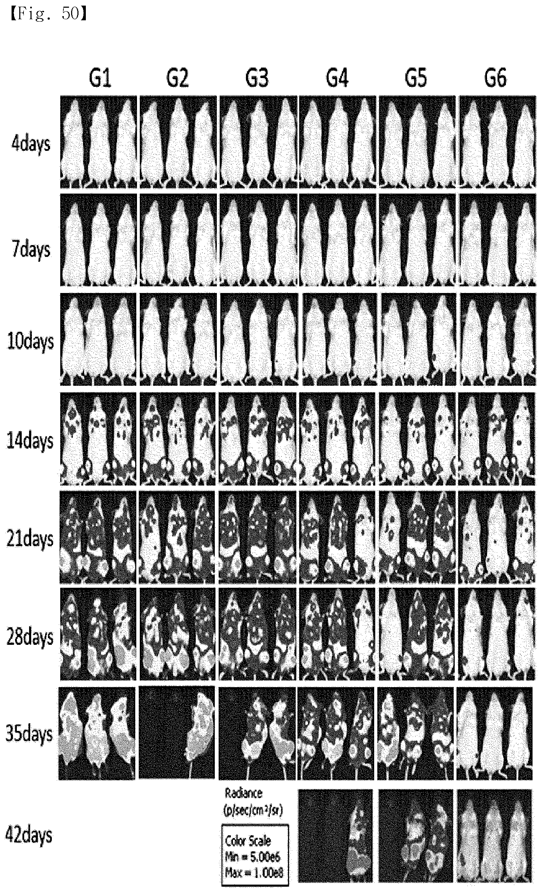

### Anti-tumor potency assessed in vivo for CD19-specific CAR-T cells (I) (Figures 49 and 50)

The anti-tumor potency of CAR-T cell_19bbz and CAR-T cell_19bbz#F was evaluated at the indicated multiple doses (1×10⁶, 3×10⁶, and 1×10⁷).

As a result, as shown in Figure 50, it was found that the control group treated with the conventional 2nd generation CAR-T cells (19bbz) did not effectively inhibit the growth of tumor so that rapid tumor growth was observed. On the other hand, CAR-T dose-dependent anticancer effect was observed in the group treated with CAR-T cells (19bbz#F) that overexpressed FKBP12, an immunophilin, as a cytoplasmic immune modulator. In particular, the group treated with CAR-T cells (19bbz#F) at a high dose (1×10⁷) showed an outstanding anticancer effect rapidly controlling the aggressive tumor growth. Therefore, these results indicate that overexpressing an immunophilin, FKBP12, is a very useful technique for augmenting the anticancer activity of immune cells.

### FKBP12 binding to TGF-β receptor (Figures 51 to 53)

Figure 51 shows the cocrytal structure of TGF-β type 1 receptor kinase domain bound to FKBP12 revealed for the three TGF-β type 1 receptors, respectively. The figure is based on the following protein data bank data: TGFβR1 (PDB ID: 1b6c), ACVR1A (PDB ID: 3h9r) and BMPR1B (PDB ID: 3mdy). The gray-colored surface structure represents the kinase domain of the TGF-β type 1 receptor, and the red ribbon skeleton structure corresponds to the FKBP12 protein. As shown in Figure 51, the sites that bind to FKBP12 in these three TGF-β type 1 receptor proteins are structurally very well conserved. This is actually in good harmony with the mechanism of action in which TGF-β signaling is negatively affected by FKBP12 binding for all the seven TGF-β type 1 receptors identified so far. The structures reveal consistently the fact that FKBP12 binding sterically blocks the site to be phosphrylated by the TGF-β type 2 receptor.

Figure 52 shows the tetrad aromatic amino acids (Aromatic Residues) of FKBP12, which are pivotally involved in FKBP12 binding to TGF-β type 1 receptor as revealed in the cocrystal structure for the three the TGF-β type 1 receptors. The aromatic amino acids of FKBP12, which are considered to be critical in the binding, are 27th tyrosine, 47th phenylalanine, 60th tryptophan, and 100th phenylalanine, and their positions are very well conserved in the three-dimensional structure.

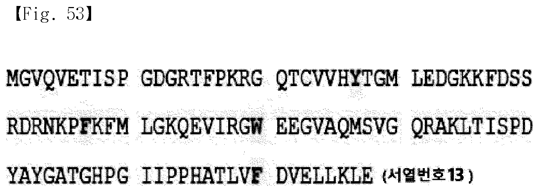

Figure 53 shows the positions of aromatic amino acids (27th tyrosine, 47th phenylalanine, 60th tryptophan, and 100th phenylalanine), which are pivotally involved in its TGF-β type 1 receptor binding, on the amino acid sequence of the FKBP12 protein.

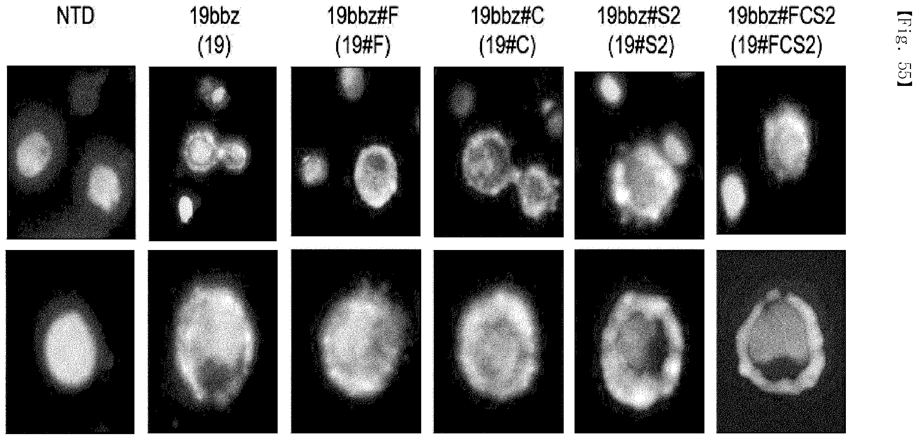

### Feature of surface CAR expression in CD19-specific CAR-T cells revealed by immunofluorescence microscopy (Figure 55)

In the case of CAR-T, the function of CAR T to screen out the specific target tumor cell depends on the "surface expression level" of the CAR molecule. However, when excessive CAR expression is induced above an appropriate level, "abnormal CAR clustering even in the absence of an antigen (Ag)" is often induced intrinsically by aggregation. In such cases, CAR molecules are likely not to show a uniform distribution on the cell surface, but to show a discontinuity in distribution repeating conglomerate and absence (punctate phenotype). It is reported that this phenomenon is associated with antigen-independent T cell signaling, resulting in an exhausted T cell phenotype with loss of effector functions required for antitumor effecacy.

In this Example, it was aimed to examine whether the intended and enforced expression of CAR together with the cytoplasmic immune modulator using a lentivirus is to cause any untoward phenotype of "excessive CAR clustering in the absence of an antigen" and abnormal CAR distribution of discontinuity in distribution (punctate phenotype). For this purpose, CAR-T cells were subjected to immunofluorescence analysis using a Cy3 labeled goat anti-mouse IgG antibody to label a CD19-specific CAR. Staining was performed on CAR-T cells obtained in culture on day 7 after initial stimulation. As shown in the results, it was confirmed that the CAR molecules were uniformly distributed on the cell surface in these CAR T cells presumed to be in a state of CAR expression stabilized enough to show a representaive distribution of CAR.

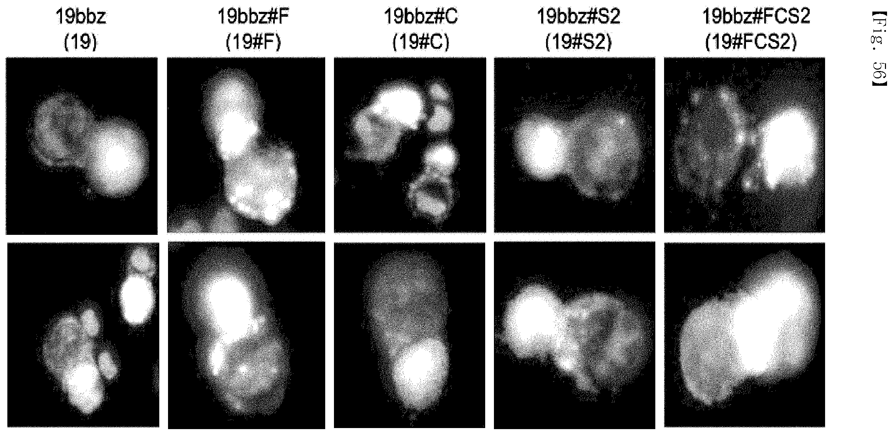

### CAR-mediated interaction between CAR-T cells and target tumor cells confirmed by immunofluorescence analysis (Figure 56)

In this Example, in order to confirm whether the prepared CAR-T interacts with the CD19-positive cancer cells directly through the CAR molecule on the surface of the CAR-T cell, the CAR-T cells were coincubated with the Daudi cells expressing eGFP to show green fluorescence for 1 hour. Thereafter, immunostaining of CAR molecules using a Cy3 labeled goat anti-mouse IgG antibody was performed on a slide glass, and observed with a fluorescence microscope.

As shown in the figure, it was confirmed that the CAR molecules labeled with Cyanine 3 (red) are actually mediating the close contact between CAR-T and the target cancer cell (green) indicated by eGFP fluorescence. In addition, it appeared that the green fluorescence of Daudi cells undergoing intimate interaction with CAR-T cells was slightly reduced in brightness compared to the green fluorescence of free Daudi cells.

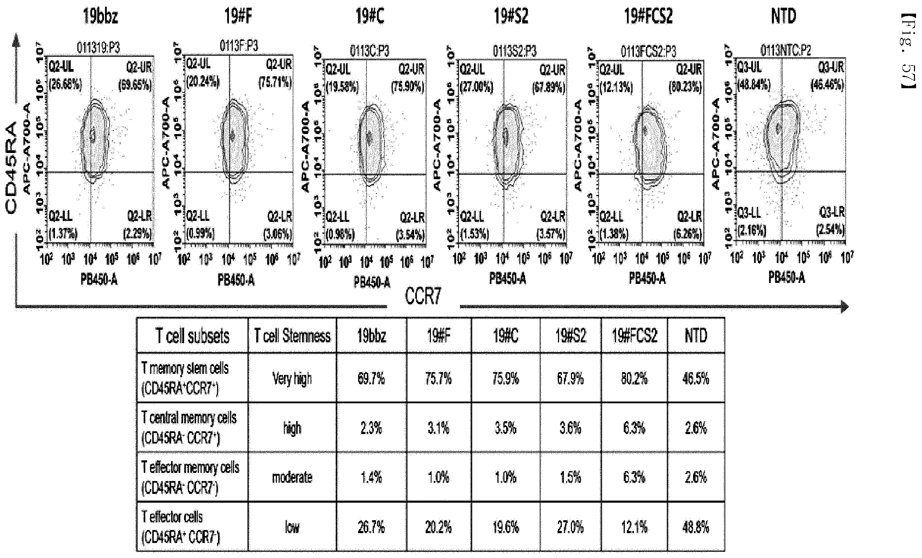

### T cell subset analysis on the CAR-T cells (Figure 57)

Recently, the clinical application of CAR-T cells to patients and many non-clinical animal model studies revealed that adoptive CAR-T cell therapy should be designed to ensure the persistence of anticancer immunity to prevent tumor relapse. For this reason, many researches have been conducted to reveal the potential impact of CAR-T cell subsets on the clinical outcome and finally stressed the advantage of having T cells enriched with relatively high T cell stemness such as stem cell-like memory T cell (Tscm, CD45RA⁺CCR7⁺) and central memory T cell (CD45RA⁻CCR7⁺) to get the cells to highly proliferate *in vivo* and sustain for a longer time. Therefore, in the field of preparing CAR-T cells for anticancer treatment, there is a growing interest in technology for inducing cells with high stemness such as the method using IL-7, IL-15, IL-21 or the like rather than IL-2.

According to the recent literature, in the characterization of GD2-specific CAR-T cells (HA-28z) engineered to have higher affinity to target antigen, the phenotypic T cell analysis revealed that T cell exhaustion was induced and accompanied with significant reduction in T cells with high T cell stemness.

Therefore, it was aimed to examine whether the CAR-T cells prepared in one embodiment can well maintain T cell stemness which is important for long-term persistence of anticancer immunity while attaining high effector T cell activity through the overexpression of cytoplasmic immune regulators. To this end, functional analysis such as tumor cell killing activity and cytokine release activity, T cell motility and the like of the prepared CAR-T cells was performed, and in parallel therewith, in order to confirm T cell stemness, phenotype analysis was performed.

For the use in these analyses, as a negative control cells, non-transduced control T cells (NTD) was derived from the cells of the same donor and cultured in parallel with the CAR-T culture through the same procedures of immunobead-based stimulation and T cell expansion but without performing lentiviral transduction. When the negative control T cells were subjected to T cell subset analysis, it was shown that the effector T cell population corresponding to the differentiated cells with the least stemness (renew potential) was about half (49%), and Tscm subset was about 47%.

On the other hand, in the case of Control CAR-T (19bbz) expressing only the conventional 2nd generation CAR, the Tscm was about 70%, which was found to be maintained higher than NTD by 20% or more. In the case of CAR-T cells (19bbz#S2) overexpressing the N-terminal SH2 domain of SHP2, it was shown to have a T cell population most similar to Control CAR-T cells (19bbz) expressing only the 2nd generation CAR.

On the other hand, in the case of CAR-T cells (19bbz#F, 19bbz#C) overexpressing an immunophilin protein such as FKBP12 or cyclophilin A, the proportion of Tscm subset was about 76%, which was found to be maintained higher than the 2nd generation Control CAR-T cells (19bbz) by about 6%. In addition, in the case of CAR-T cells (19bbz#FCS2) expressing the two types of immunophilins and the N-terminal SH2 domain of SHP2 at the same time, the proportion of Tscm subset was about 80%, which was found to be maintained higher than the 2nd generation Control CAR-T by 10% or more. Thus, the CAR-T cells (19bbz#FCS2) appeared to have the highest stemness among the tested cells.

Therefore, it was indicated that the overexpression of the cell signaling pathway modulator(s) introduced in the present invention was not likely to cause a decrease in stemness of T cells, which is a desirable property for anticancer efficacy of CAR-T cells.

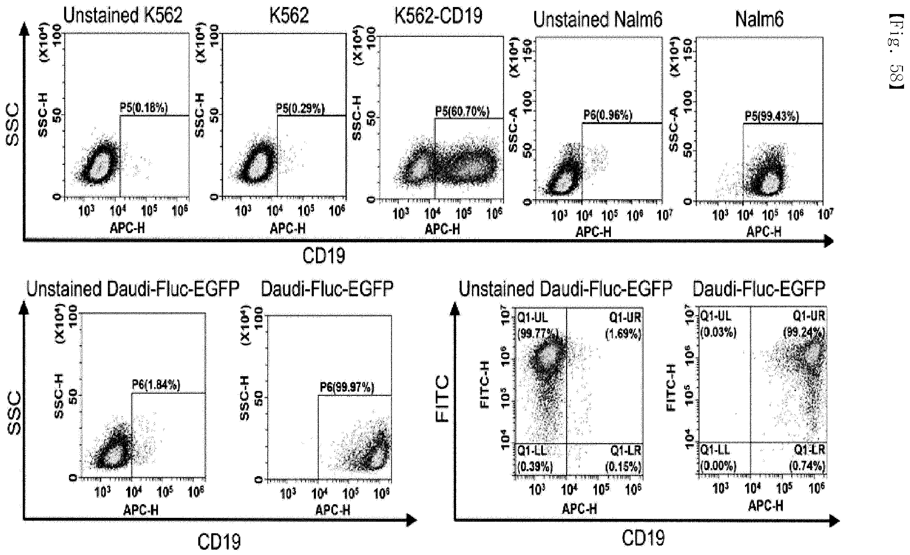

### Analysis of CD19 expression in target tumor cells (Figure 58)

For the use in the assessment of *in vitro* tumor cell killing ability and cytokine release activity of the prepared CD19-specific CAR-T cells, CD19-expressing tumor cells were cultured. In addition, the expression level of CD19 was analyzed by using APC-labeled mouse anti-CD19 antibody and flow cytometry.

According to the mean fluorescence units acquired by flow cytometry, the expression level of CD19 was shown to be approximately two-fold higher in Daudi cells than that of Nalm6. In addition, it was found that the expression of the CD19 was approximately three-fold higher in Nalm 6 than that of K562-CD19 (CD19 expression level: Daudi > Nalm6 > K562-CD19).

In particular, the Daudi cells stably expressing an emerald green fluorescent protein (eGFP) gene was used as target cells in the fluorescence microscopic analysis. In addition, as the Daudi cells were also expressing a firefly luciferase (Fluc) gene, the cells were used for *in vitro* tumor cell killing activity analysis by bioluminescence measurement. In addition, the Daudi-Fluc-Puro cells were used as target cells in the previous evaluation *of in vivo* CAR-T efficacy using the animal model of hematological malignancy.

As a negative control lacking CD19 expression, K562 cells were used in the other *in vitro* functional analyses on CD19-specific CAR-T cells. In addition, K562-CD19 cells were also used as target cells easily discernible from T cells by having a larger cell size, especially for the experiments using microscope and flow cytometry.

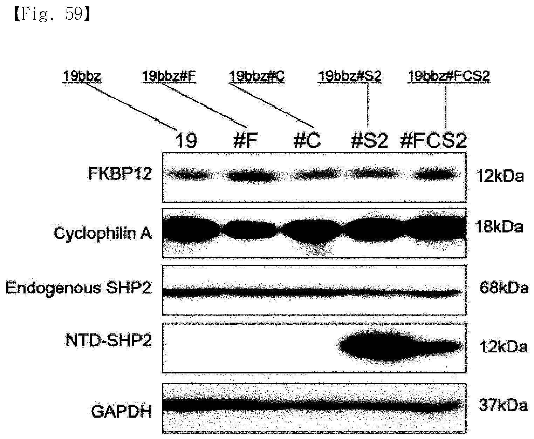

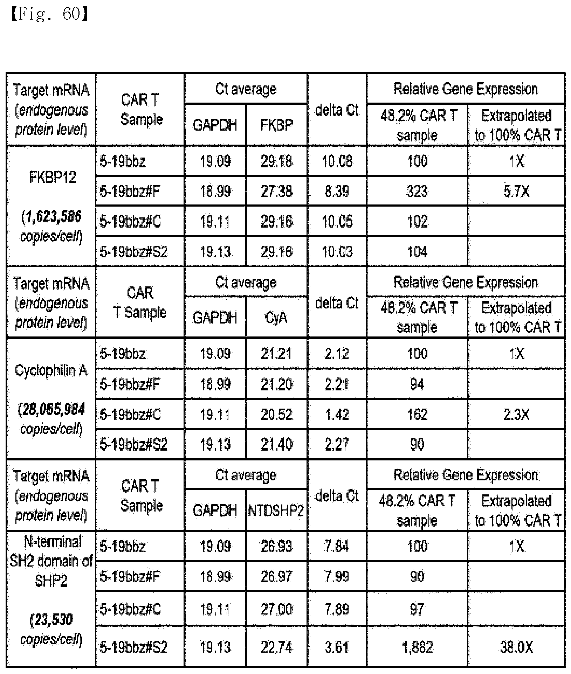

### Expression level of FKBP12, cyclophilin A. and the N-terminal SH2 domain of the SHP2 protein in CAR-T (Figures 59 and 60)

The degree of overexpression of the foreign gene encoding a specific immune regulator introduced into the cell through lentiviral vector transduction was confirmed for the prepared CAR- T by comparing it with the intrinsic level of the relevant intracellular protein. Specifically, the assessment was performed by qRT-PCR analysis and immunoblot analysis using antibodies.

As indicated in Figure 60, according to the published proteomic data on T lymphocyte (Hukelmann et al., Nature Immunology, 2015), the intrinsic level of relevant intracellular protein is revealed as 1,623,586 copies/cell for FKBP12, 28,065,984 copies/cell for cyclophilin A, and 23,530 copies/cell for SHP2, respectively. These data indicate the intrinsic levels of proteins are in the order of Cyclophilin A» FKBP12 » SHP2 in the protein copy number and it is obvious that the SHP2 protein transducing the signals of various inhibitory immune checkpoints actually exists in a remarkably small amount among them.

Therefore, although all these signaling pathway modulators were expressed through the same promoter as the CAR gene, the fold of overexpression was apparently quite different among the signaling pathway modulators. Specifically, in the case of CAR-T cells (19bbz#F) overexpressing FKBP12, the enforced overexpression of FKBP12 was about six-fold more compared to the endogenous expression. In addition, in the case of CAR-T cells (19bbz#C) overexpressing cyclophilin A, the enforced overexpression of cyclophilin A was about two-fold more compared to the endogenous expression. On the other hand, in the case of CAR-T cells (19bbz#S2) overexpressing the N-terminal SH2 domain of SHP2, the enforced overexpression of N-terminal SH2 domain of SHP2 was confirmed to be about 38-fold more compared to the endogenous level of SHP2 protein.

As mentioned above, the endogenous protein levels are quite different. The proportion of CAR positive cells were approximately 40% in all the CAR T samples of this analysis. As shown in the immunoblot image, the achieved extent of overexpression turned out to be quite different when assessed relatively to the endogenous level. That is, in the case of CAR-T cells (19bbz#S2) overexpressing the N-terminal SH2 domain of SHP2, the observed overexpression was quite obvious (at least 30-fold more compared to the endogenous level of SHP2). In addition, in the case of CAR-T cells (19bbz#F) overexpressing FKBP12, the observed level of overexpression was lower than the above and in the range of 2- to 7-fold more compared to endogenous level of FKBP12. In the case of CAR-T cells (19bbz#C) overexpressing cyclophilin A, as the level of endogenous protein was too high, the difference forced by overexpression was found often to fall within the error range of general blot analysis, making the quantitation not very reliable.

In the case of CAR-T cells (19bbz#FCS2) expressing three types of signaling pathway modulators at the same time, the level of FKBP12 overexpression was observed to be similar to that of CAR-T cells (19bbz#F). However, the level of overexpression of the N-terminal SH2 domain of SHP2, which may be limited by the need of three sequential events of self-cleavage to be exerted successfully on three P2As, was observed to be at the level of 1/3 of the expression level observed in CAR-T cells (19bbz#S2), in which the overexpression of the N-terminal SH2 domain of SHP2 is dependent on only one event of self-cleavage activity on one P2A.

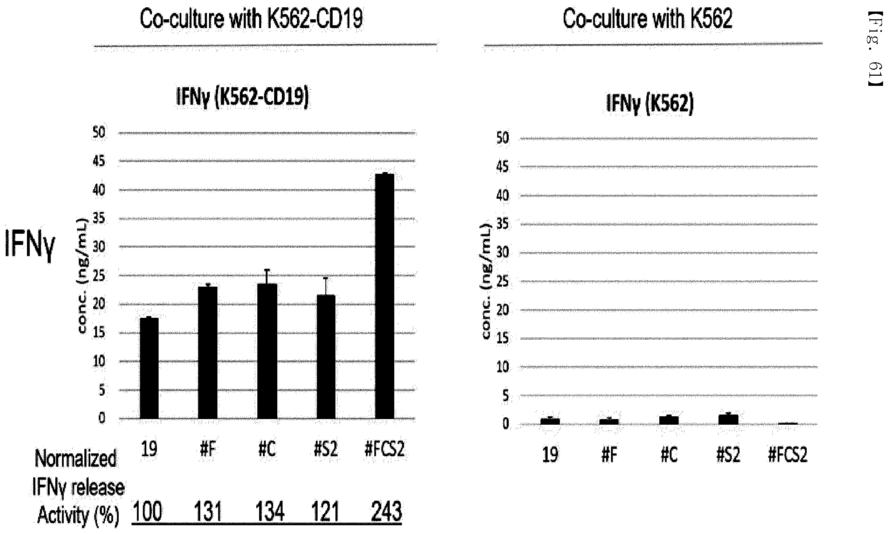

### Measurement of IFNγ, TNFα and IL-2 secreted from CAR-T cells upon antigenic stimulation (Figures 61 to 63)

In general, it is known that the anticancer effect of CAR-T may come from i) tumor cell killing activity dependent on perforin/granzyme granule secreted after CAR-T forms an immune synapse with an antigen-positive target tumor cell, ii) tumor cell killing and bystander killing activity dependent on Fas ligand with increased expression on the surface of activated CAR-T cells, and iii) tumor cell killing activity based on cytokine-mediated indirect mechanism (secondary mechanism).

IFNγ, one of the key cytokines secreted from activated CAR-T, exhibits the following effects. i) By increasing IFNγ receptor expression on the surface of stroma cells, it induces the increased secretion of chemokines such as IP10 (CXCL10), MIC (CXCL9) and the like to cause the effect of increasing the infiltration of immune cells such as T cells, NK cells, DCs, monocytes/macrophages and the like into tumor tissues. In addition, ii) it may also directly cause tumor stroma cell destruction. In addition, iii) it is known to exhibit a growth inhibitory effect (cytostatic effect) directly by acting on tumor cells. In addition, iv) it induces polarization of tumor-associated macrophages and tumor-resident T cells to get them out of immune suppressed state. As a result, it is known to indirectly impart an anticancer activity capable of killing tumor cells and exert a secondary anticancer effect through this.

TNFα (Tumor necrosis factor alpha), which is another important cytokine secreted from activated CAR-T, i) directly induces tumor cell death by signaling through the TNFα receptor expressed on the surface of tumor cells. In addition, ii) TNFα activates effector T cells and NK cells by blocking regulatory T cells. In addition, iii) TNFα acts on endothelial cells to induce tumor microvasculature collapse and disruption of neoangiogenesis. In addition, iv) TNFα has an activity to induce polarization of M2 macrophage without an anticancer activity to M1 macrophage having an anticancer activity. In addition, v) TNFα acts to attract and activate neutrophils and monocytes to the tumor site to have an anticancer activity. Finally, vi) TNFα is known to downregulate IL-13 expression by eosinophilic-like cells in cancer tissues, thereby interfering with the differentiation of monocytes into immunosuppressive cells.

IL-2 (Interleukin-2), which is another important cytokine secreted from activated CAR-T, has been already successfully used in anticancer immunotherapy. As such, in addition to the well-known activity of stimulating T cell proliferation, it is also well known to contribute to anticancer immunity through the role of greatly enhancing the cytolytic activity of NK cells and lymphokine-activated killer cells.

Therefore, in this Example, the cytokine release activity of CAR-T (19bbz#F), CAR-T (19bbz#C), CAR-T (19bbz#S2), and CAR-T (19bbz#FCS2) prepared by the present invention was compared side-by-side to test whether the invented CAR T is superior to the conventional 2nd generation CAR-T.

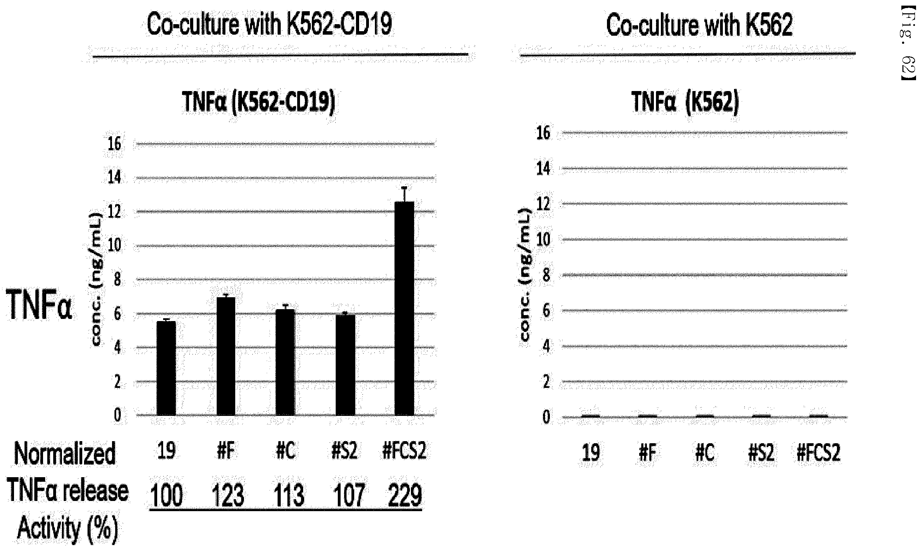

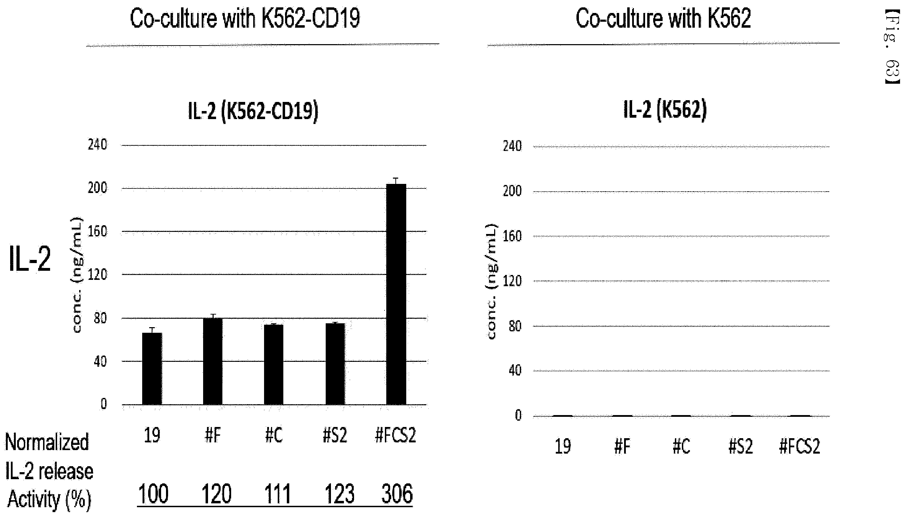

As shown in the figure, when evaluating the secretion of three types of cytokines (IFNγ (Figure 61), TNFα (Figure 62), IL-2 (Figure 63)), no significant level of cytokine secretion was detected in all the co-cultures of CAR-T with K562 cells lacking the expression of target CD19 after 24 hours.

On the other hand, a considerable quantity of cytokines was detected in all the co-cultures of CAR-T with K562-CD19 cells engineered to express CD19 after 24 hours.

As expected from the design concept, the three type of CAR-Ts, CAR-T cells (19bbz#F), CAR-T cells (19bbz#C), and CAR-T cells (19bbz#S2), which overexpressed an immunophilin known to be involved in the basal immune activity of T cells or overexpressed the N-terminal SH2 domain of SHP2 capable of blocking pTyr dephosphorylation by immune checkpoint signal, appeared to show a slightly augmented cytokine secretion compared to that of the conventional control CAR-T cells (19bbz), respectively.

Interestingly, upon target tumor cell stimulation, the CAR-T cells (19bbz#FCS2) that simultaneously overexpressed these three types of signaling pathway modulators showed a significantly enhanced level of cytokine secretion compared to that of the conventional control CAR-T cells (19bbz).

According to the clinical experience of CAR-T in humans so far, it is known that solid cancer is more challenging than hematological malignancy for CAR-T to access the tumor site. Even if CAR-T reaches the tumor site, its action is to be impeded by several mechanisms pre-established in the immune suppressive tumor microenvironment (TME). Therefore, there has been a need to design a super-armed CAR-T that can withstand even under the immunosuppressive signal present in such TME.

According to the results of this Example, it is expected that CAR-T (19bbz#FCS2) may secret a considerably large amount of cytokines even in an immunosuppressive environment. Therefore, by secreting a large amount of cytokines which can convert the tumor microenvironment from the immunosuppressive to a more immune-friendly environment, it is likely to exert a considerable anticancer efficacy.

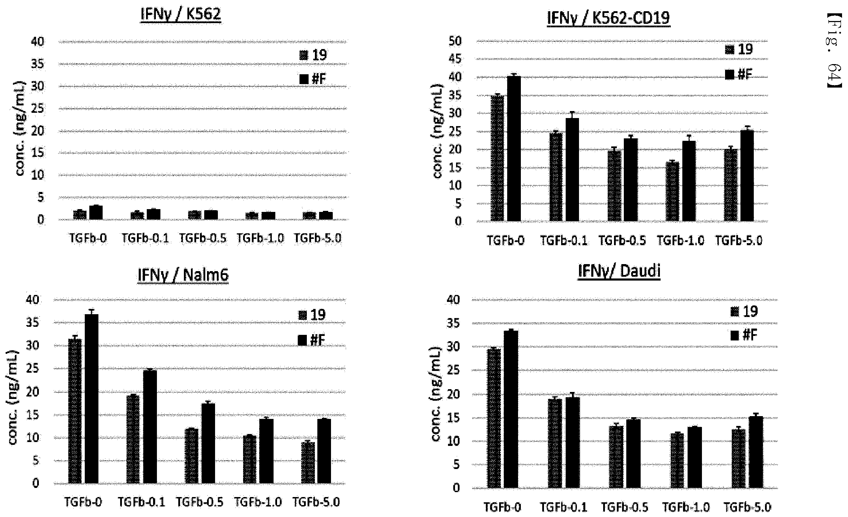

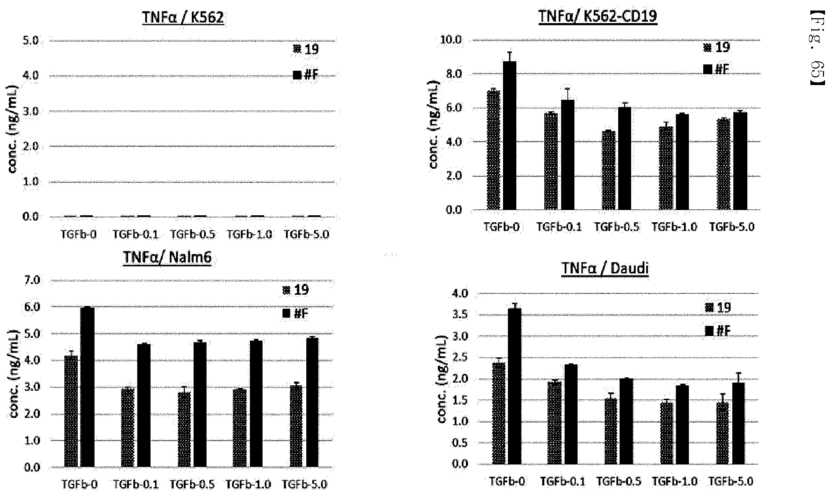

### Measurement of IFNγ and TNFα secreted from CAR-T cells upon antigenic in the presence of TGF-β1 (Figures 64 and 65)

In general, it is known that a high concentration of TGF-β in the tumor tissues can greatly inhibit the normal T cell functions such as T cell activation, proliferation and differentiation, by triggering immunesuppressive signal through the TGFβ receptors (TGFβRI, TGFβRII) expressed on the surface of T cells.

However, through the mouse model studies it is known that the interruption of TGF-β signal in naive T cells can induce autoimmune diseases. Therefore, while it is needed to develop a way to inhibit the TGF-β signal in an effort to augment anticancer efficacy of CAR-T cells, it is desirable not to design a novel device to completely shut down the TGF-β signaling in the adopted T cells considering the potential risk of autoimmunity.

In the case of the concentration range of TGF-β1 used in this Example, it was considered that the half maximal effective concentration (EC50) in which a significant reaction occurs concentrationdependently *in vivo* was 0.04-0.2 ng/mL. In addition, the test concentration range was set to sufficiently exceed the upper limit of the high concentration that can be accumulated in the pathological environment.

In addition, it was considered that microheterogeneity of tumor cells frequently occurred during tumor development and progression. Therefore, in order to represent such a situation, CAR-T activity was tested for three types of cancer cells that differ in the "expression level of CD19, a target antigen" and "cell size" (the order of cell size: K562-CD19» Nalm6, Daudi; the order of Ag expression level: Daudi> Nalm6> K562-CD19).

As an evaluation method, we tried to evaluate the secretion activity of cytokines critical in the anticancer immune mechanism of CAR-T. As a result, it was shown that the degree to which CAR-T cells are affected by the concentration of TGFβ1 appeared to be somewhat different depending on the target cells and secreted cytokines.

However, throughout a wide range of concentrations of TGFβ1 (0 ng/mL-5 ng/mL), it was observed in common in all three types of target cell responses that CAR-T cells (19bbz#F) overexpressing FKBP12 showed activities higher than or equal to that of the conventional 2nd generation CAR-T cells (19bbz).

The difference in CAR-T activity caused by FKBP12 overexpression was apparently more obvious in the reactions with Nalm6 or K562-CD19 in which the antigenic stimulation of CAR-T cells is predicted to occur less easily and slightly because the amount of antigen expression is relatively low, than in the case of the reaction with Daudi in which antigenic stimulation of CAR-T cells can be assured easily and strongly due to the high level of antigen expression on the target surface.

Generally, it is well known that the decreased level of antigen is one of the immune evasive mechanisms to be adopted by cancer cells during tumor progression. Considering this information and the results together, the overexpression of a small-sized modulator such as FKBP12 would be advantageous to modify CAR-Tcells to sustain the ability to fight aginst the Ag-low tumor cells which could drive tumor growth sneakily.

The basal activities of T cells are considered to be kept low by tonic signaling (TGFβ-independent dimerization-mediated signaling), in which TGFβRII, which remains active even in the absence of TGFβ, binds to TGFβRI to induce TGFβ signaling. However, when FKBP12, a competitive inhibitor of receptor heterotetramerization, is overexpressed, the basal activity of such T cells can be in a less depressed state to some extent. Therefore, it is expected that T cell activation is relatively easily made even in reactions with the cancer cells in which the amount of antigen expression is low.

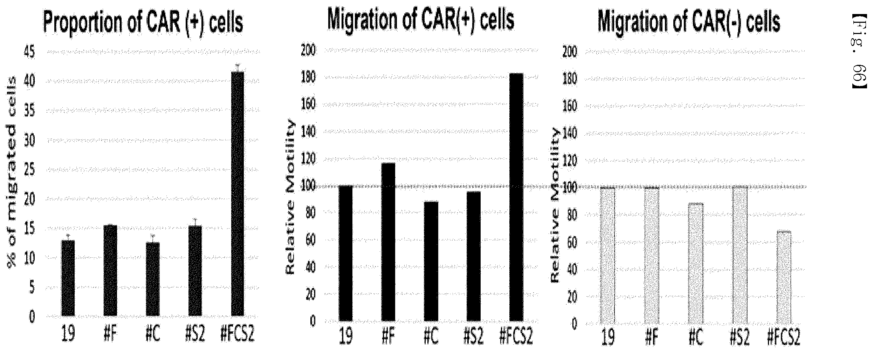

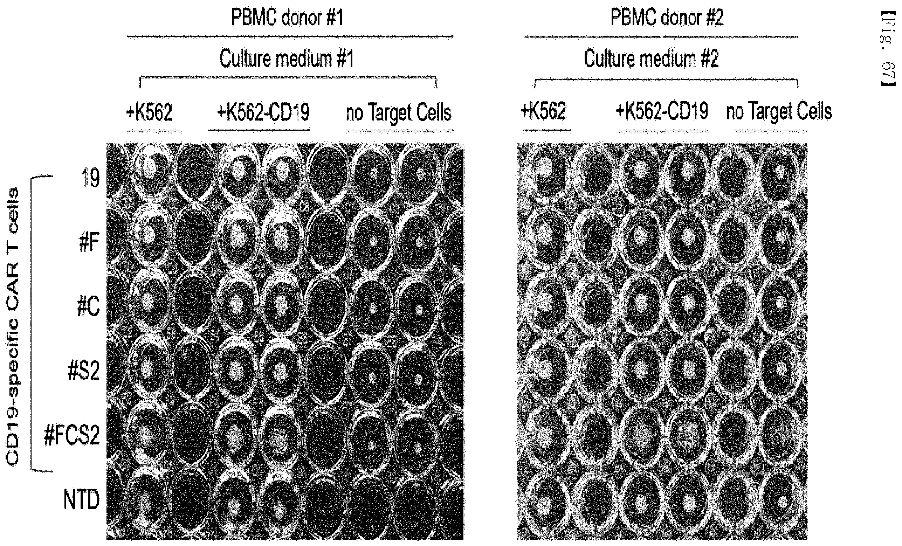

### Confirmation of the migration ability of CAR-T cells (Figures 66 and 67)

For the adoptive transfer of CAR-T cell, the ability to migrate effectively to the tumor site *in vivo* is an important point-to-consider in the design of the overall efficacy of CAR-T.

In general, the T cell trafficking to TME (tumor microenvironment) is accomplished through a tightly controlled process of several consecutive steps. During the consecutive steps of T cell trafficking, particularly integrin-mediated T cell adhesion and downstream events leading to T cell migration are known to be sensitively affected by an immunophilin such as FKBP12 and cyclophilin A.

In this Example, in order to compare the *in vitro* migration activity of CAR-T, CAR-T were activated using anti-CD3/CD28 Dynabeads during the culture, and then the debeaded cells were collected and washed, and were suspended in a serum-free medium in which 0.25% human serum albumin is added, and transwell migration was performed. In general, animal cells are to pass through the pores of the biomatrix in amoeboid motion.

As shown in the bottom graph, the migration ability of CAR(+) T cells was compared by assessing the CAR(+) % of the cells migrated to the lower chamber for 1 hour. Although the concentration of total T cells and CAR(+) T cells were adjusted to be the same for all the tested CAR-Ts prior to migration, a specific CAR-T (19bbz#FCS2) showed significantly higher migration ability compared to the other CAR-Ts.

In addition, T cell migration was assessed in the presence of EGF, a cytokine known to interfere with T cell infiltration with the cytokine added into the upper chamber. Even in this case, as shown in the middle graph, when comparing the number of CAR(+) cells migrated to the lower chamber, it was found that a specific CAR-T (19bbz#FCS2) showed significantly superior migration ability compared to the other CAR-Ts.

On the other hand, as shown in the top graph, it was found that the CAR (-) cells of CAR-T (19bbz#FCS2) sample did not show superioty in migration ability. Therefore, it was confirmed that the high migration ability seen in these CAR-T (19bbz#FCS2) samples was an activity limited to the CAR(+) cells overexpressing three types of cytoplasmic modulators.

It was also clearly observed that CAR-T (19bbz#FCS2) that overexpress three types of cytoplasmic modulators at the same time showed significantly superior intrinsic motility in the photograph photographed after performing co-culture with target cells on a 96 well plate for 48 hours (Figure 60).

As shown in the photograph, it was found that the motility of the corresponding CAR-T cells was most active in the co-culture with K562-CD19 cells, which are able to stimulate T cells by expressing the antigen CD19 on the surface.

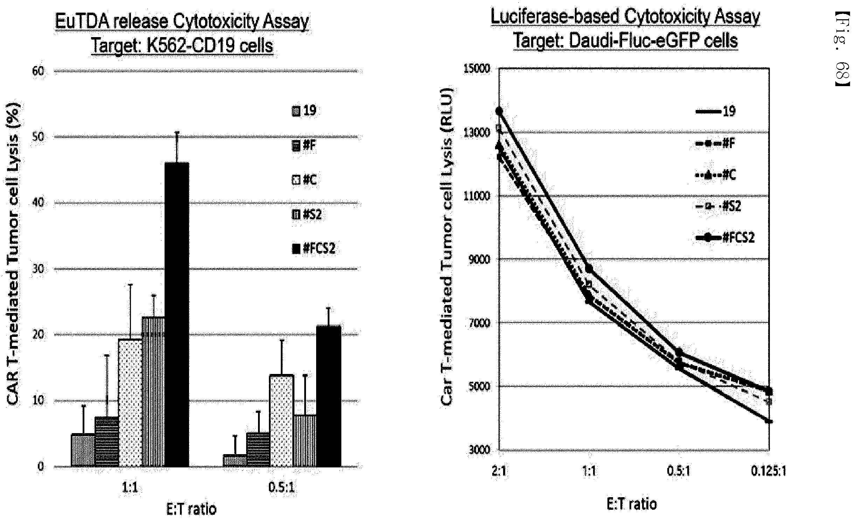

### In vitro tumor cell killing activity of CD19-specific CAR-T cells (Figure 68)

In this Example, in order to evaluate the tumor cell killing activity of CAR-T cells, two different methods were used as follows.

In the evaluation using K562-CD19 cells, as a method of observing tumor cell lysis, a method of quantifying TDA (2,2':6',2"-terpyridine-6,6"-dicarboxylic acid) secreted into the medium by cell lysis through TRF (Time-Resolved Fluorometry) was used.

As a result, as shown in the bottom graph of the figure, it was found that the results of evaluating tumor cell killing activity by CAR(+)-T cells was somewhat consistent with the results of evaluating cytokine secretion activity described above.

The three types of CAR-Ts, CAR-T cells (19bbz#F), CAR-T cells (19bbz#C), and CAR-T cells (19bbz#S2), which overexpressed an immunophilin known to be involved in the basal immune activity of T cells or overexpressed the N-terminal SH2 domain of SHP2 capable of inhibiting pTyr dephosphorylation by immune checkpoint signal, appeared to have a relatively slightly higher tumor cell killing activity compared to that of the conventional control CAR-T cells (19bbz), respectively.

On the other hand, it was confirmed that CAR-T cells (19bbz#FCS2), which overexpressed these three types of small-sized cytoplasmic immune modulators at the same time, exhibited significantly higher activity compared to the conventional control CAR-T cells (19bbz).

In another set, the tumor cell killing activity of CAR-T cells were measured against Daudi cells expressing luciferase. The tumor cell lysis by CAR(+)-T cells was compared by the extent of reduction in bioluminescence. As shown in the top graph, it was found that the results were similar to those of the evaluation by Eu-TDA release.

The three types of CAR-Ts, CAR-T cells (19bbz#F), CAR-T cells (19bbz#C), CAR-T cells (19bbz#S2) that overexpressed an immunophilin or the N-terminal SH2 domain of SHP2, showed slightly higher tumor cell killing activity compared to that of the conventional control CAR-T cells (19bbz), respectively. In particular, it was confirmed that CAR-T cells (19bbz#FCS2) showed the highest activity over the entire tested range of E:T ratio.

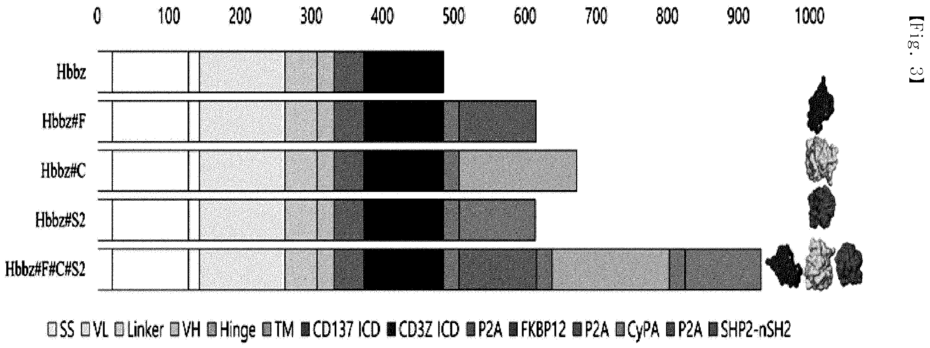

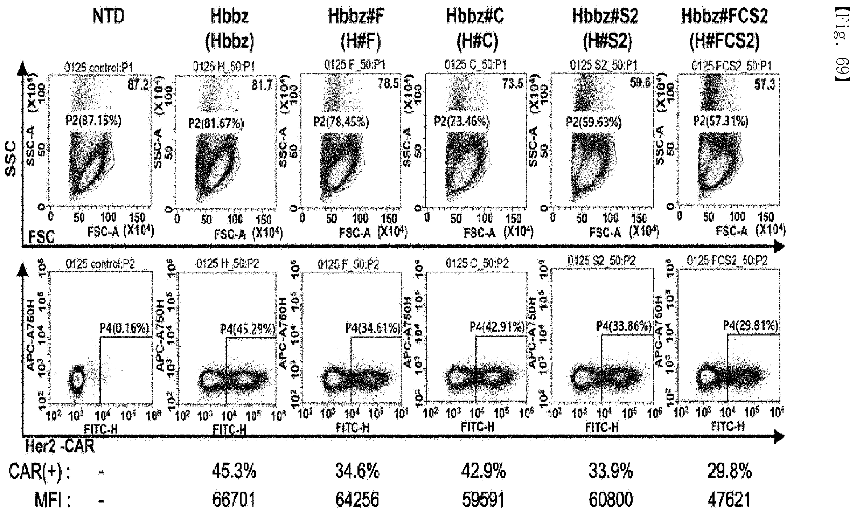

### Preparation of Her2-specific CAR-T cells (Figures 3 and 69)

As another example of the present invention, as a CAR-T targeting Her2 positive cancer cells, "Her2-specific CAR-T cells (Hbbz)" representing the conventional 2nd generation CAR-T were prepared. In addition, two types of CAR-T cells (Hbbz#F and Hbbz#C) expressing two known immunophilins, FKBP12 (12 kDa) and cyclophilin A (18 kDa), respectively, which are predicted to be capable of enhancing the anticancer function of CAR-T cells, were prepared, respectively.

In addition, CAR-T cells (Hbbz#S2) overexpressing the N-terminal SH2 domain of SHP2 that can inhibit pTyr dephosphorylation by immune checkpoint signal were also prepared. In addition, CAR-T cells (Hbbz#FCS2) that overexpressed these three types of signaling pathway modulators at the same time were also prepared. The CAR expression measured on the day 4 after lentiviral transduction is as shown in Figure 69. Thereafter, through the following expansion culture, the proportion of CAR(+) cells in CAR-T (Hbbz#FCS2) was observed to reach a level of 40-50%.

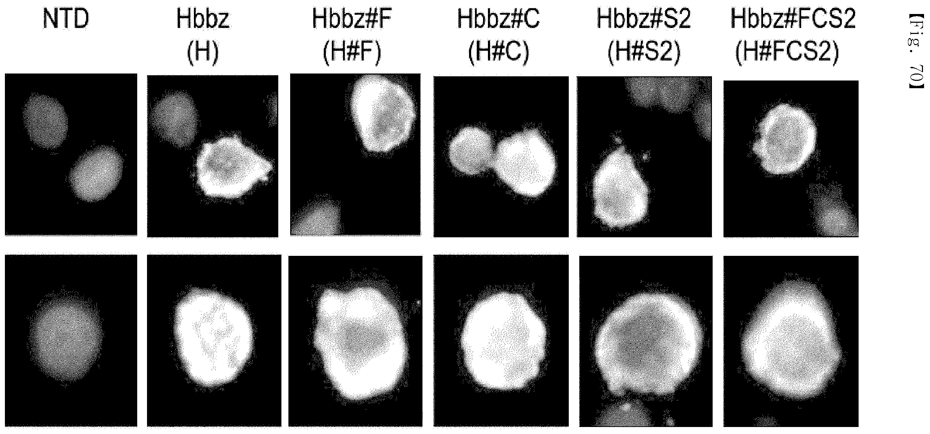

### Feature of surface CAR expression in Her2-specific CAR-T cells revealed by immunofluorescence microscopy (Figure 70)

As can be seen from the results, the CAR molecules were uniformly distributed on the cell surface when expressed in the prepared CAR-T cells.

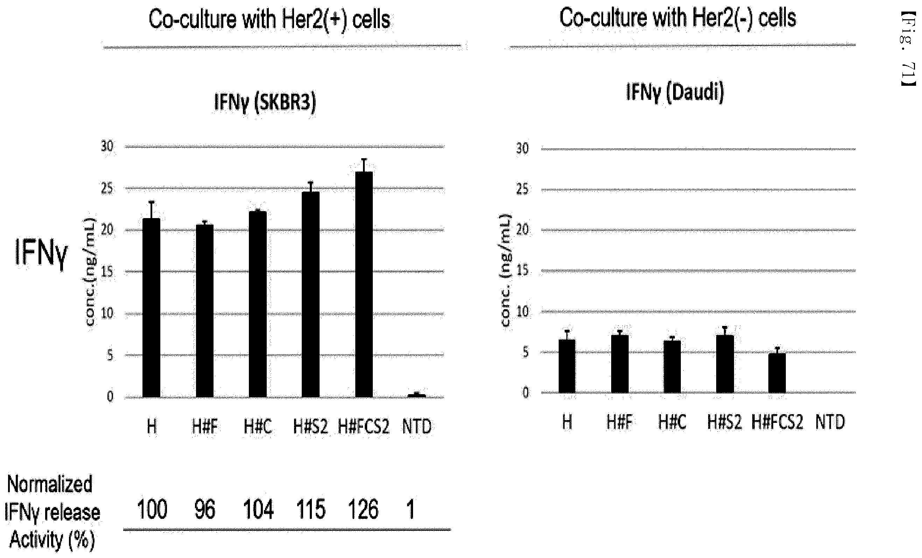

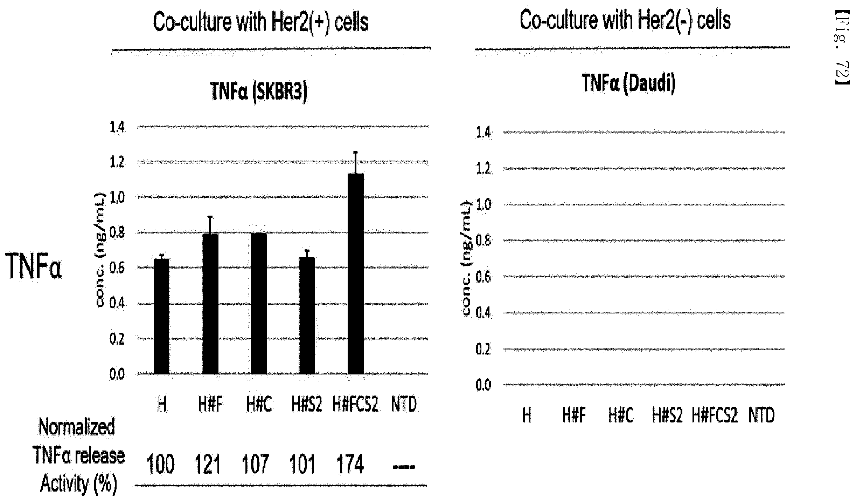

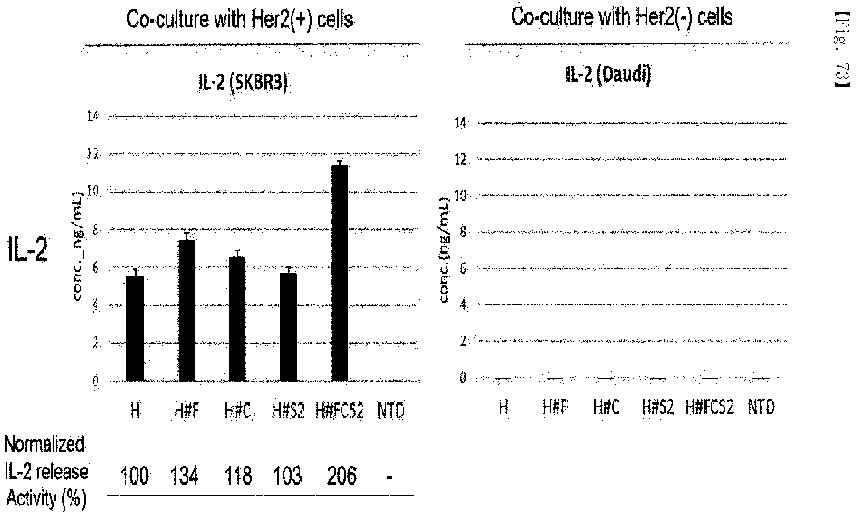

### Measurement of IFNγ, TNFα and IL-2 secreted from Her2-specific CAR-T cells upon antigenic stimulation (Figures 71 to 73)

In this Example, the cytokine release activity of CAR-T (Hbbz#F), CAR-T (Hbbz#C), CAR-T (Hbbz#S2), and CAR-T (Hbbz#FCS2) prepared by the present invention was compared side-by-side to test whether the invented CAR T is superior to the conventional 2nd generation CAR-T.

As shown in the figures, when evaluating the secretion of three types of cytokines (IFNγ, TNFα, and IL-2), almost no significant level of TNFa and IL-2 cytokine secretion was detected in all the co-cultures of CAR-T with Daudi cells lacking the expression of target Her2 after 24 hours. However, in the case of IFNγ, unlike TNFα and IL-2 cytokines, secretion of IFNγ was detected corresponding to within 1/3 of the amount of cytokine secreted in culture with SKBR3 cells expressing the target antigen Her2. This cause is believed to be because Daudi does not express Her2, but expresses some costimulatory molecules as a B lymphocyte. Thereby, perhaps the Daudi cells, acting as a potential antigen presenting cell derived from different person, is capable of inducing T cell activation in some T cells with a TCR responding to a specific histocompatibility antigen.

On the other hand, a considerable quantity of cytokines was detected in all the co-cultures of CAR-T with SKBR3 cells expressing Her2 after 24 hours.

As a result, the three types of CAR-Ts, CAR-T cells (Hbbz#F), CAR-T cells (Hbbz#C), and CAR-T cells (Hbbz#S2), exhibited almost the same as or slightly augmented cytokine secretion compared to that of the conventional 2nd generation control CAR-T cells (Hbbz), respectively.

In particular, upon target tumor cell stimulation, the CAR-T cells (Hbbz#FCS2) showed a significantly enhanced level of cytokine secretion compared to that of the conventional 2nd generation control CAR-T cells (Hbbz).

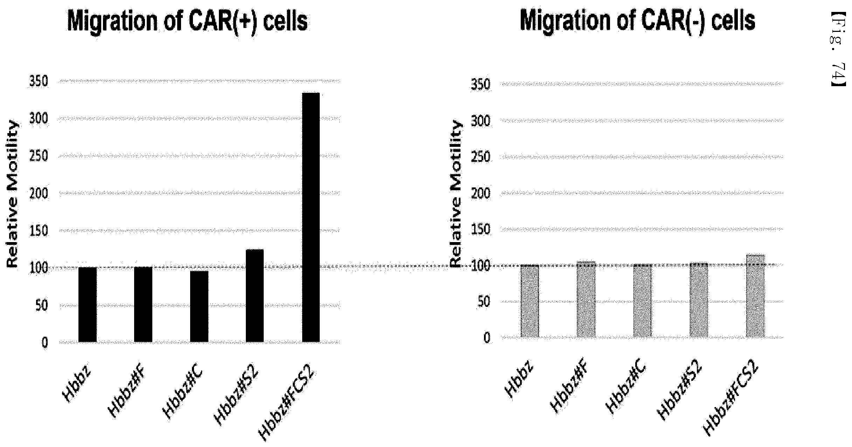

### In vitro migration of Her2-specific CAR-T (Figure 74)

In this Example, in order to compare the *in vitro* migration activity of Her2-specific CAR-T cells, the CAR-Ts were activated using anti-CD3/CD28 Dynabeads during during the culture, and then the debeaded cells were collected and washed, and were suspended in a serum-free medium in which 0.25% human serum albumin is added, and transwell migration was performed.

As a result, although the concentration of total T cells and CAR(+) T cells were adjusted to be the same for all the tested CAR-Ts prior to migration, a specific CAR-T (Hbbz#FCS2) showed significantly higher migration ability compared to the other CAR-Ts.

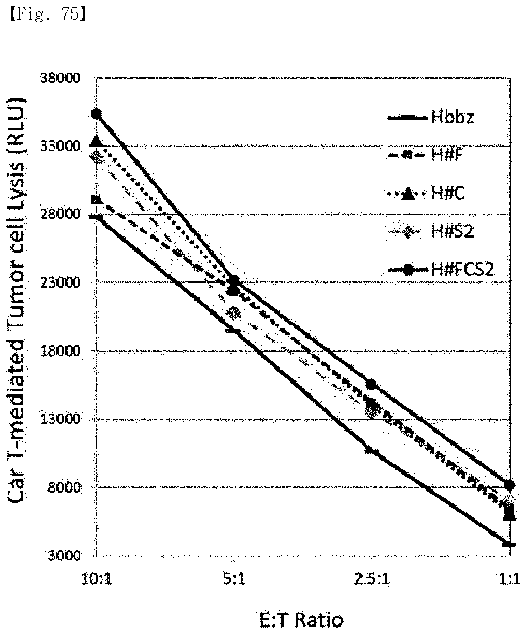

### In vitro tumor killing activity of Her2-specific CAR-T cells (Figure 75)

It was confirmed that all the three types of CAR-Ts, CAR-T cells (Hbbz#F), CAR-T cells (Hbbz#C), and CAR-T cells (Hbbz#S2), exhibited a slightly higher tumor cell killing activity compared to that of the conventional 2nd generation control CAR-T cells (Hbbz), respectively.

On the other hand, it was confirmed that the CAR-T cells (Hbbz#FCS2) that overexpressed these three types of modulators at the same time showed a significantly high activity over the entire tested range of E:T ratio.

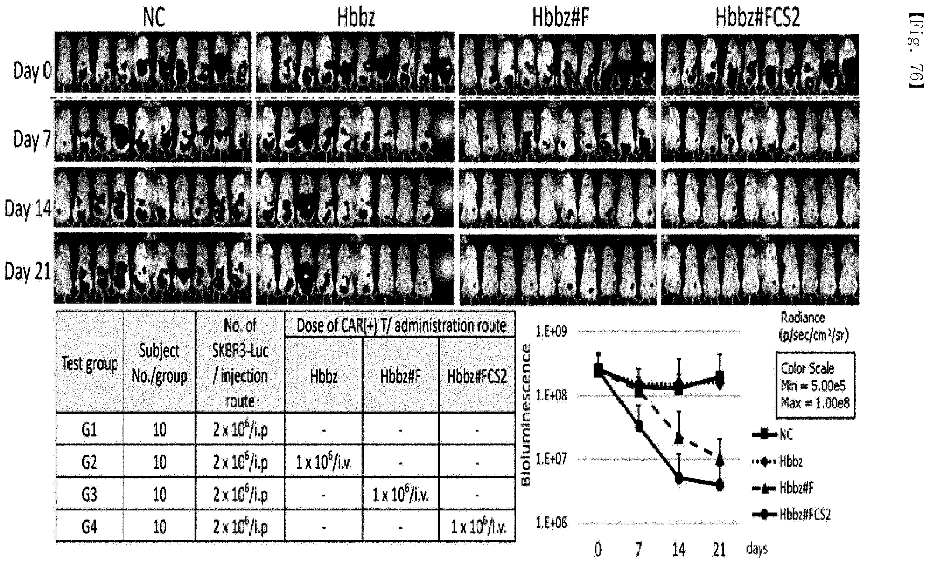

### Anti-tumor potency assessed in vivo for Her2-specific CAR-T cells (Figure 76)

The usefulness of the CAR-T cell technology introduced in the present invention was tested in a solid cancer model. SKBR3-Luc cells (2×10⁶ cells per a subject) were injected to immunoincompetent NSGA mice to induce intraperitoneal xenograft tumor for 14 days. Each designated CAR-T cell was intravenously administered to 10 subjects in each group in the same amount (1×10⁶ CAR(+)-T cells). Thereafter, the tumor burden was monitored by bioluminescence imaging using IVIS equipment at weekly intervals.

As a result, as shown in the figure, it was found that the group treated with the conventional 2nd generation control CAR-T cells (Hbbz) did not effectively inhibit the growth of tumors established in the abdominal cavity. On the other hand, the effect of inhibiting and eradicating solid cancer growing in the abdominal cavity was quickly and clearly observed in the group treated with CAR-T cells (Hbbz#F) that overexpressed an immunophilin FKBP12 as a signaling pathway modulator and the group administered with CAR-T cells (Hbbz#FCS2) that overexpressed three types of signaling pathway modulators at the same time.

In addition, the results were consistent with those observed in the evaluation of several *in vitro* CAR-T activities (cytokine secretion, *in vitro* migration, and *in vitro* tumor cell killing ability) performed as other Examples, and it was also confirmed that CAR-T cells (Hbbz#FCS2) that overexpressed three types of signaling pathway modulators at the same time exhibited a significantly superior performance *in vivo* in antitumor potency against a solid cancer established in animal model study.

In addition, the results were consistent with those observed in the evaluation of antitumor potency in a hematological malignacy model performed as other Examples, and it was also confirmed that even in the antitumor potency evaluated in the solid cancer animal model, the group treated with CAR-T cells (Hbbz#F) overexpressing only FKBP12, an immunophilin, exhibited an excellent anticancer activity compared to the conventional 2nd generation control CAR-T cells (Hbbz).

In addition, in the solid cancer model results of this Example, it was indicated that CAR-T cells (Hbbz#FCS2) that overexpressed three types of signaling pathway modulators at the same time exhibited significantly superior anticancer efficacy *in vivo,* which is due to the anticipated synergistic effects of multiple signaling pathway modulator-mediated potentiation based on different mechanisms.

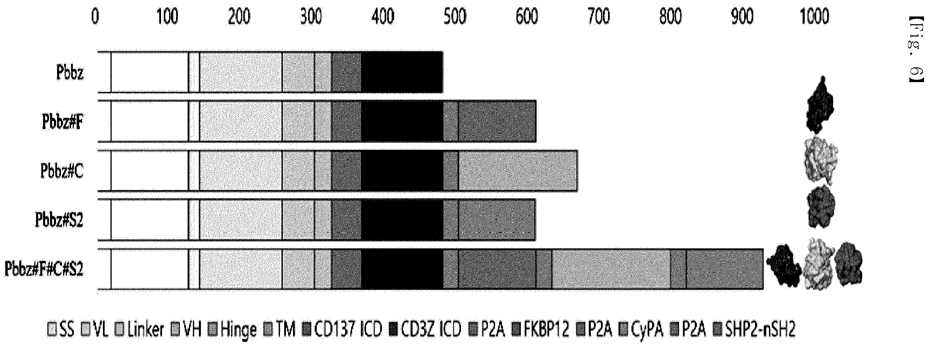

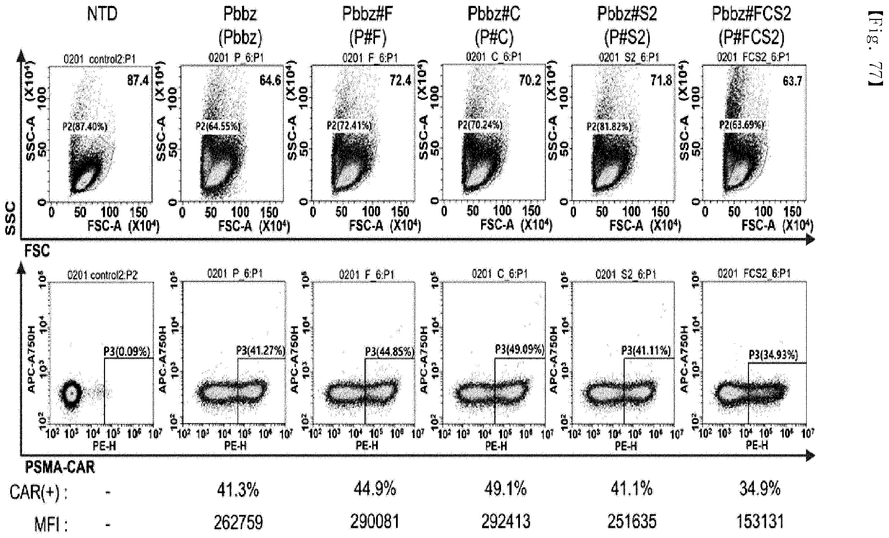

### Preparation of PSMA- specific CAR-T cells (Figures 6 and 77)

As another Example of the present invention, as a CAR-T targeting Her2 positive cancer cells, "PSMA-specific CAR-T cells (Pbbz)" representing the conventional 2nd generation CAR-T cells were prepared. In addition, in this case, two types of CAR-T cells (Pbbz#F and Pbbz#C) expressing two immunophilins, FKBP12 (12kDa) and cyclophilin A (18kDa), respectively, which are predicted to be capable of enhancing the anticancer function of CAR-T cells, were prepared, respectively.

In addition, CAR-T cells (Pbbz#S2) overexpressing the N-terminal SH2 domain of SHP2 that can inhibit pTyr dephosphorylation by immune checkpoint signal were also prepared. In addition, CAR-T cells (Pbbz#FCS2) that overexpressed these three types of signaling pathway modulators at the same time were also prepared. The CAR expression ratio measured on the day 4 after lentiviral transduction is as shown in Figure 77. Thereafter, through the following expansion culture, the proportion of CAR(+) cells in CAR-T cells (Pbbz#FCS2) was observed to reach a level of 40-50%.

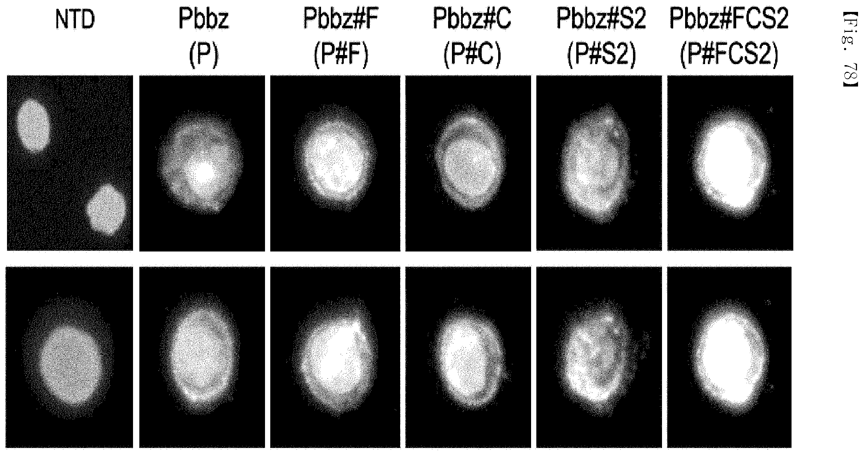

### Feature of surface CAR expression in PSMA-specific CAR-T cells revealed by immunofluorescence microscopy (Figure 78)

As shown in the figure, the CAR molecules were uniformly distributed on the cell surface when expressed in the prepared CAR-T cells.

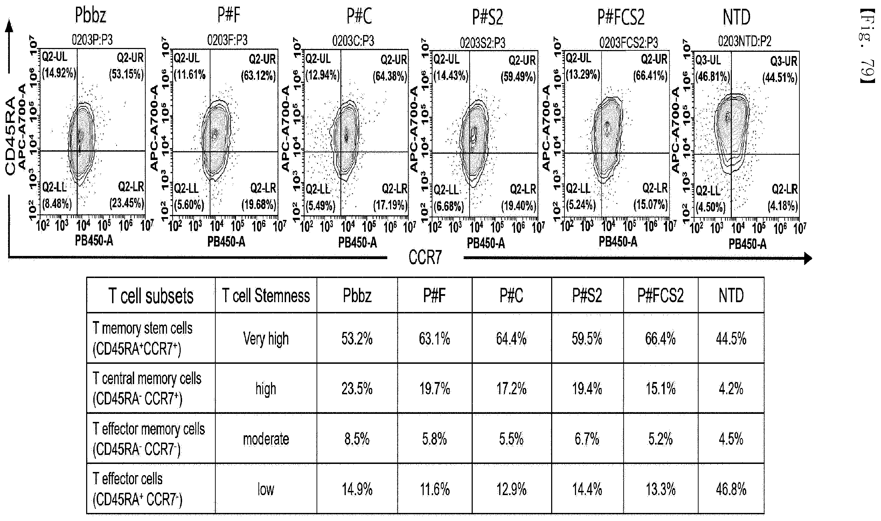

### T cell subset analysis on PSMA-specific CAR(+)-T cells (Figure 79)

In the case of NTD (Non-transduced control T cells), it was found that the effector T cell population corresponding to differentiated cells with the least stemness (renew potential) was almost half (47%), and the Tscm subset was about 45%. In contrast, in the case of Control CAR-T cells (Pbbz) expressing only the conventional 2nd generation CAR, the Tscm ratio was about 53%, which was found to be about 8% higher than that of NTD.

It was found that CAR-T cells (Pbbz#S2) overexpressing the N-terminal SH2 domain of SHP2 had relatively the most similar T cell populations to Control CAR-T cells (Pbbz) expressing only the conventional 2nd generation CAR.

On the other hand, in the case of CAR-T cells (Pbbz#F, Pbbz##C) overexpressing an immunophilin protein such as FKBP12 or cyclophilin A, the proportion of Tscm subset was 63% and 64%, respectively, and it was found to be maintained about 10% higher than that of the 2nd generation control CAR-T cells (Pbbz), and in the case of CAR-T cells (Pbbz#FCS2) expressing two types of immunophilins and the N-terminal SH2 domain of SHP2 at the same time, the proportion of Tscm subset was about 66%, and it was found to be maintained at least 10% higher than that of the 2nd generation control CAR-T cells, confirming that it has the highest stemness.

From the results of the T cell subset analysis of this Example, even when the functional activity of CAR-T cells was augmented through the overexpression of the cell signaling pathway modulator(s), it was found that T cell stemness, which is a preferred property of therapeutic CAR-T cells, was not impaired and was still well maintained.

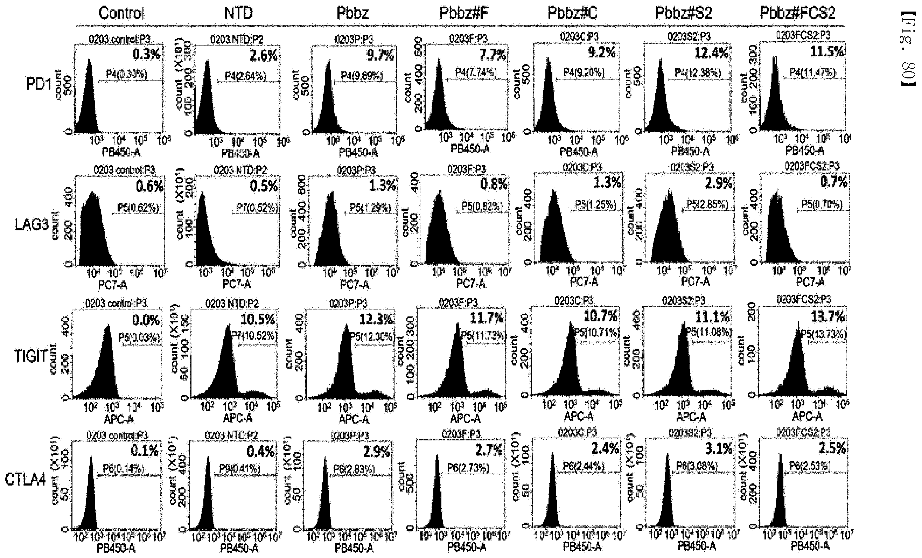

### Phenotype analysis for T cell exhaustion (Figure 80)

As a result of flow cytometric analysis using specific antibodies, it was revealed for the CAR-T cells within the gate of CAR(+) cells that the proportion of PD-1 positive cells was all in the range of 10 +/-3 %, that of LAG-3 positive cells was all less than 3%, that of TIGIT positive cells was all in the range of 12 +/- 3 %, and that of CTLA-4 positive cells was all approximately at the level of 2-3%. In general, these expression levels of T cell exhaustion markers were not considered very high. Therefore, it was indicated that the overexpression of a signaling pathway modulator(s) introduced in the present invention is not likely to accompany CAR-T cell exhaustion.

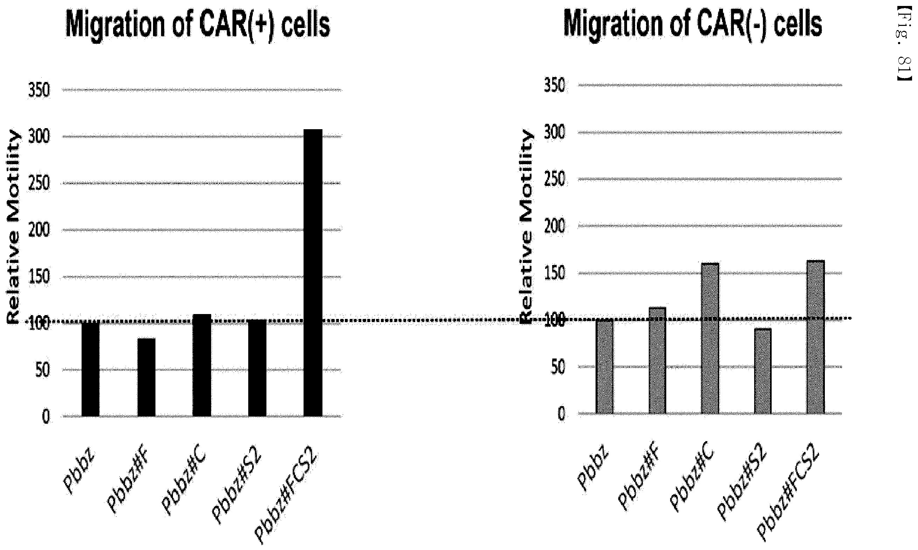

### In vitro migration of PSMA-specific CAR-T cells (Figure 81)

As shown in the bottom graph, the total number of cells migrated to the lower chamber for 1 hour and the CAR(+) % of the migrated cells was measured to compare the motility of CAR(+)-T cells. Although the concentration of total T cells and CAR(+) T cells were adjusted to be the same for all the tested CAR-Ts prior to migration, a specific CAR-T (Pbbz#FCS2) showed significantly higher migration ability compared to the other CAR-Ts.

On the other hand, in the number of CAR (-)-T cells migrated to the lower chamber during the same time, it was found that such a remarkable difference was not shown between the CAR-T samples (top). This suggests that the high migration ability seen in these CAR-T (Pbbz#FCS2) samples is limited to the CAR(+) cells overexpressing three types of signaling pathway modulators.

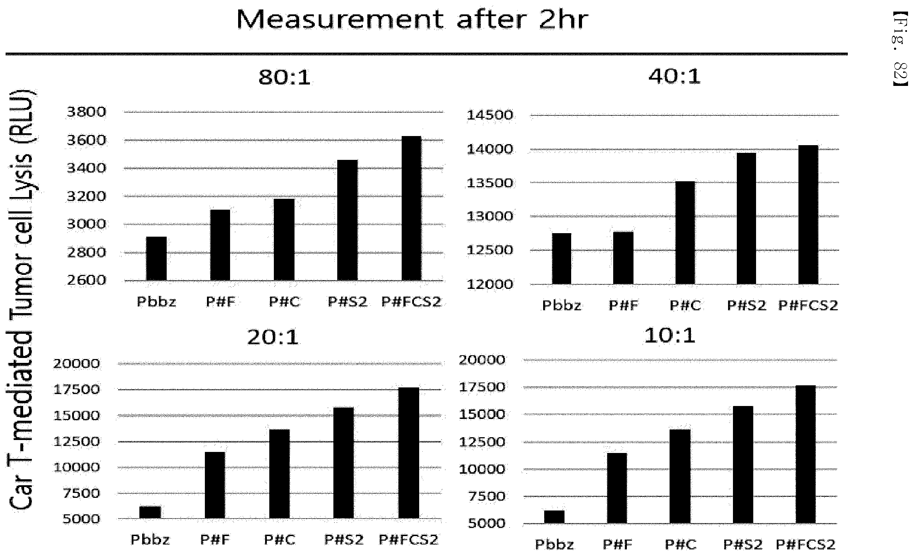

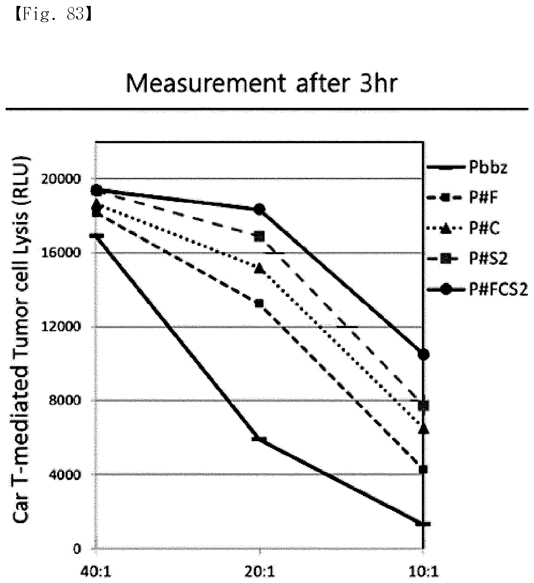

### In vitro tumor cell killing activity of PSMA-specific CAR-T cells (Figures 82 and 83)

As shown in the evaluation results at 2 hour time point (Figure 82) and 3 hour time point (Figure 83) of the reaction, all the three types of CAR-Ts, CAR-T cells (Pbbz#F), and CAR-T cells (Pbbz#C), CAR-T cells (Pbbz#S2), showed significantly higher target tumor cell killing activity than the conventional 2nd generation control CAR-T cells (Pbbz), respectively.

On the other hand, it was confirmed that the CAR-T cells (Pbbz#FCS2) that overexpressed these three types of small-sized cytoplasmic immune modulators at the same time showed remarkably augmented activity over the entire tested range of E:T ratio.

### Mode for Carrying out the Invention

Hereinafter, preferred examples are presented to aid the understanding of the present invention. However, the following examples are merely provided for easier understanding of the present invention, and the contents of the present invention are not limited by the following examples.

### I. Preparation of CAR-T into which a signaling pathway modulator is introduced

### Example 1. Design of constructs of a fusion protein

### Example 1.1. Design of construct of FKBP12

The construct of a fusion protein including a chimeric antigen receptor and FKBP12 was designed to include a signal peptide, an antigen binding domain, a hinge and transmembrane domain, an intracellular signaling domain, a self-cleavage peptide, and FKBP12.

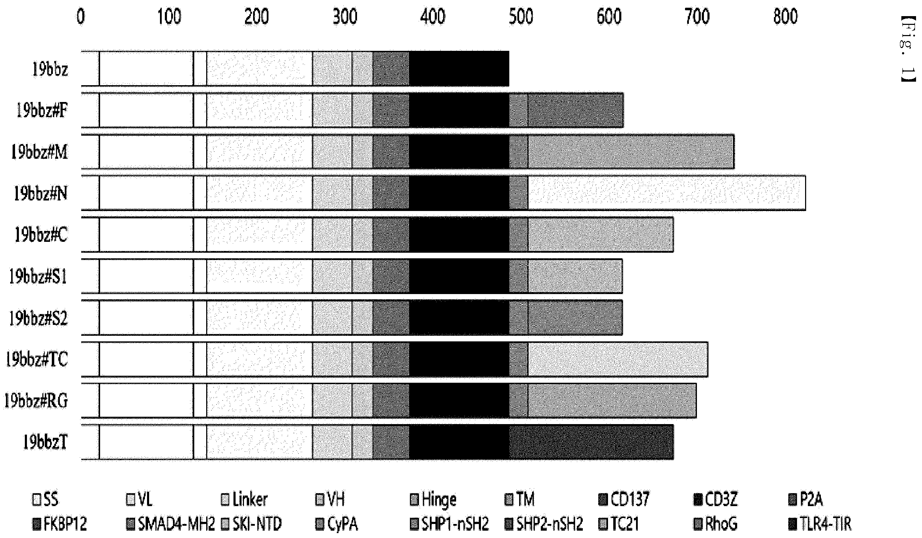

As an example, as shown in Figure 1, it was designed to include genes for a signal peptide (ss), an antigen binding domain (CD19 scFv), a hinge and transmembrane domain (H+TM), an intracellular signaling domain (4-1BB and CD3Z), a self-cleavage peptide (P2A) and FKBP12, a whole construct of which was referred to as "19bbz#F." In contrast, a chimeric antigen receptor including a signal peptide (ss), an antigen binding domain (CD19 scFv), a hinge and transmembrane domain (H+TM) and an intracellular signaling domain (4-1BB and CD3Q was referred to as "19bbz."

Specifically, the CD19-specific CAR was constructed by linking i) a nucleotide sequence derived from a CD8 signal peptide (Uniprot: P01732-1, 1-21 aa, SEQ ID NO: 1) and ii) a nucleotide sequence to a fragment of scFv derived from a CD19-specific FMC63 antibody (Nichoison et al, 1997) (SEQ ID NO: 3), and then linking nucleotide sequences derived from iii) a CD8 hinge-transmembrane domain (Uniprot: P01732-1, 138-206 aa, SEQ ID NO: 5), iv) a human 4-1BB co-stimulatory factor domain cytoplasmic region (Uniprot: Q07011, 214-255 aa, SEQ ID NO: 7), and v) a human CD3ζ intracellular domain (GenBank: NP 000725.1, 52-163 aa, SEQ ID NO: 9).

### Example 1.2. Design of vector construct including other signaling pathway modulators

In the same manner as in Example 1.1., it was designed to include Cyclophilin A (#C), the C-terminal MH2 domain of the SMAD4 protein (#M), N-SKI (#N), the N-terminal SH2 domain of the SHP-1 protein (#S1), the N-terminal NH2 domain of the SHP-2 protein (#S2), TC21 (#TC), RhoG (#RG), FKBP12/Cyclophilin A/the N-terminal SH2 domain of the SHP-2 protein (#F#C#S2, or briefly #FCS2), instead of FKBP12. These were referred to as "19bbz#C," "19bbz#M," "19bbz#N," "19bbz#S1," "19bbz#S2," "19bbz#TC," "19bbz#RG," and "19bbz#FCS2," respectively. In addition, a construct was made in the same manner as in Example 1.1, except that FKBP12 is replaced by a TLR4 intracellular domain (T) without a self-cleavage sequence, and referred to as "19bbzT" (Figures 1 to 2).

### Example 2. Construction of a vector encoding a fusion protein

### Example 2.1. Construction of a vector including FKBP12

As a vector, pPVLV5 vector, which is a 3rd generation self-inactive lentiviral vector including a human elongation factor α (EF1α: 531 bp or 212 bp) promoter, was used. A gene encoding a chimeric antigen receptor including a signal peptide (ss), a single chain variable fragment specifically binding to CD19 (CD19 scFv), a hinge and transmembrane domain of human CD8 (H+TM), and an intracellular signaling domain (4-1BB and CD3Q, and a self-cleavage peptide (P2A) and FKBP12 was inserted into the pPVLV5 vector, and referred to as "p_19bbz#F."

In addition, a gene encoding a chimeric antigen receptor including a signal peptide (ss), a single chain variable fragment specifically binding to CD19 (CD19 scFv), a hinge and transmembrane domain of human CD8 (H+TM), and an intracellular signaling domain (4-1BB and CD3Q was inserted into the pPVLV5 vector, and referred to as "p_19bbz."

The amino acid sequence and nucleotide sequence forf the chimeric antigen receptor used in the experiment are shown in Table 1 below, and the amino acid and nucleotide sequences of FKBP12 (#F) are shown in Table 2 below.

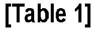

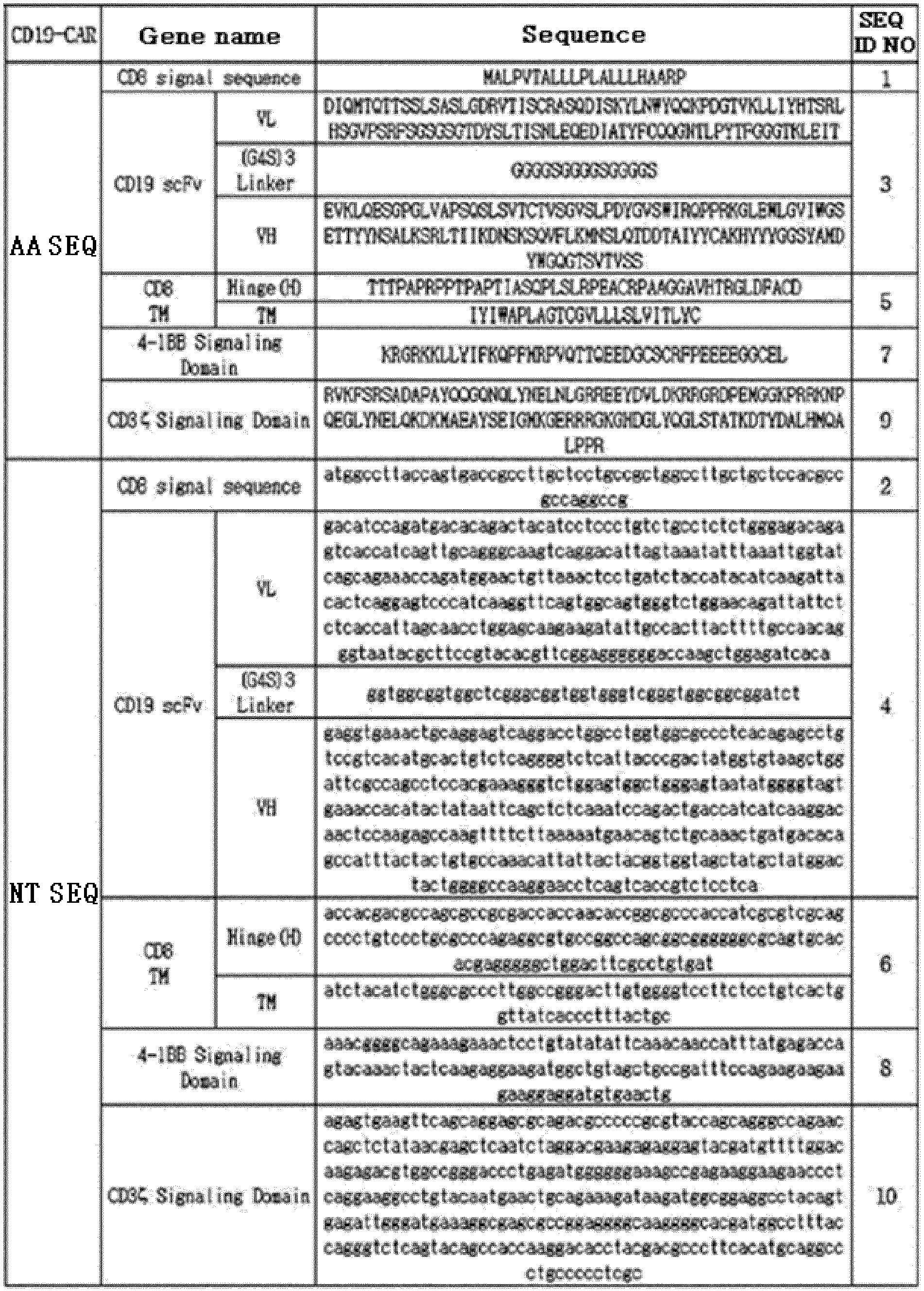

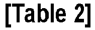

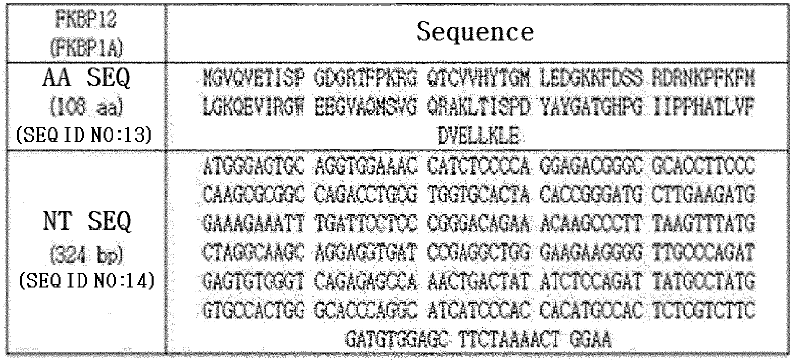

As a vector, a 3rd generation lentiviral vector modified with pLenti-EF1a-Backbone (NG) (Addgene, #27963) was used, and a gene encoding CAR was loaded onto the vector by using DNA assembly Master Mix (NEB, #E2621).

In addition, in order to express the immunomodulatory protein in the cytoplasm, a nucleotide sequence encoding the immunomodulatory protein was inserted to downstream of the CAR sequence. The immunomodulatory protein was FKBP12 (#F, SEQ ID NO: 14).

### Example 2.2. Construction of vector encoding CAR and genes for other signaling pathway modulators

In the same manner as in Example 2.1., using a lentiviral vector, a vector was constructed to include cyclophilin A (#C, SEQ ID NO: 25), the C-terminal MH2 domain of the SMAD4 protein (#M, SEQ ID NO: 21), N-SKI (#N, SEQ ID NO: 23), the N-SH2 domain of the SHP-1 protein (#S1, SEQ ID NO: 27), the N-SH2 domain of the SHP-2 protein (#S2, SEQ ID NO: 29), TC21 (#TC, SEQ ID NO: 33), RhoG (#RG, SEQ ID NO: 35), or FKBP12/cyclophilin A/the N-SH2 domain of the SHP2 protein (#FCS2, SEQ ID NO: 14, SEQ ID NO: 25, SEQ ID NO: 29). These were referred to as "p_19bbz#F" "p_19bbz#C," "p_19bbz#M," "p_19bbz#N," "p_19bbz#S1," "p_19bbz#S2," "p_19bbz#TC" "p_19bbz#RG," and "p_19bbz#F#CS#2," respectively. In addition, in the same manner as in Example 2.1., a vector was constructed to include a TLR4 cytoplasmic domain (T, SEQ ID NO: 31) instead of FKBP12 without a self-cleavage sequence, and it was referred to as "p_19bbzT."

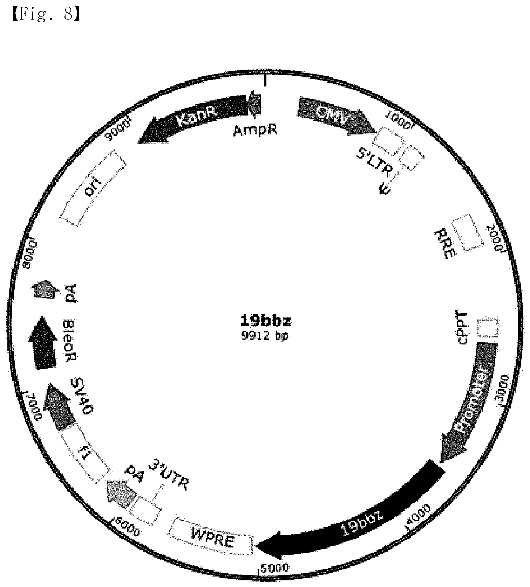

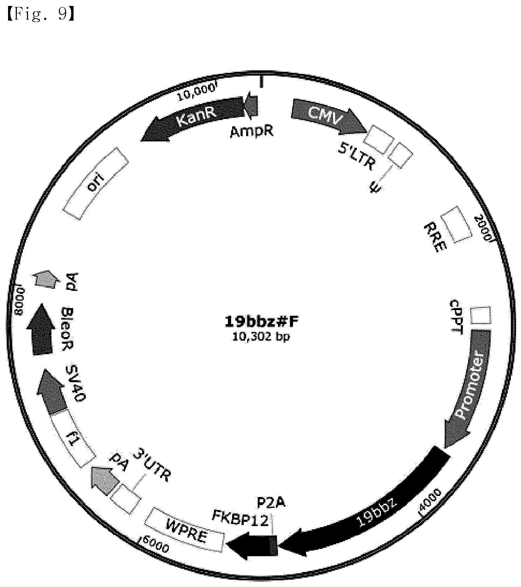

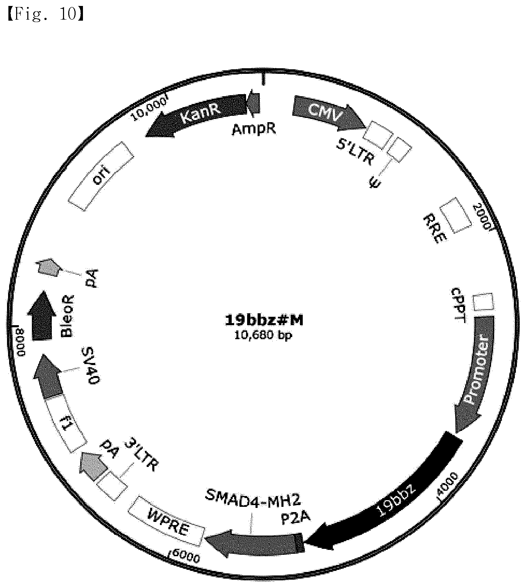

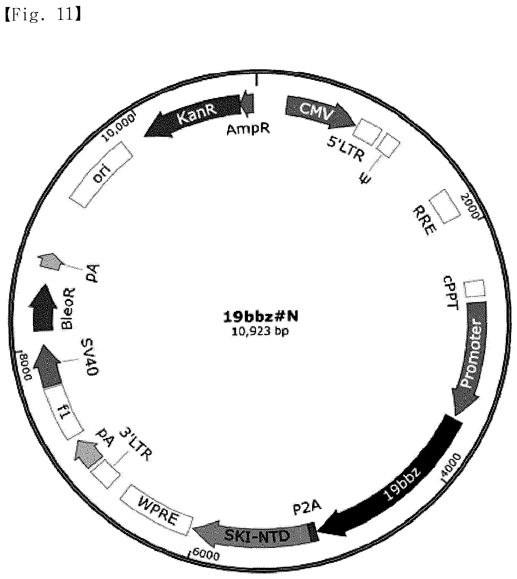

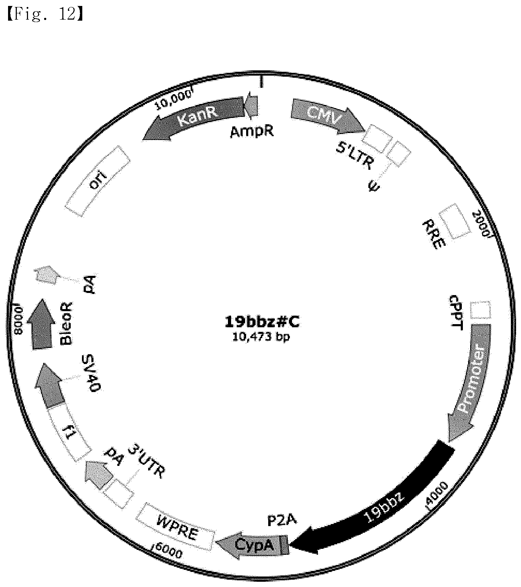

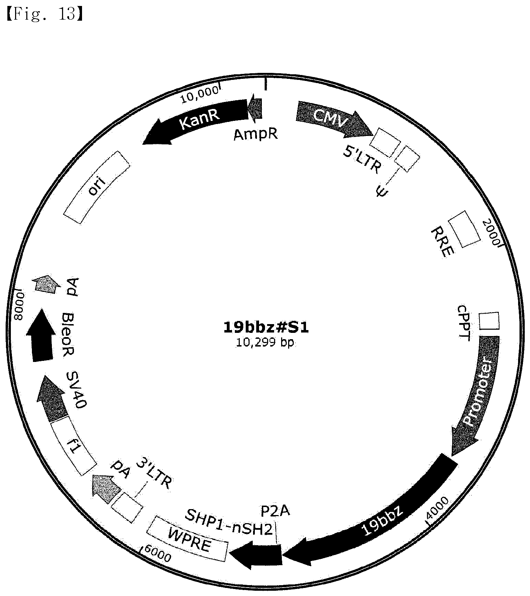

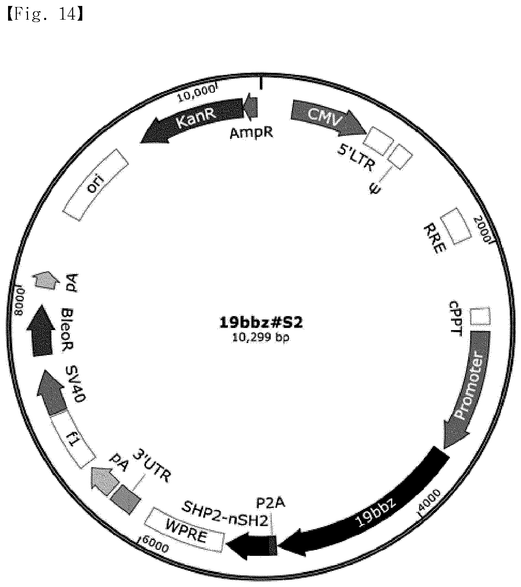

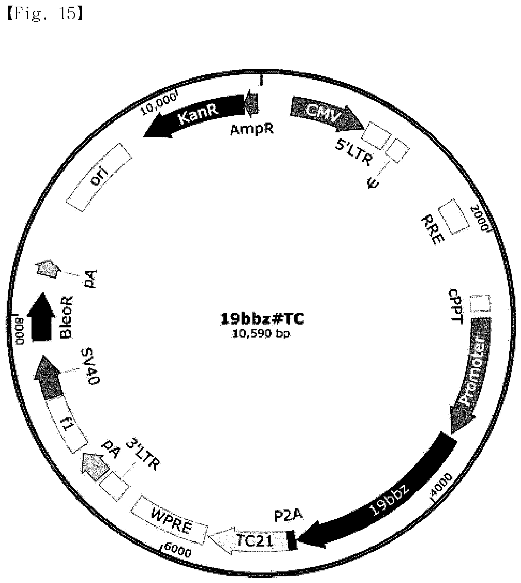

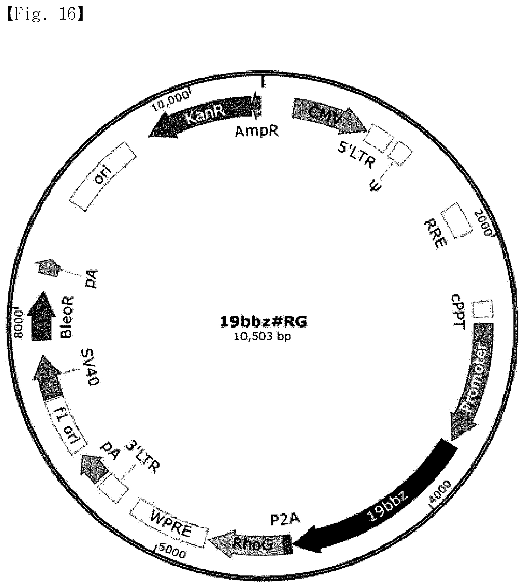

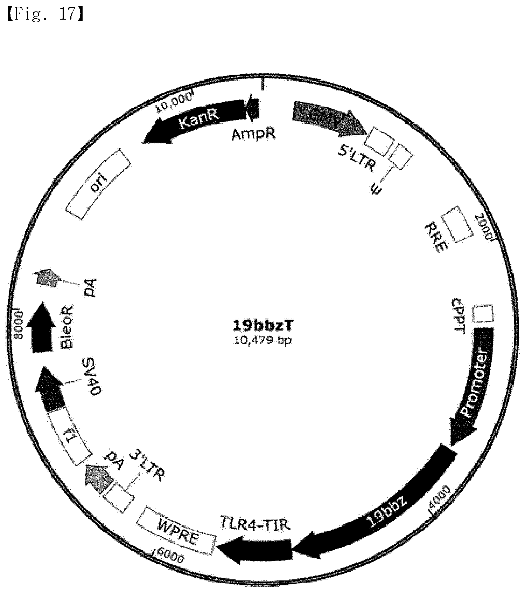

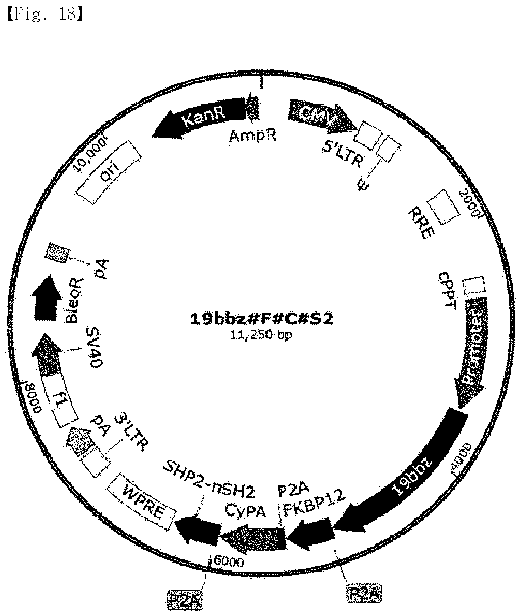

The amino acid sequences and nucleotide sequences corresponding to proteins or fragments thereof used in the preparation of the p_19bbz#C, p_19bbz#M, p_19bbz#N, p_19bbz#S1, p_19bbz#S2, "p_19bbz#TC," p_19bbz#RG, p_19bbz#F#C#S2 and p_19bbzT vectors are shown in Tables 3 to 10 below. In addition, the structural diagram of each plasmid is shown in Figures 8 to 18.

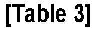

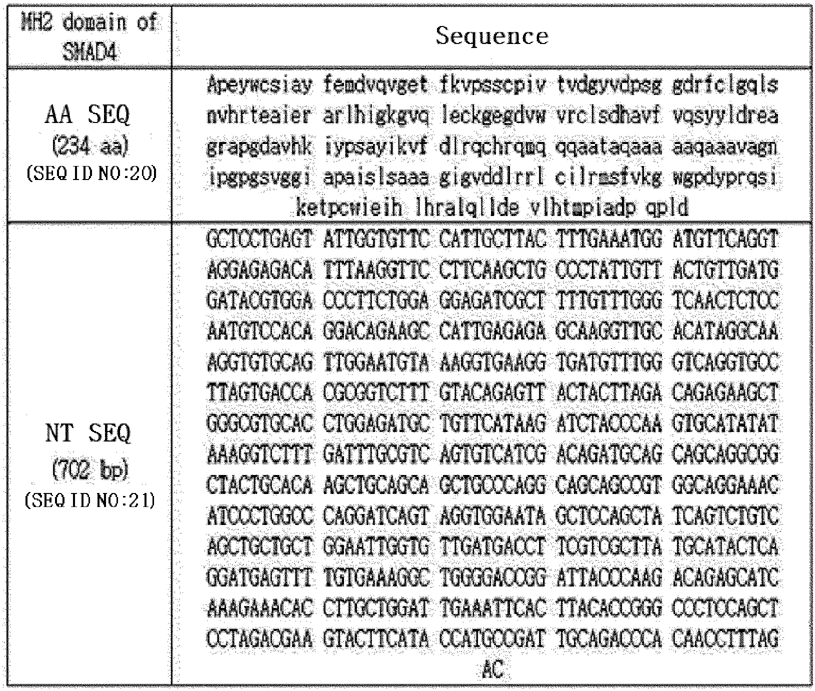

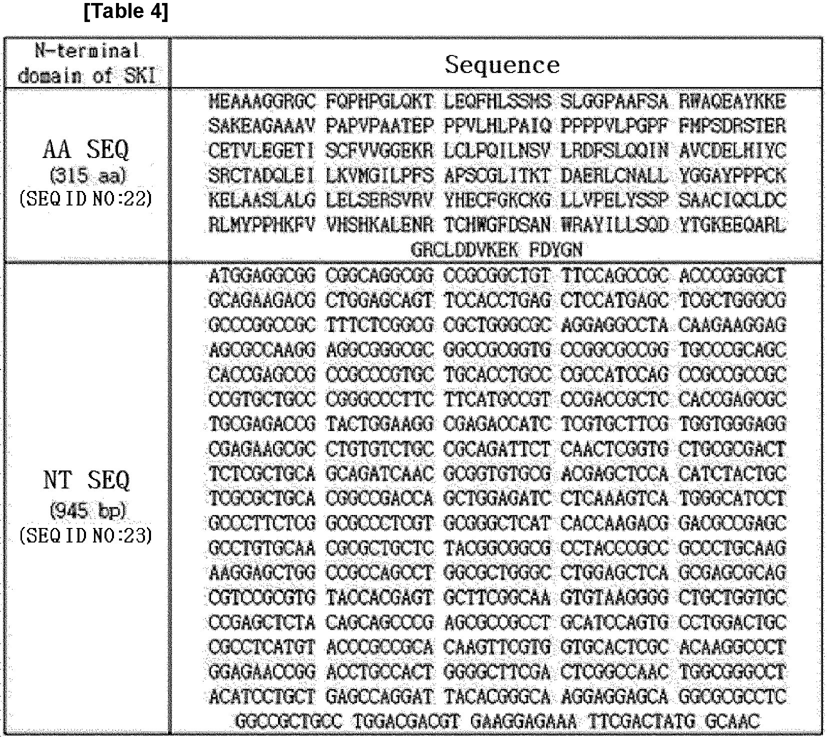

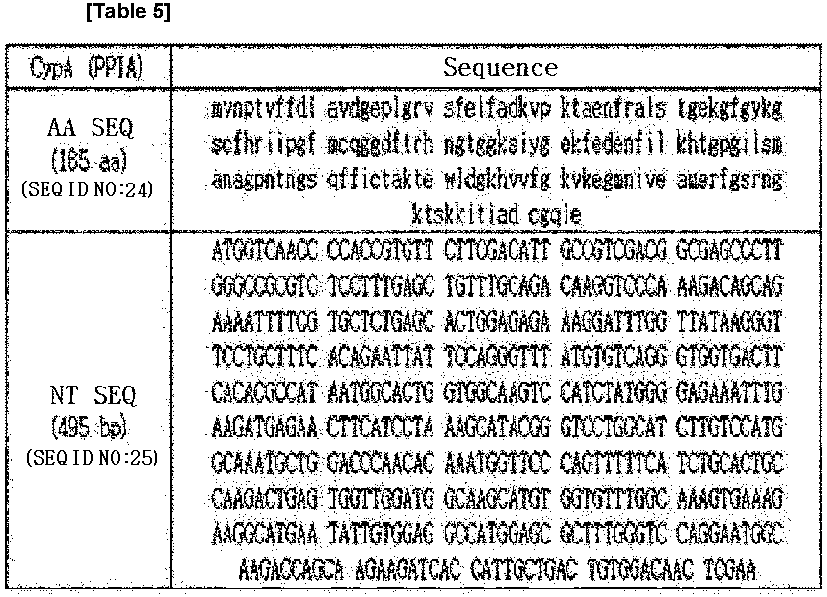

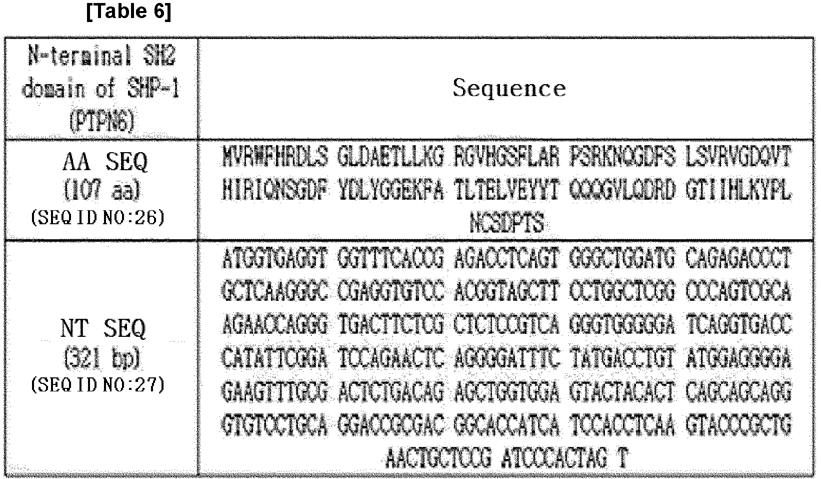

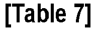

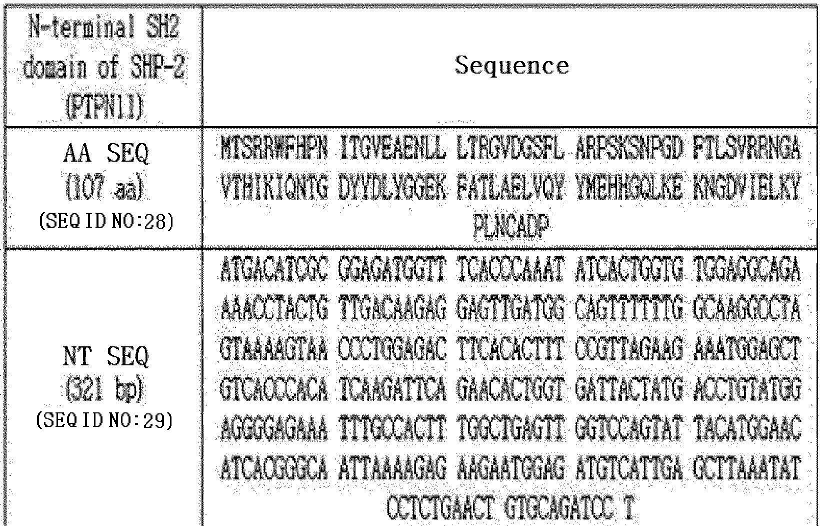

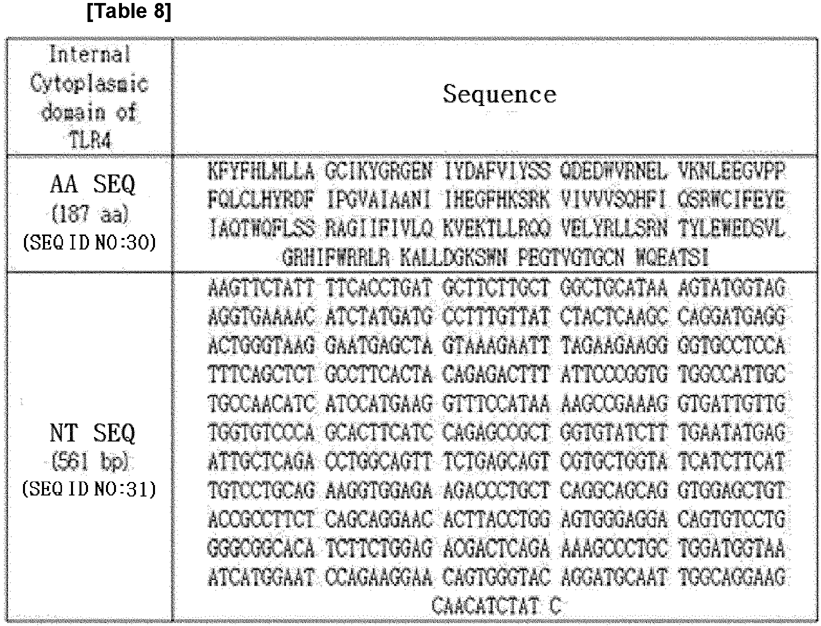

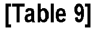

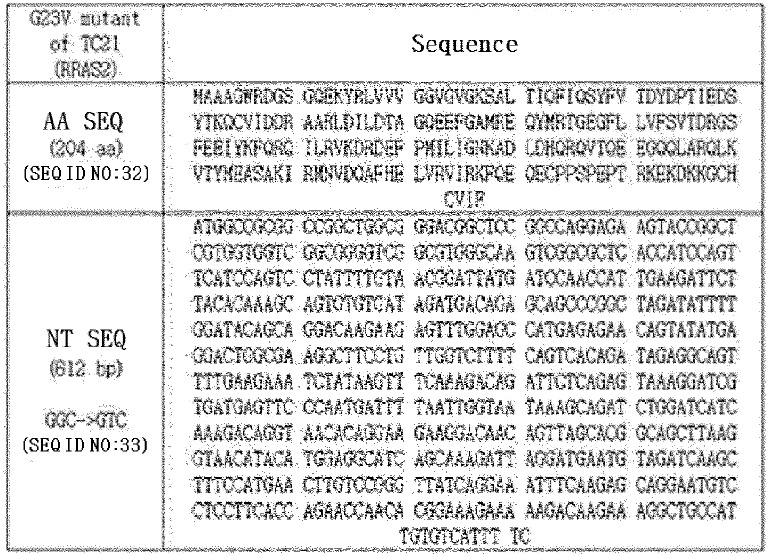

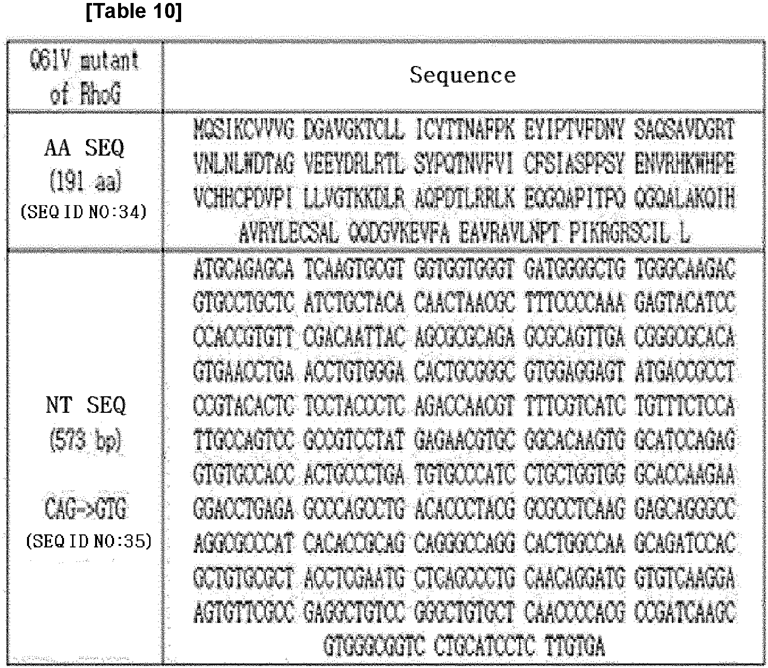

### Example 3. Production of lentivirus

### Example 3.1. Production of lentivirus containing genes for CAR and FKBP12

In order to produce each lentiviral batch, an optimized lentivirus production kit, LV-MAX Lentiviral Production System (Gibco, #A35684) was used. The 3rd generation lentivirus transfer plasmid constructed above was used to co-transfect pMD2.G (Addgene, #12259), pMDLg/pRRE (Addgene, #12251) and pRSV-Rev (Addgene, #12253) with modified lentivirus envelope plasmid and packaging plasmid to prepare a lentivirus. After transducing the vector into HEK293F cells adapted to a serum-free medium and culturing for 50 to 54 hours, the culture medium including the virus was collected and concentrated using a Lenti-X concentrator (Takara, #631232). The concentrated virus was stored at -80°C until use. The produced lentivirus was referred to as "Lenti_19bbz#F."

### Example 3.2. Production of lentivirus containing genes for CAR and other signaling pathway modulators

In the same manner as in Example 3.1., the p_19bbz#C, p_19bbz#M, p_19bbz#N, p_19bbz#S1, p_19bbz#S2, p_19bbz#TC, p_19bbz#RG, "p_19bbz#F#CS#2," and p_19bbzT lentivirus transfer plasmids prepared in Example 2.2 were used to produce lentiviruses, and these were referred to as "Lenti_19bbz#C," "Lenti_19bbz#M," "Lenti_19bbz#N," "Lenti_19bbz#S1," "Lenti_19bbz#S2," "Lenti_19bbz#TC," "Lenti_19bbz#RG," "Lenti_19bbz#F#C#S2" and "Lenti_19bbzT," respectively.

### Example 4. Construction of CAR-T cells

### Example 4.1. Construction of CAR-T cells into which FKBP12 is introduced

CAR-T cells were constructed using healthy donor peripheral blood mononuclear cells (PBMCs). The adherent cells were removed from PBMCs, and then cultured in X-VIVO 15 medium (Lonza) medium including 200 IU/mL of rhlL-2 (BMI Korea) and anti-CD3/CD28 Dynabeads (Life Technologies) to induce activation of T cells (bead:CD3+T cell=1:1).

Two days later, the two types of lentiviruses (Lenti_19bbz#F and Lenti_19bbz) produced in Example 3.1. were treated with activated T cells, respectively. The next day, the centrifugation was performed to remove the supernatant including the lentiviruses. The T cells were resuspended at 3×10⁵ cells/mL in X-VIVO 15 medium containing 200 IU/mL of rhlL-2 and cultured for 3 days. Thereafter, anti-CD3/CD28 Dynabeads were removed and cultured, and the T cells were proliferated by replacing a new medium including rhlL-2 every 2 or 3 days. In this case, CAR-T cells prepared using Lenti_19bbz#F were referred to as "CAR-T cell_19bbz#F," and CAR-T cells prepared using Lenti_19bbz were referred to as "CAR-T cell_19bbz."

### Example 4.2. Construction of CAR-T cells into which other signaling pathway modulators are introduced

In the same manner as in Example 4.1., the "Lenti_19bbz#C," "Lenti_19bbz#M," "Lenti_19bbz#N," "Lenti_19bbz#S1," "Lenti_19bbz#S2," "Lenti_19bbz#TC," "Lenti_19bbz#RG," "Lenti_19bbz#F#C#S2" and "Lenti_19bbzT" lentiviruses produced in Example 3.2. were used to transform and construct CAR-T cells, and theses were referred to as "CAR-T cell_19bbz#C," "CAR-T cell_19bbz#M," "CAR-T cell_19bbz#N," "CAR-T cell_19bbz#S1," "CAR-T cell_19bbz#S2," "CAR-T cell_19bbz#TC," "CAR-T cell_19bbz#RG," "CAR-T cell_19bbz#FCS2," and "CAR-T cell_19bbzT," respectively.

### Example 5. Preparation of Her2-specific CAR-T

Her2-specific CAR-T was prepared in the same manner as in Examples 1 to 4, except that an antigen binding CD19 scFv domain is replaced by Her2 scFv.

Specifically, the Her2-specific CAR was constructed by linking i) a nucleotide sequence derived from a CD8 signal peptide (Uniprot: P01732-1, 1-21 aa, SEQ ID NO: 1) and ii) a nucleotide sequence for a fragment of scFv (SEQ ID NO: 45) derived from a Her2-specific antibody, trastuzumab (IMGT database) (SEQ ID NO: 46), and then linking iii) a CD8 hinge-transmembrane domain (SEQ ID NO: 5), iv) a human 4-1BB co-stimulatory factor domain cytoplasmic region (SEQ ID NO: 7), and v) a nucleotide sequence derived from a human CD3ζ intracellular domain (SEQ ID NO: 9) (Table 11).

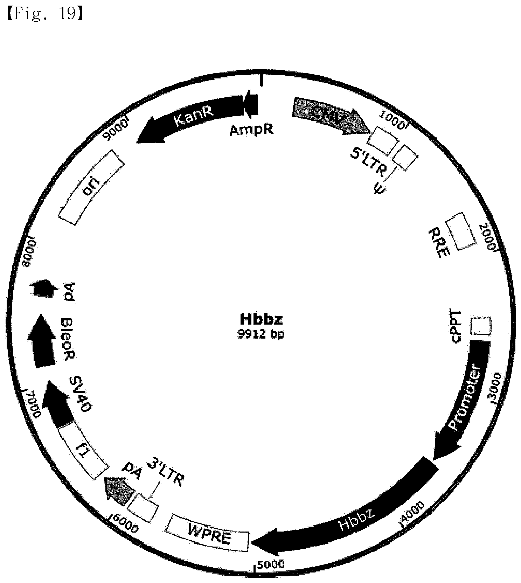

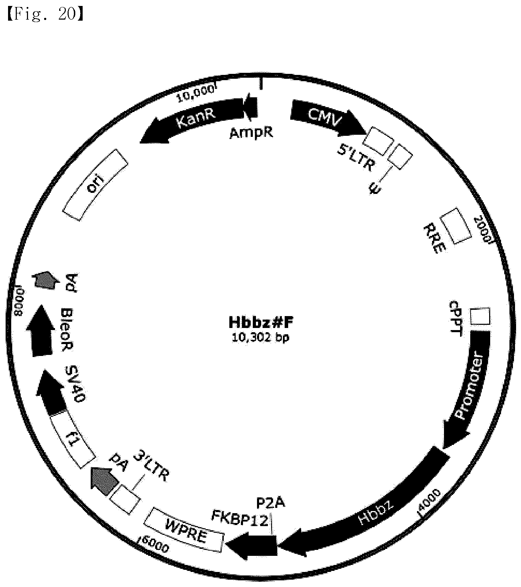

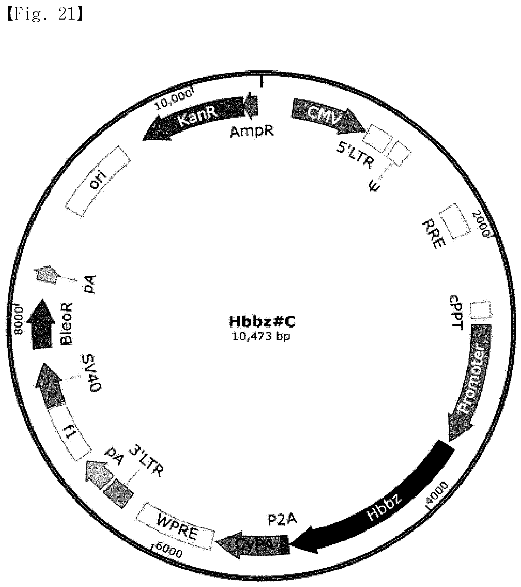

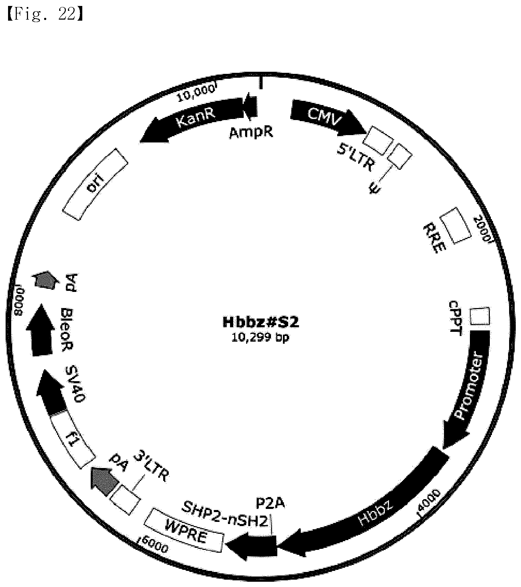

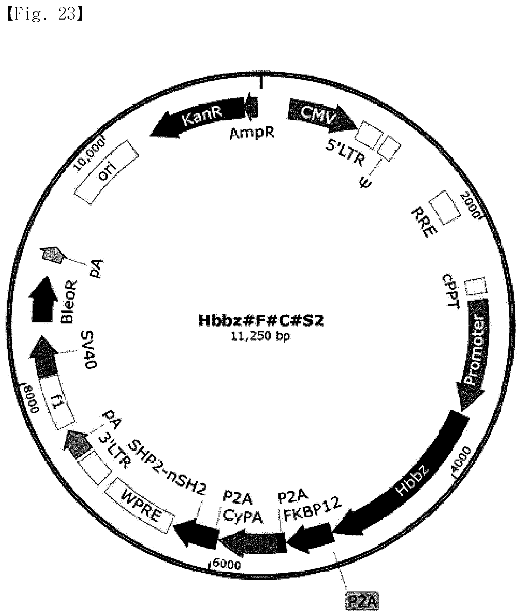

In addition, various signaling pathway modulators expressed together with Her2-specific CAR were also prepared. Specifically, the Her2 specific CAR-T expressing the FKBP12 protein, cyclophilin A and SHP2-nSH2 were prepared, respectively. In addition, CAR-T in which all of the FKBP12 protein, cyclophilin A, and SHP2-nSH2 were expressed was prepared. The vectors used in this case are as shown in Figures 19 to 23.

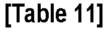

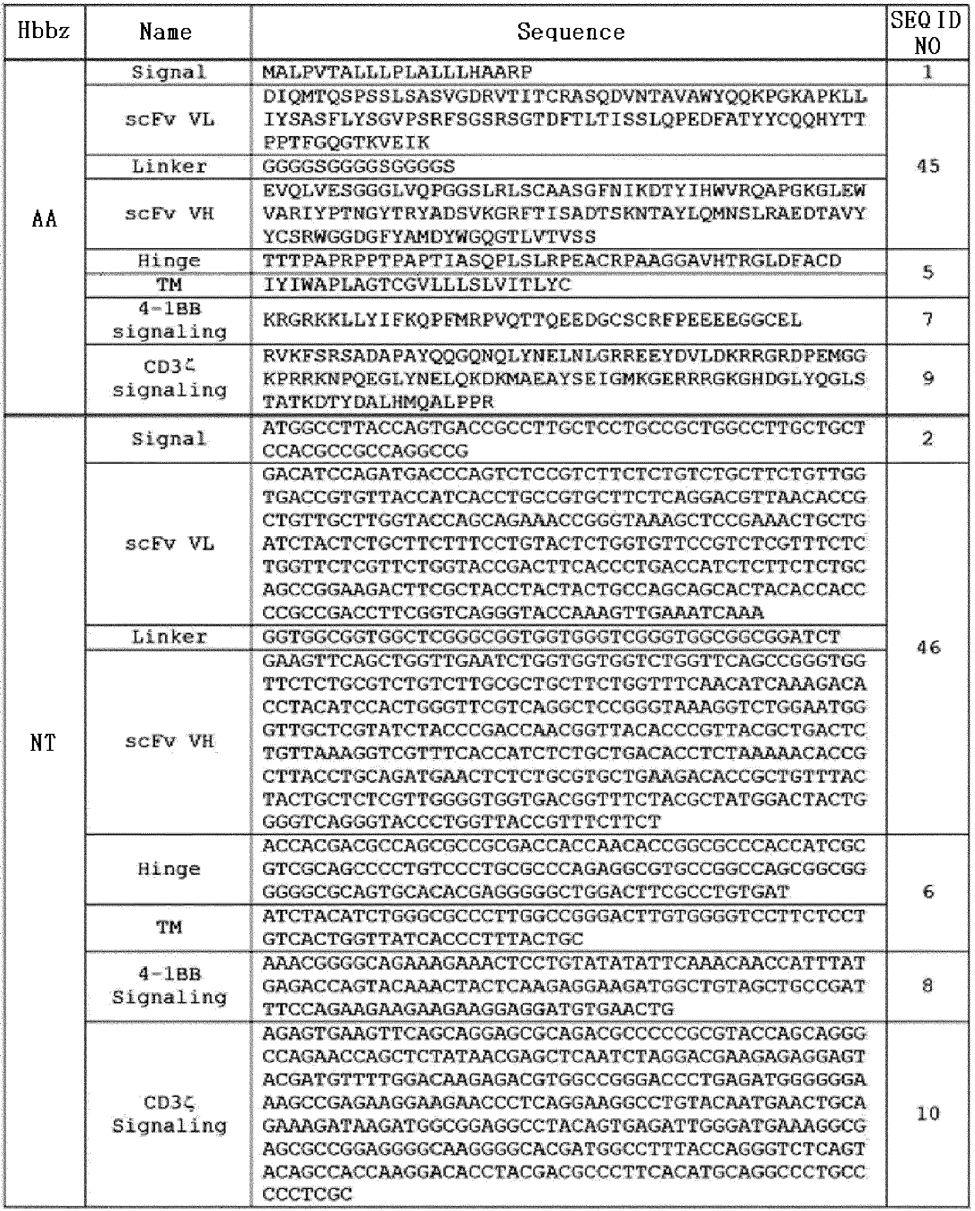

### Example 6. Preparation of PSMA-specific CAR-T

In the same manner as in Examples 1 to 4, PSMA-specific CAR-T was prepared, except that an antigen binding CD19 scFv domain is replaced by PSMA-scFv.

Specifically, the PSMA-specific CAR was constructed by linking i) a nucleotide sequence derived from a signal peptide (GenBank AAA51634.1, 1-19 aa, SEQ ID NO: 52) derived from Ig heavy chain and ii) a nucleotide sequence for a fragment of scFv (SEQ ID NO: 55) derived from a PSMA-specific J591 antibody (WO2002/098897A2), and then linking iii) a CD8 hinge-transmembrane domain (SEQ ID NO: 5), iv) a human 4-1BB co-stimulatory factor domain cytoplasmic region (SEQ ID NO: 7) and v) a nucleotide sequence derived from a human CD3ζ intracellular domain (SEQ ID NO: 9) (Table 12).

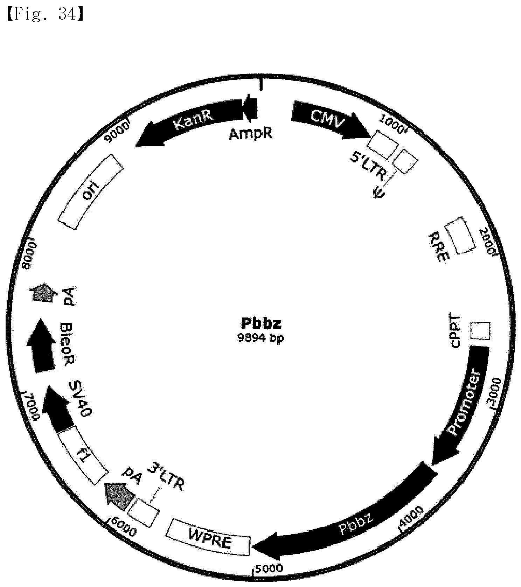

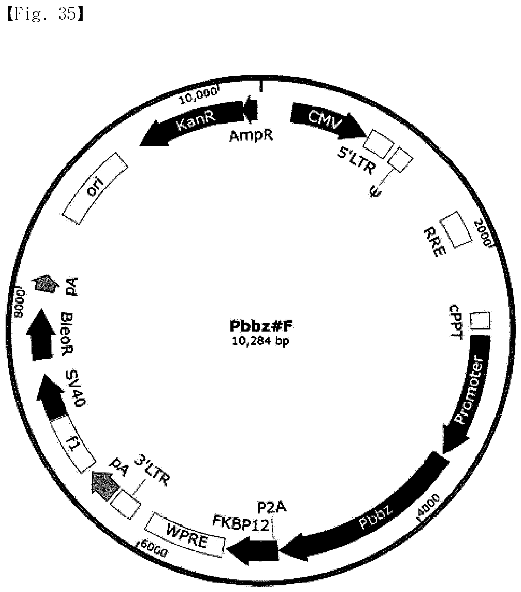

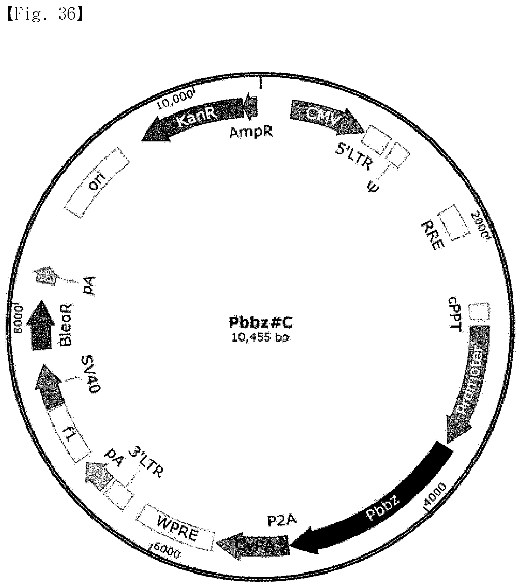

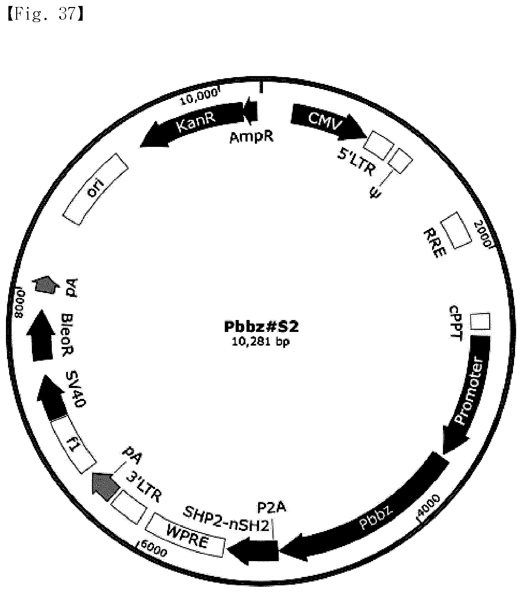

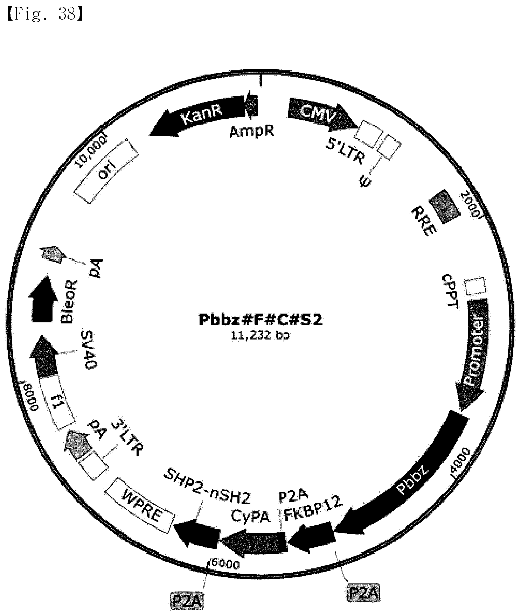

In addition, various signaling pathway modulators expressed together with PSMA-specific CAR were also prepared. Specifically, the PSMA-specific CAR-T expressing the FKBP12 protein, cyclophilin A and SHP2-nSH2 were prepared, respectively. In addition, CAR-T in which all of the FKBP12 protein, cyclophilin A, and SHP2-nSH2 were expressed was prepared. The vectors used in this case are as shown in Figures 34 to 38.

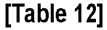

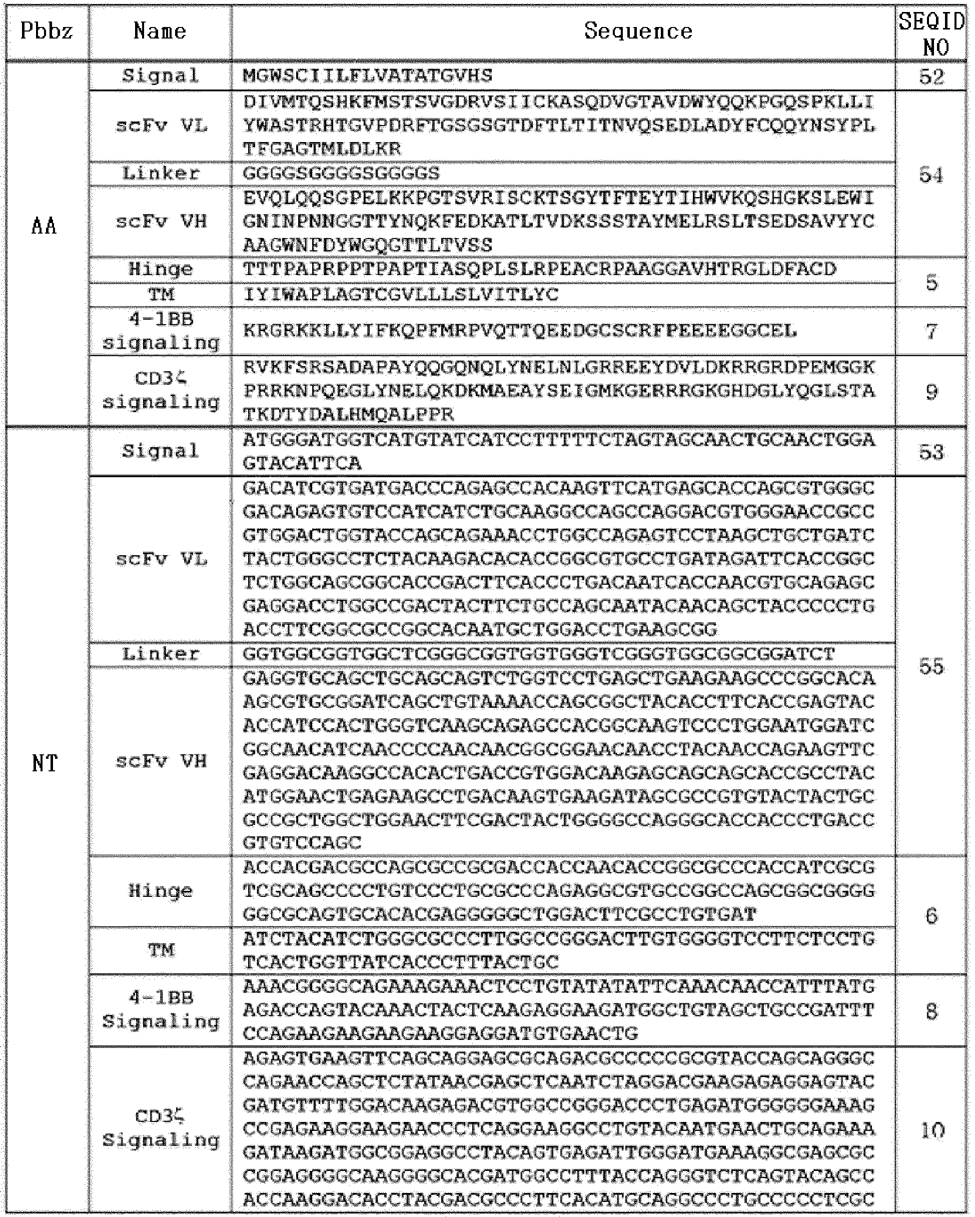

### Example 7. Preparation of CD43-specific CAR-T

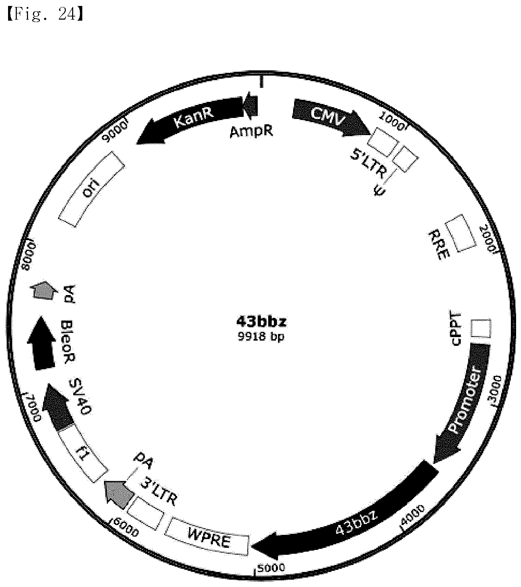

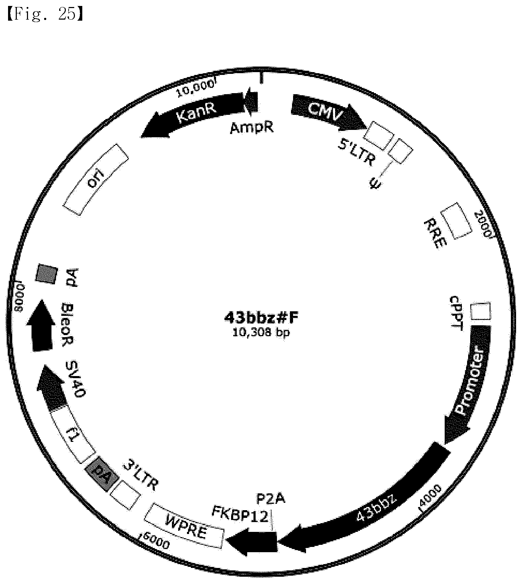

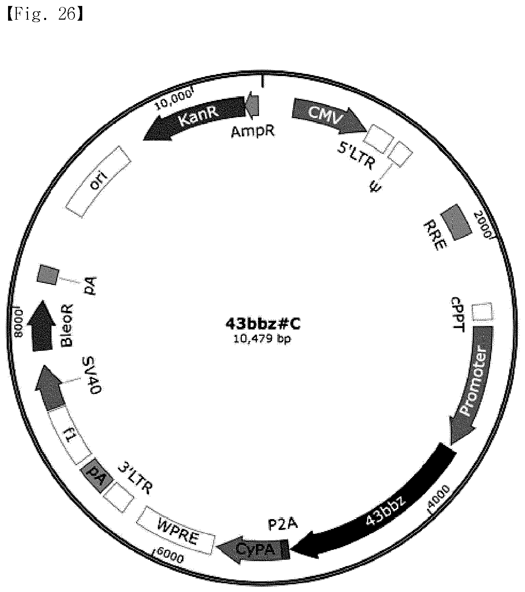

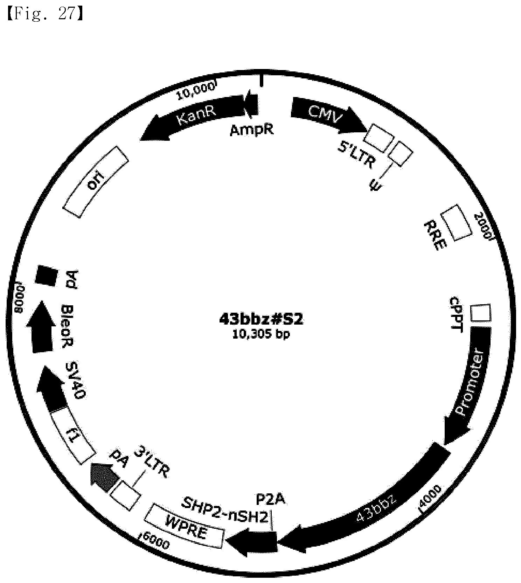

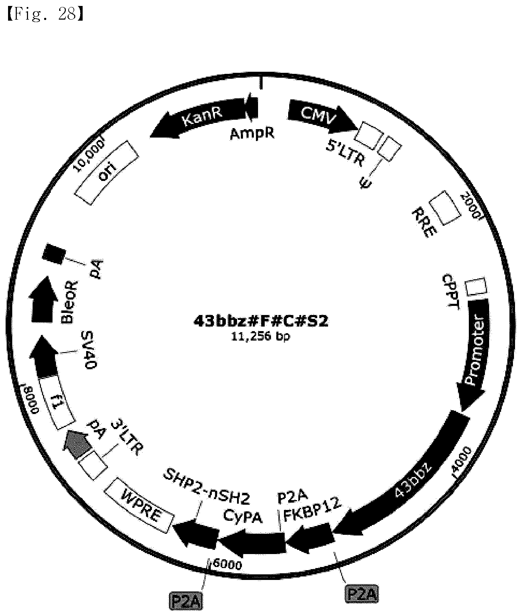

CD43-specific CAR-T was prepared in the same manner as in Examples 1 to 4, except that an antigen binding CD19 scFv domain is replaced by CD43 scFv. The sequences used at this time are shown in Table 13 below. In addition, various signaling pathway modulators expressed together with CD43-specific CAR were also prepared. Specifically, the CD43-specific CAR-T cells expressing the FKBP12 protein, cyclophilin A and SHP2-nSH2 were prepared, respectively. In addition, CAR-T in which all of the FKBP12 protein, cyclophilin A, and SHP2-nSH2 were expressed was prepared. The vectors used in this case are as shown in Figures 24 to 28.

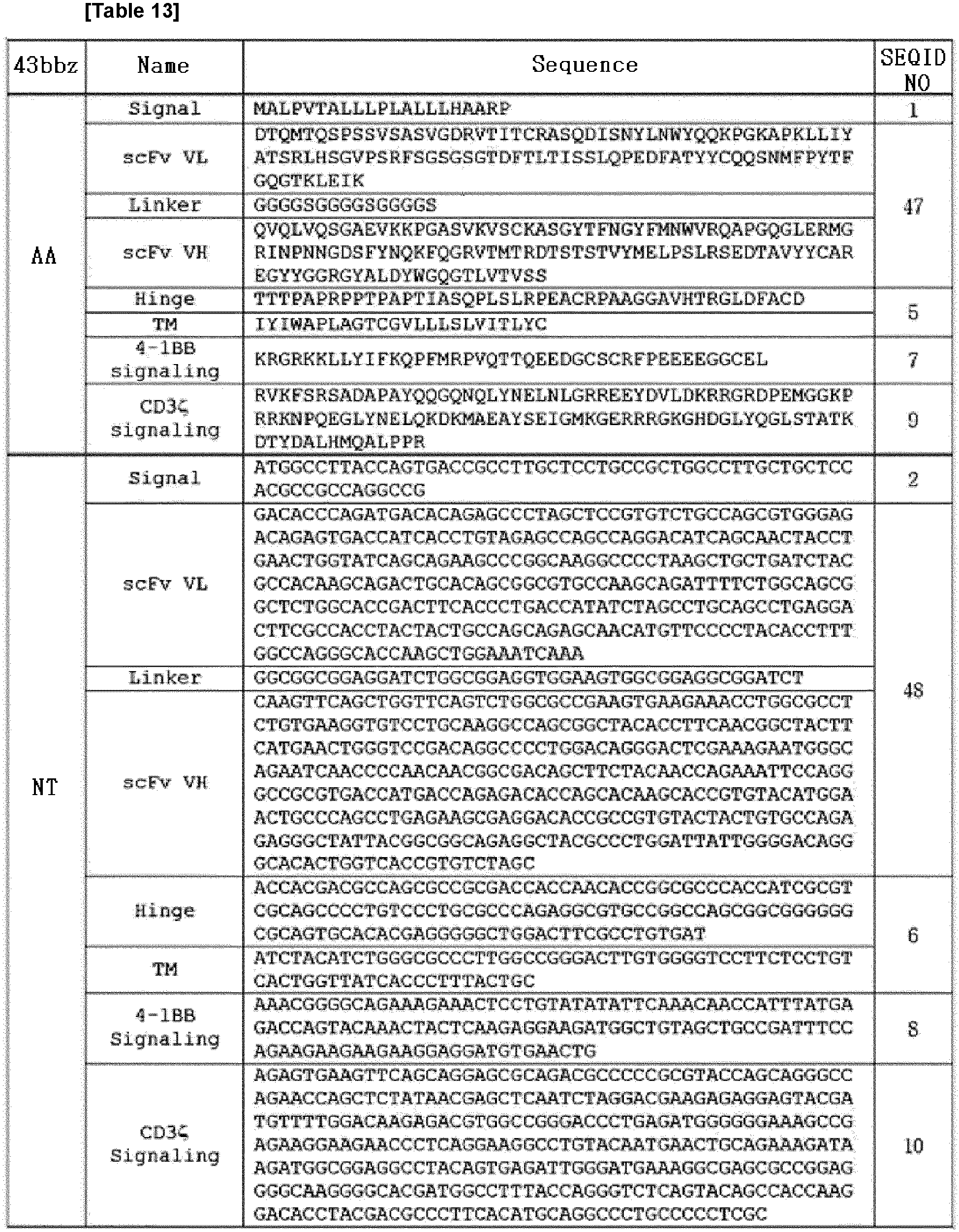

### Example 8. Preparation of CD47-specific CAR-T

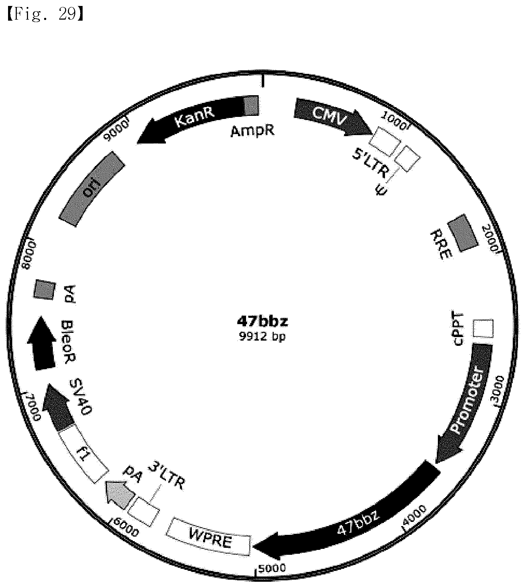

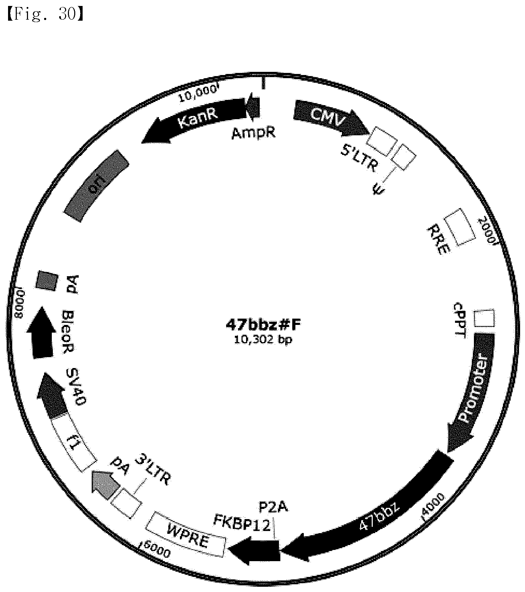

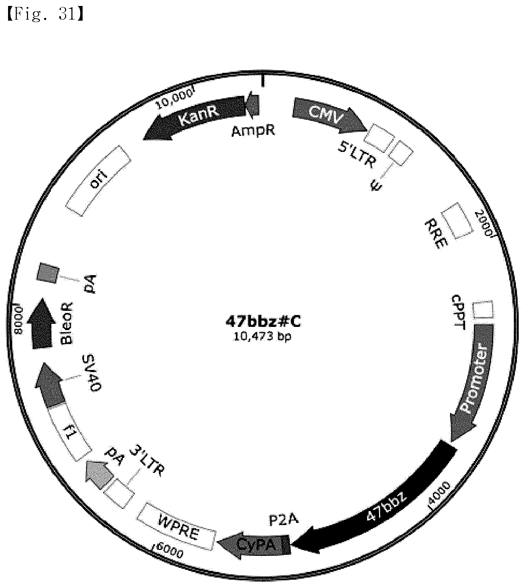

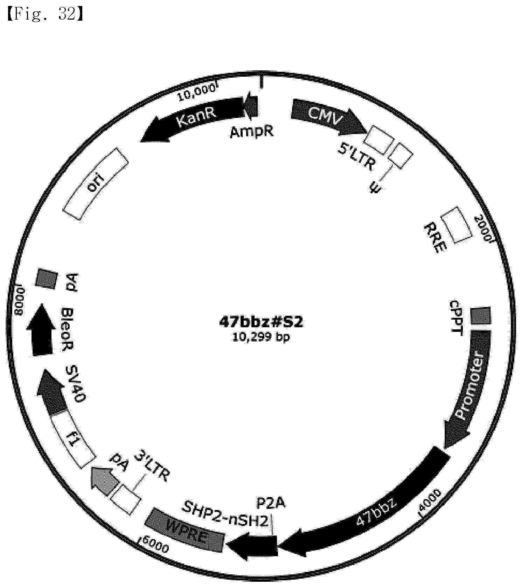

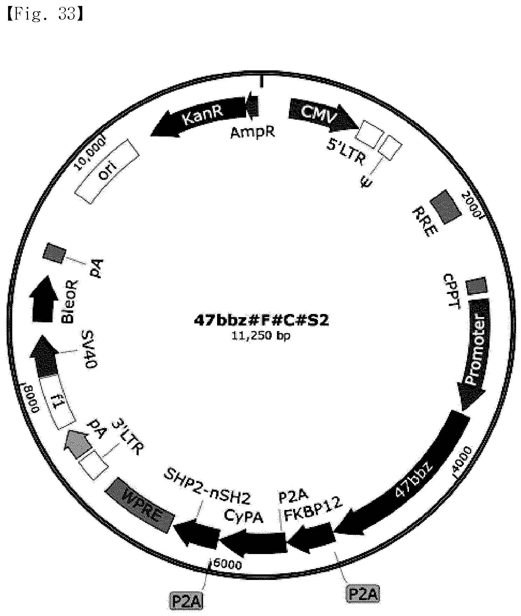

CD47-specific CAR-T was prepared in the same manner as in Examples 1 to 4, except that an antigen binding CD19 scFv domain is replaced by CD47 scFv. The sequences used in this case are shown in Table 14 below. In addition, various signaling pathway modulators expressed together with CD47-specific CAR were also prepared. Specifically, the CD47-specific CAR-T cells expressing the FKBP12 protein, cyclophilin A and SHP2-nSH2 were prepared, respectively. In addition, CAR-T in which all of the FKBP12 protein, cyclophilin A, and SHP2-nSH2 were expressed was prepared. The vectors used in this case are as shown in Figures 29 to 33.

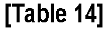

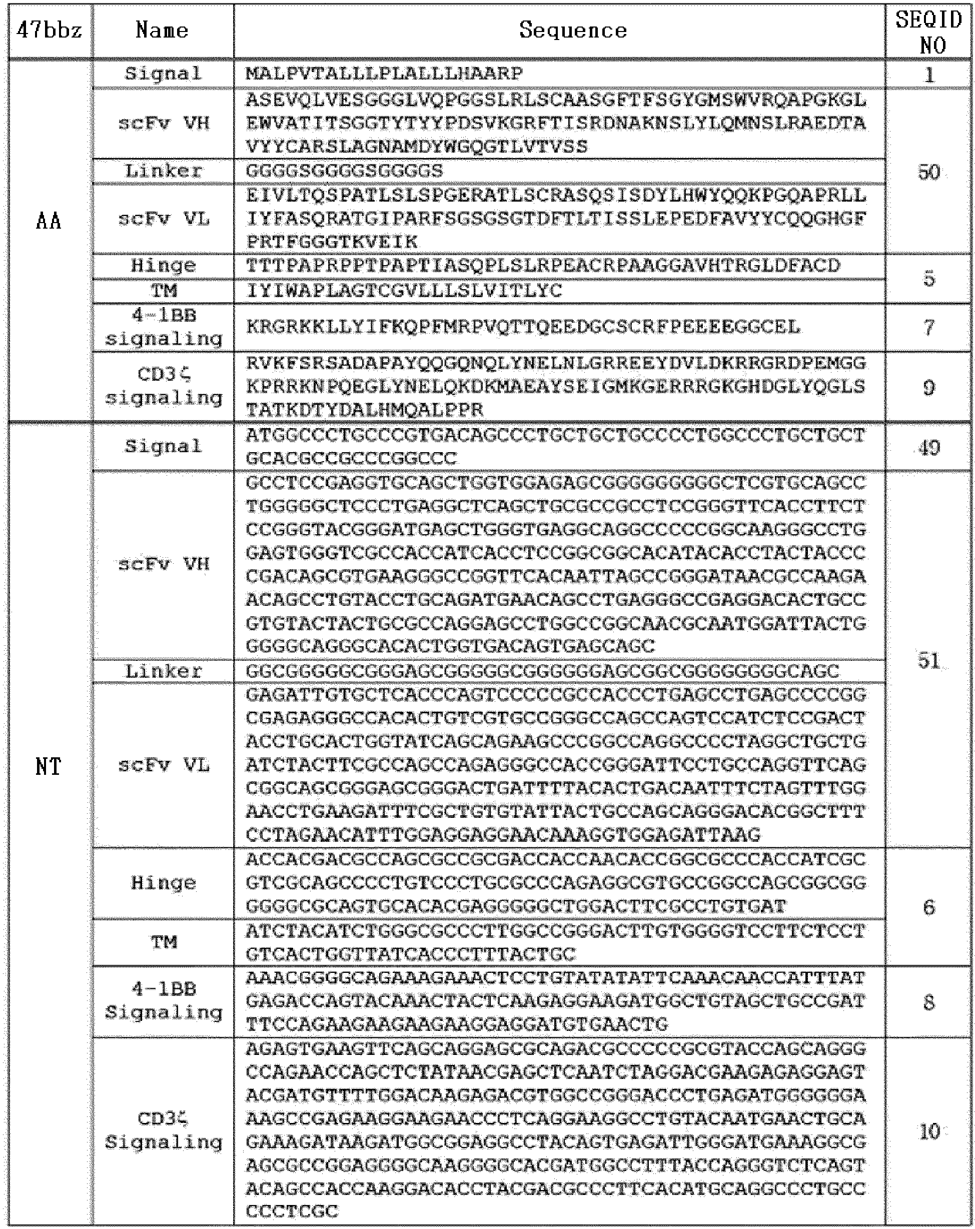

### Example 9. Preparation of HERV-E TCR-T

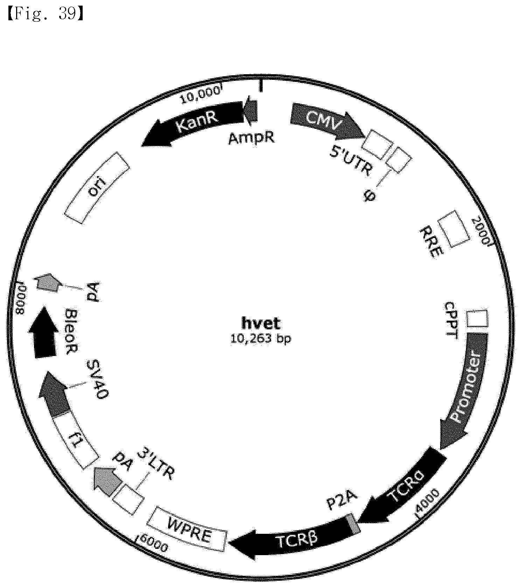

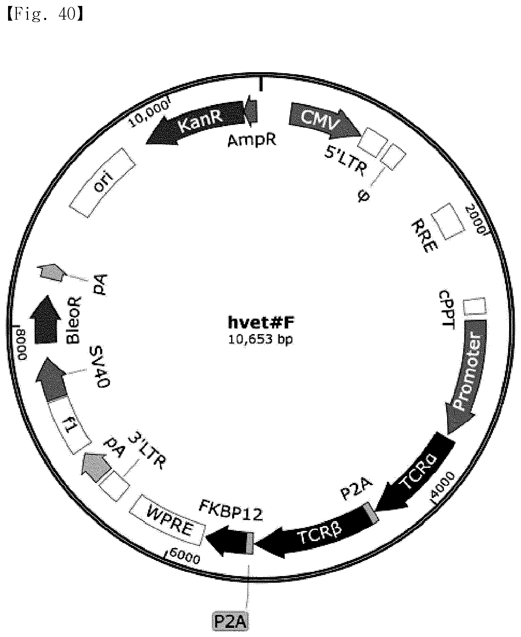

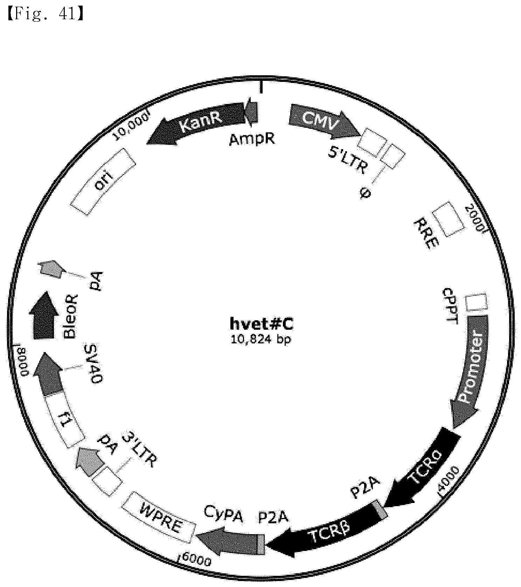

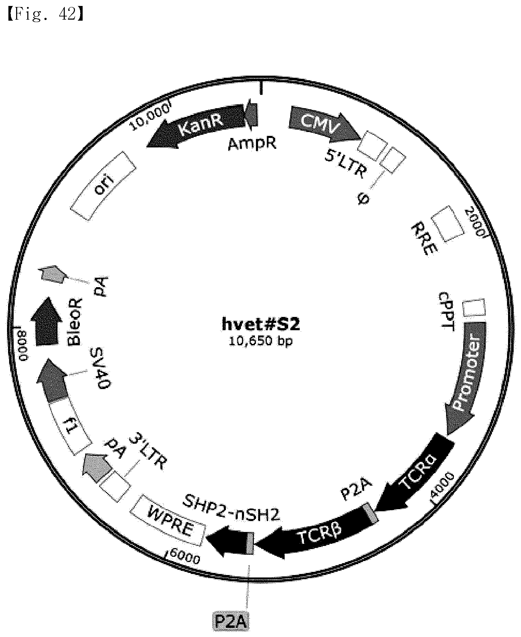

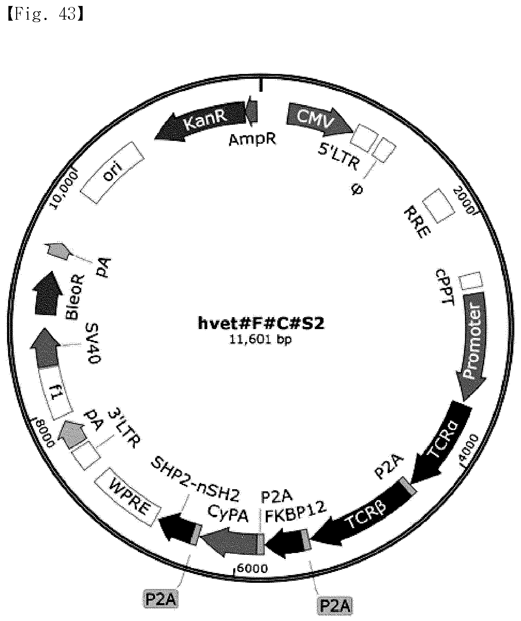

The HERV-E-specific TCR-T targeting a cancer cell expressing the HLA-A11-specific HERV-E epitope was prepared in the same manner as in Examples 1 to 4, except that the CAR region is replaced by HERV-E-specific TCR. Various signaling pathway modulators expressed together with HERV-E-specific TCR were also prepared. Specifically, the HERV-E-specific TCR-T expressing the FKBP12 protein, cyclophilin A and SHP2-nSH2 were prepared, respectively. In addition, TCR-T in which all of the FKBP12 protein, cyclophilin A, and SHP2-nSH2 were expressed was prepared. The sequences used in this case are shown in Tables 15 to 24 below. The vectors used in this case are as shown in Figures 39 to 43.

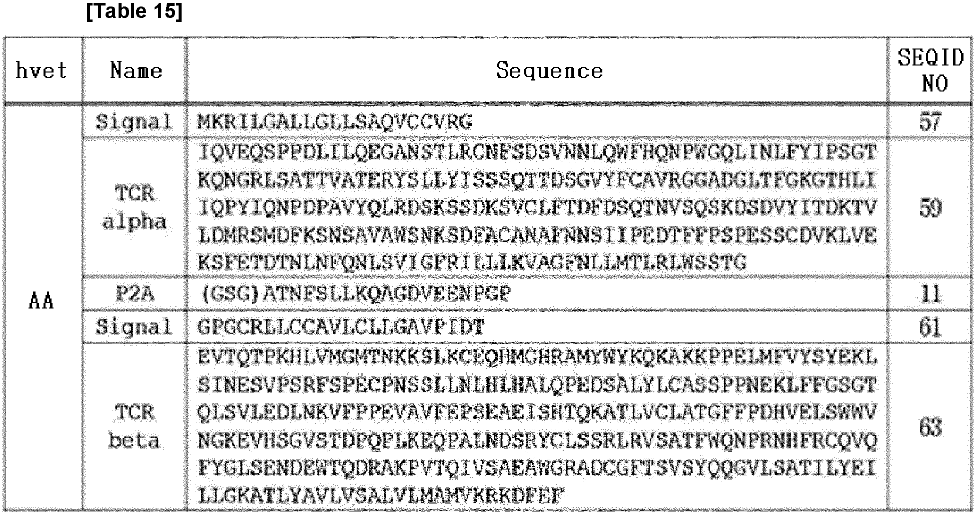

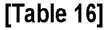

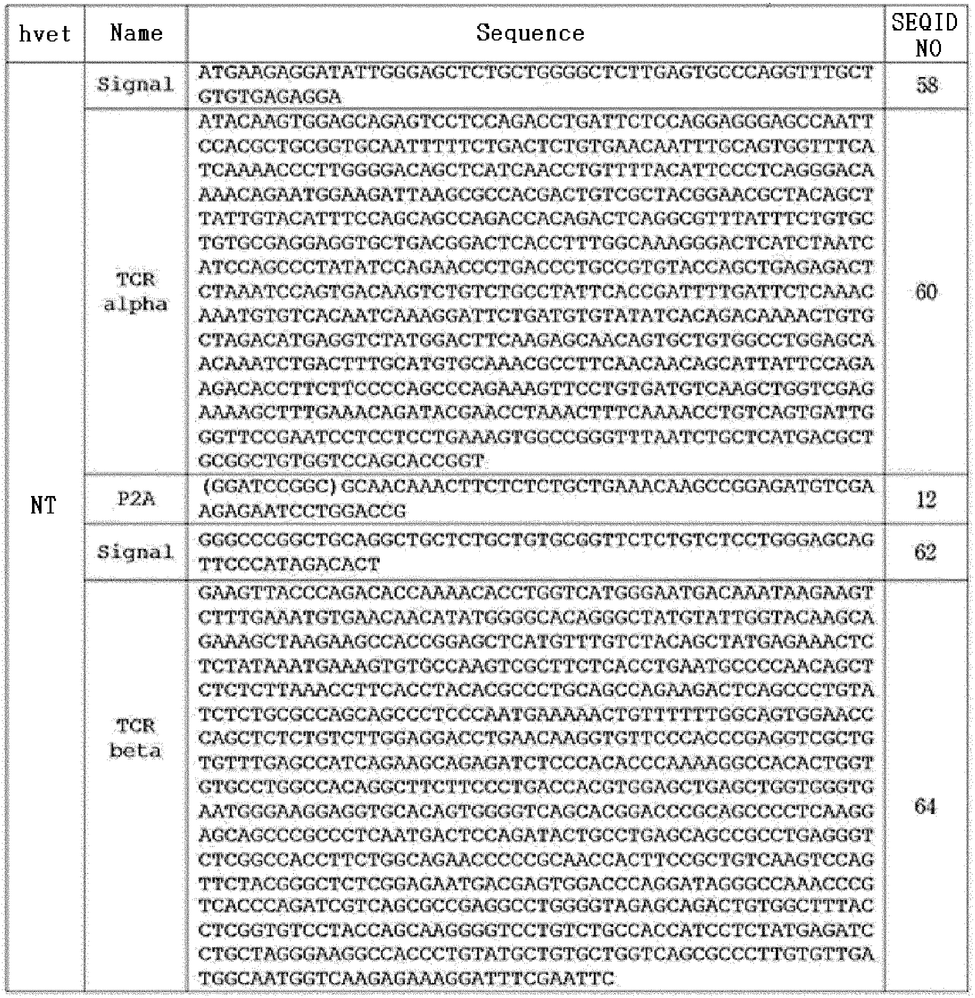

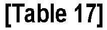

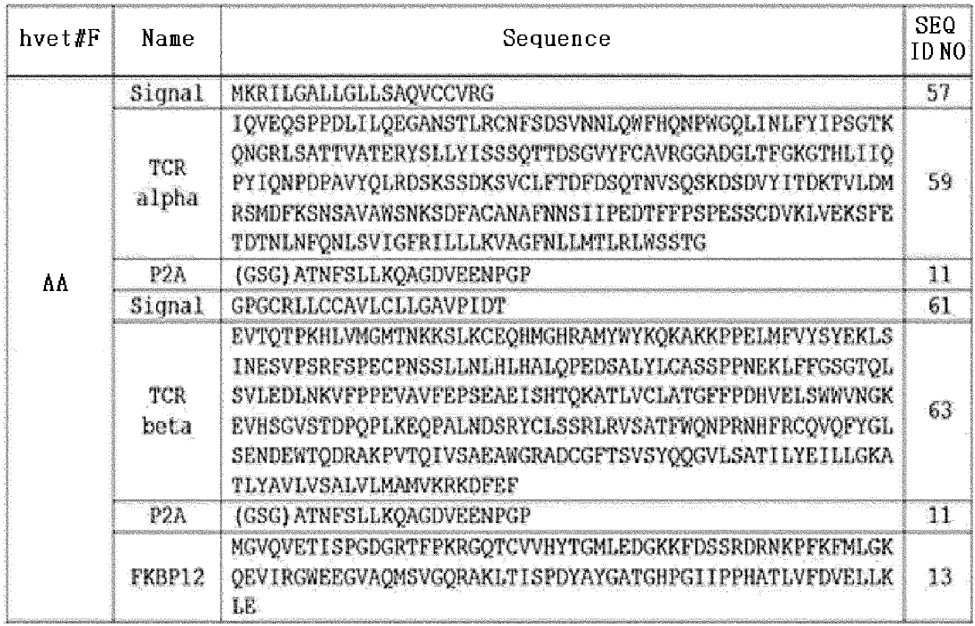

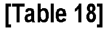

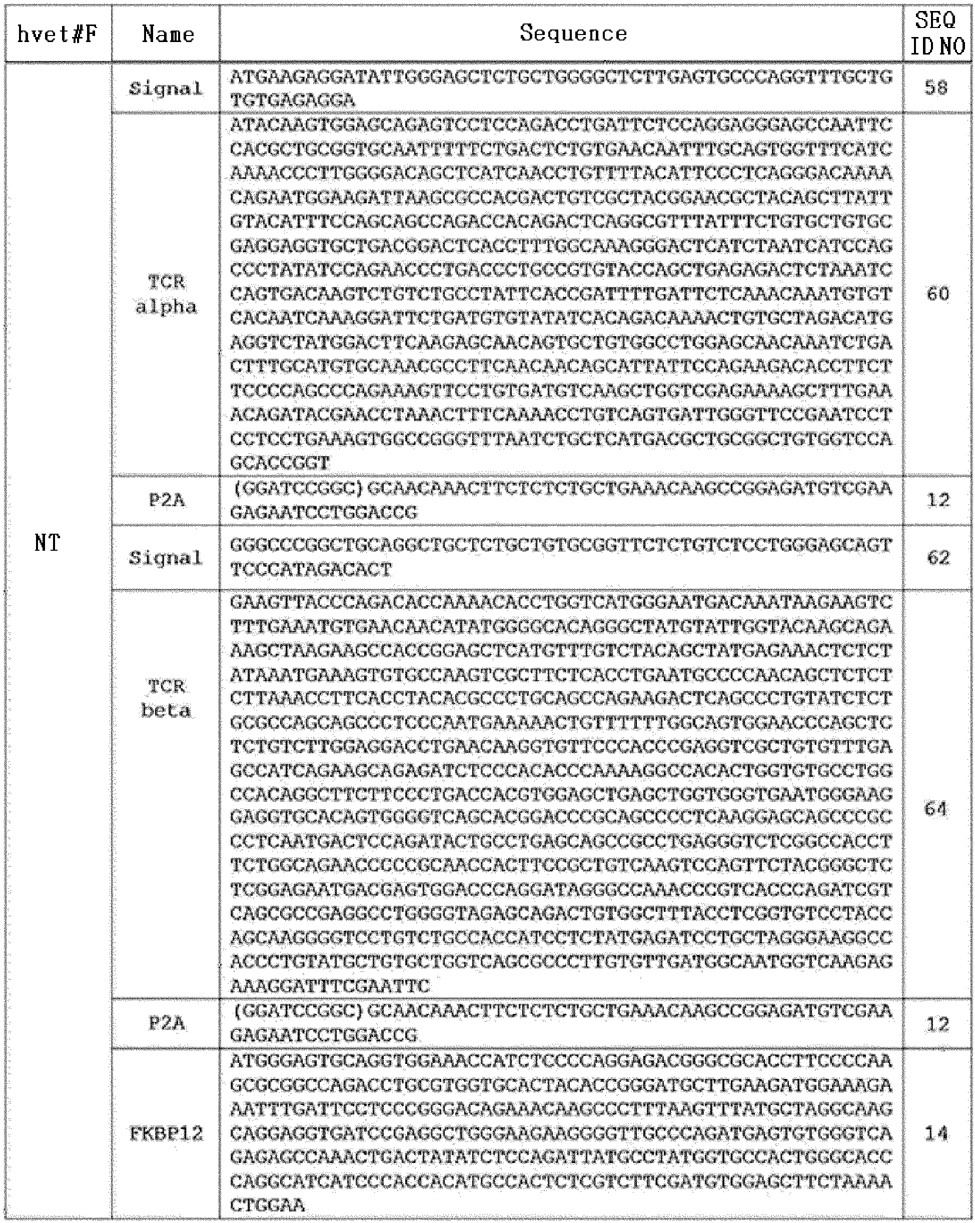

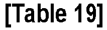

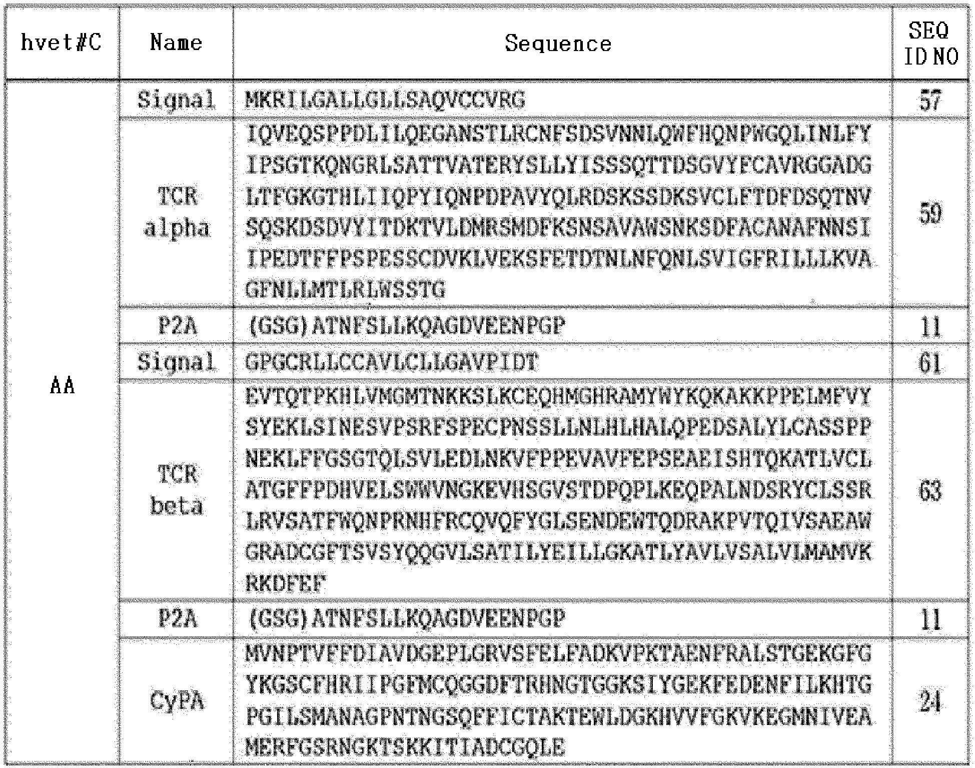

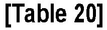

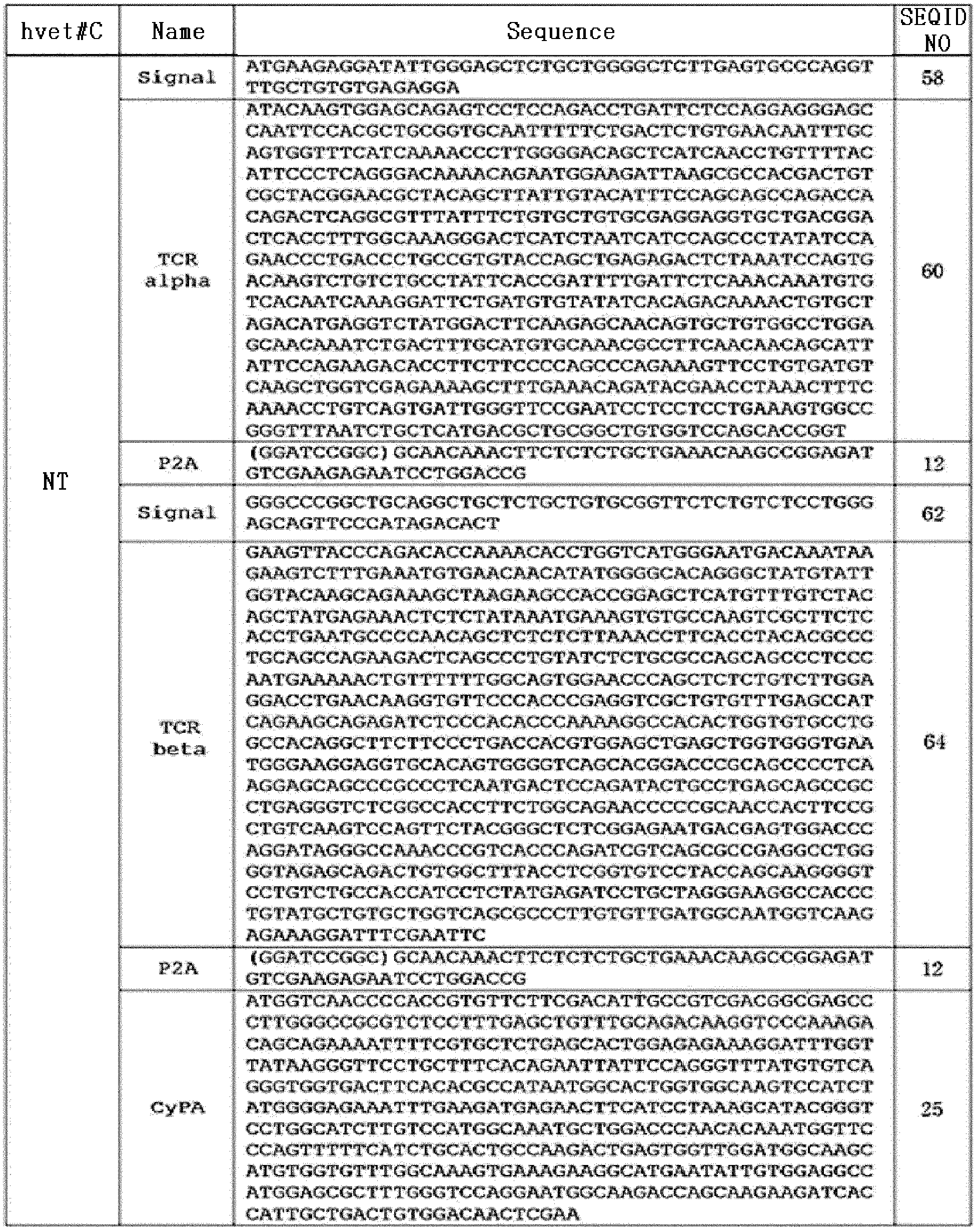

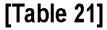

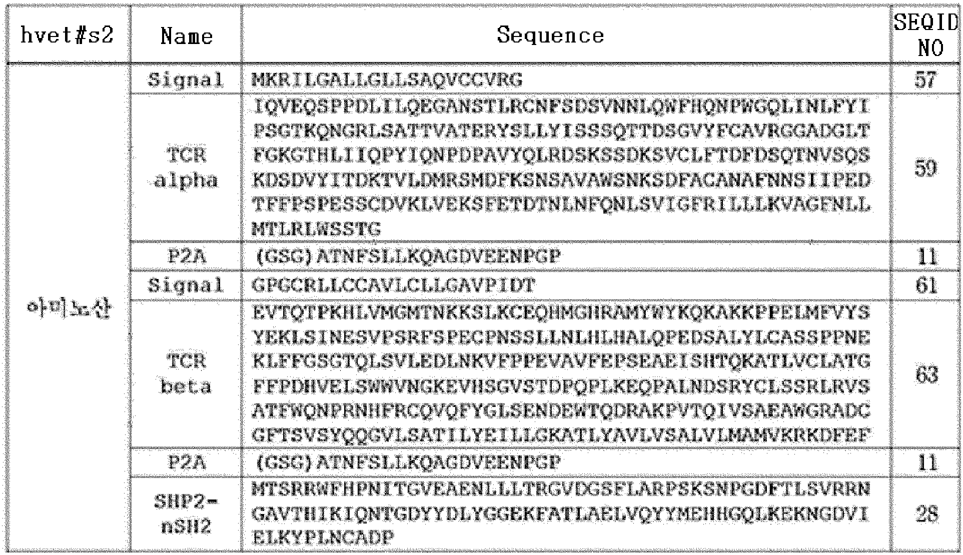

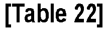

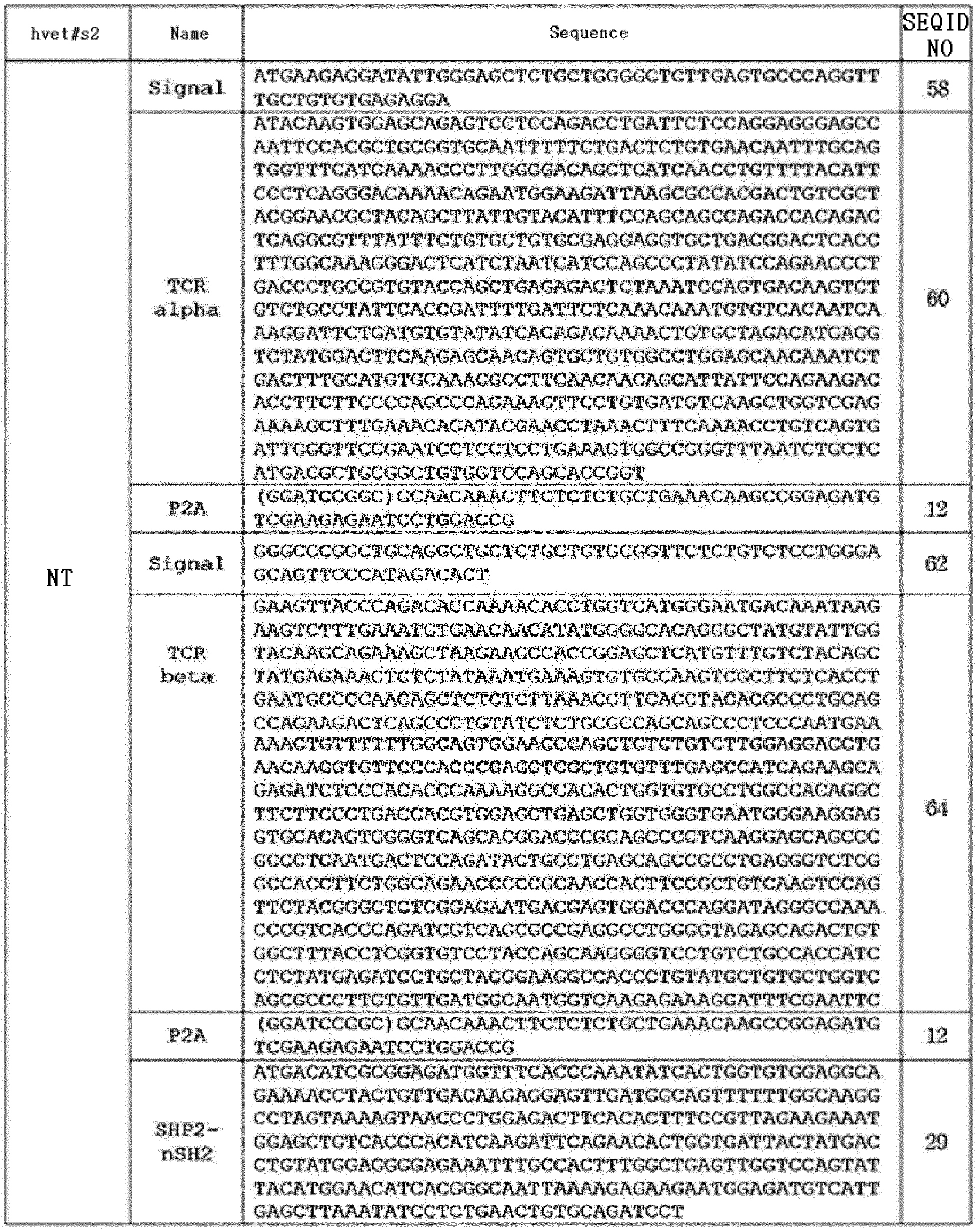

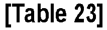

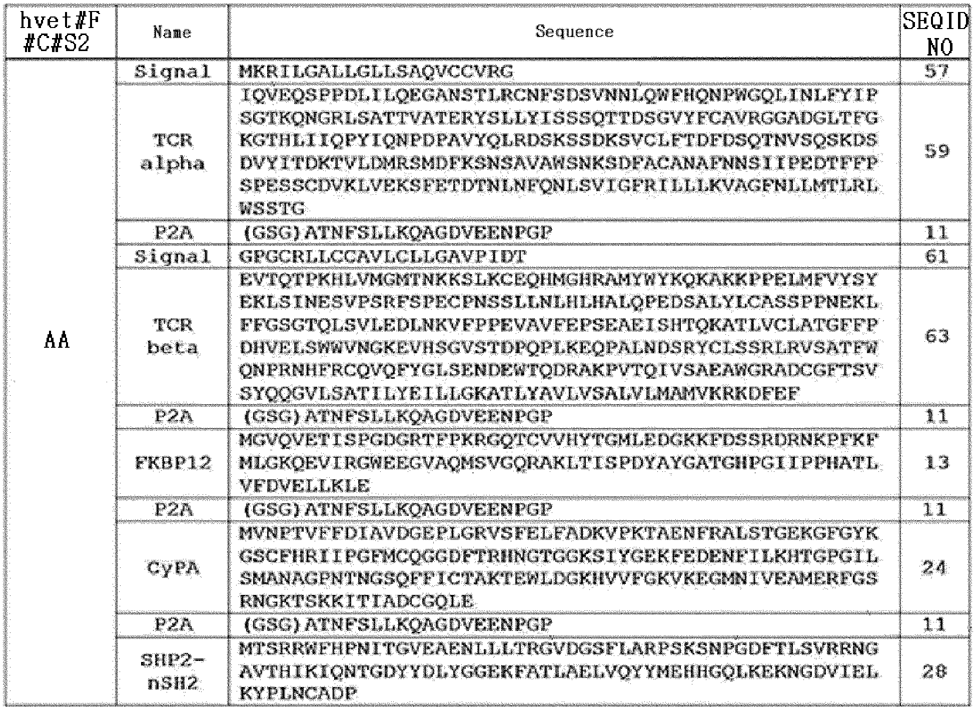

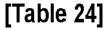

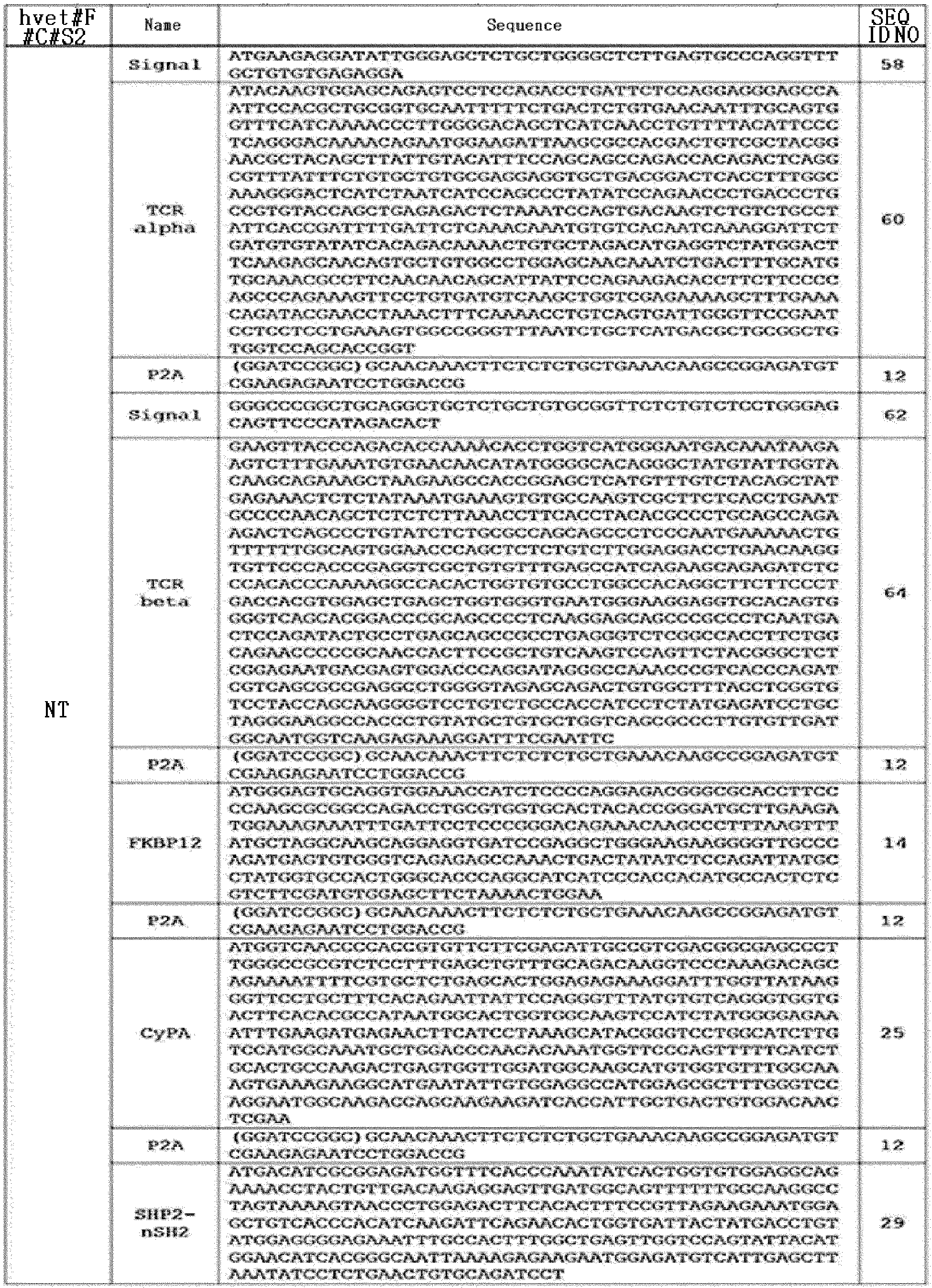

### Example 10. Preparation of NY-ESO-1-specific TCR-T

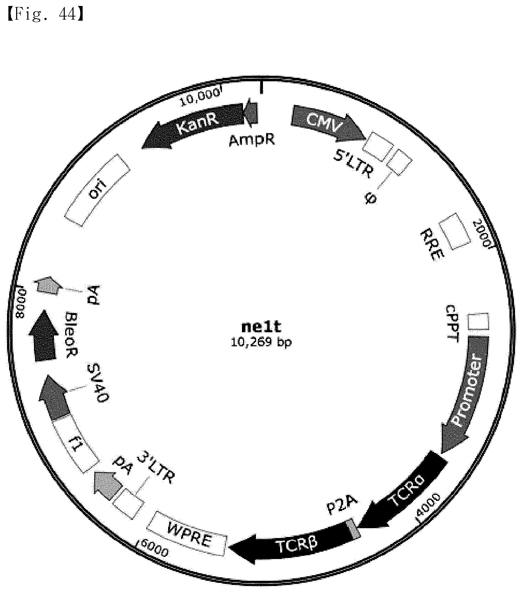

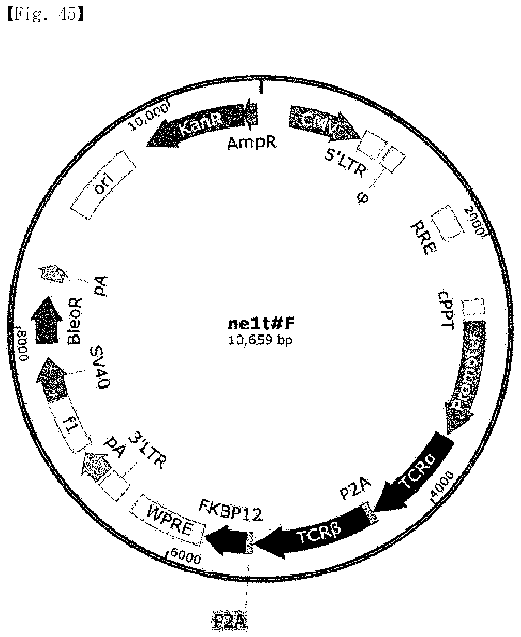

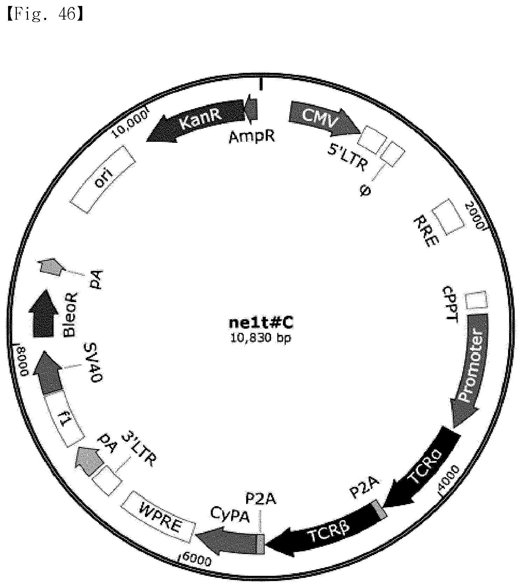

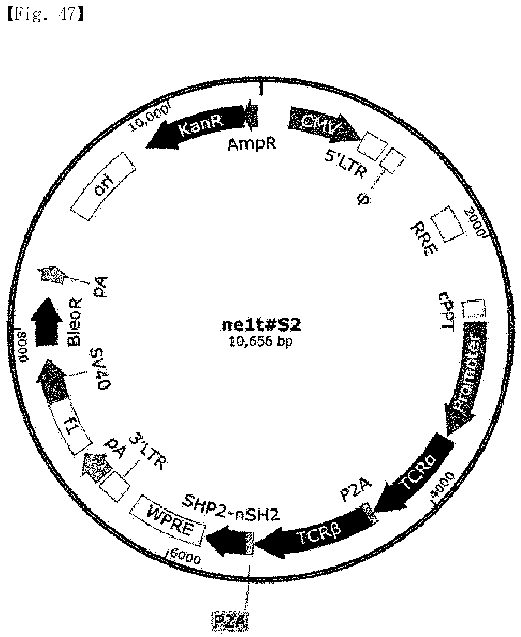

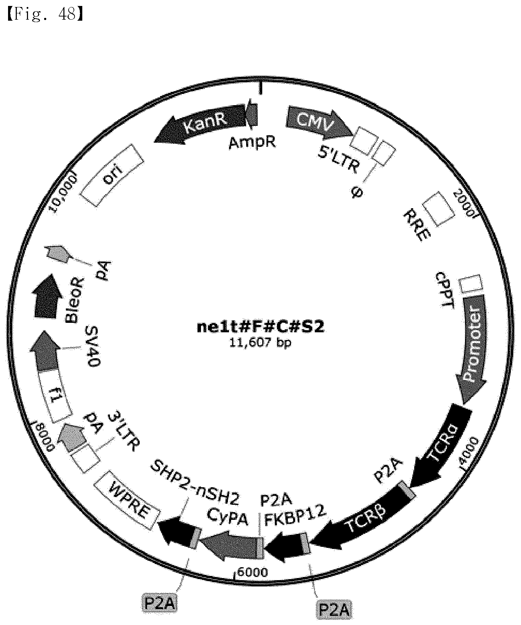

The NY-ESO-1-specific TCR-T targeting a cancer cell expressing the HLA-A2-specific NY-ESO-1 epitope was prepared in the same manner as in Examples 1 to 4, except that the CAR region is replaced by NY-ESO-1-specific TCR. Various signaling pathway modulators expressed together with NY-ESO-1-specific TCR were also prepared. Specifically, the NY-ESO-1-specific TCR-T expressing the FKBP12 protein, cyclophilin A and SHP2-nSH2 were prepared, respectively. In addition, TCR-T in which all of the FKBP12 protein, cyclophilin A, and SHP2-nSH2 were expressed was prepared. The sequences used in this case are shown in Tables 25 to 34 below. The vectors used in this case are as shown in Figures 44 to 48.

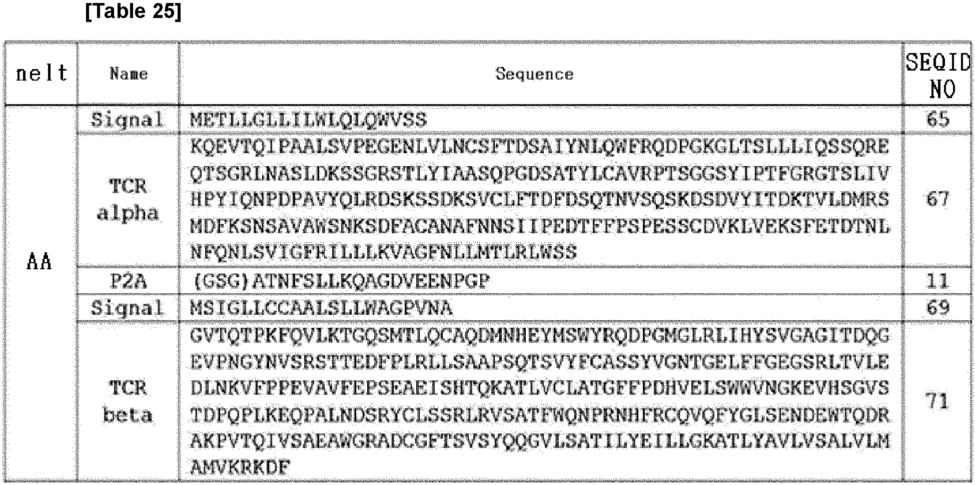

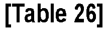

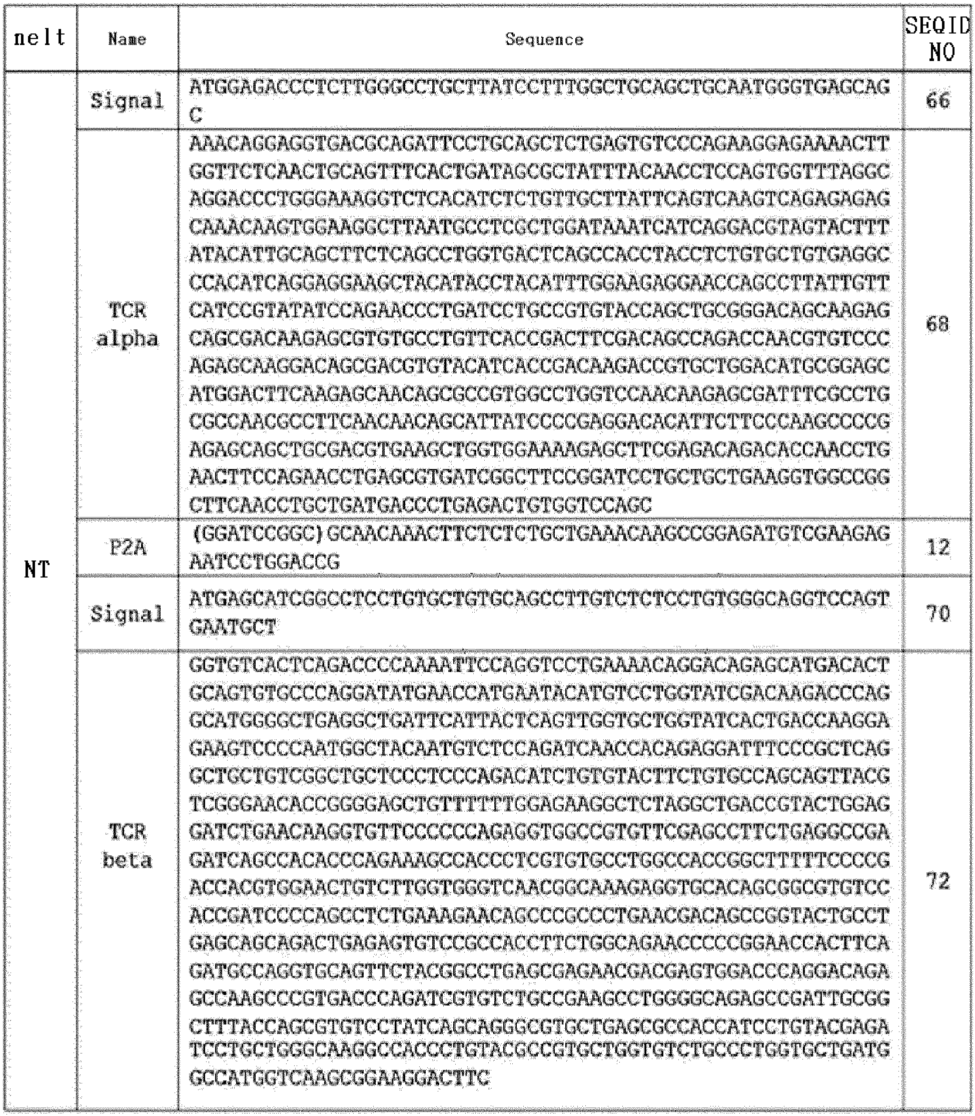

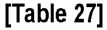

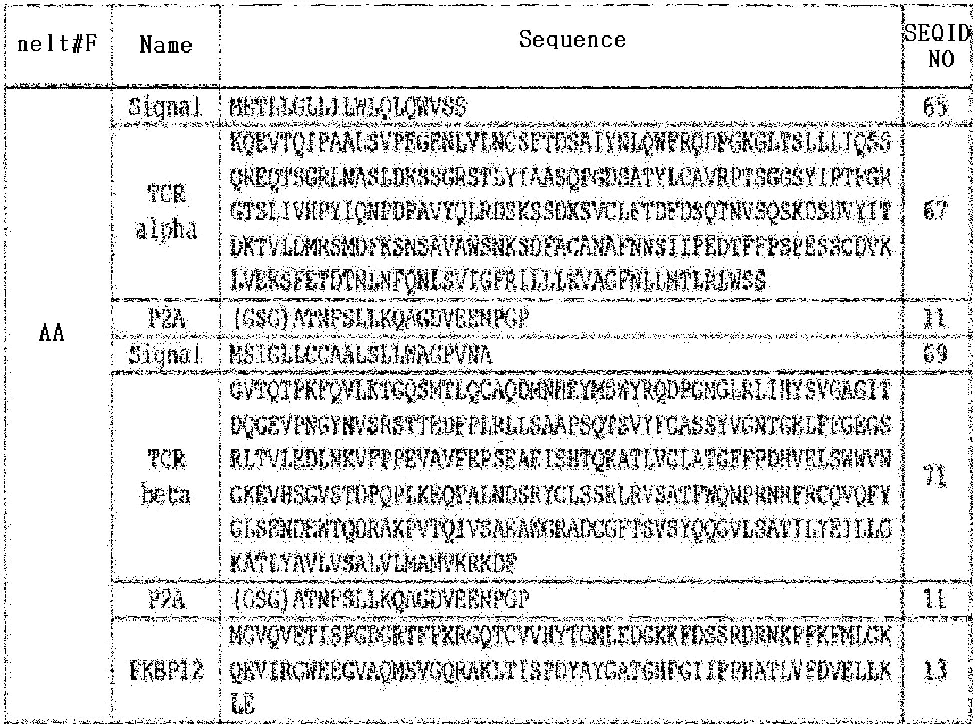

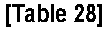

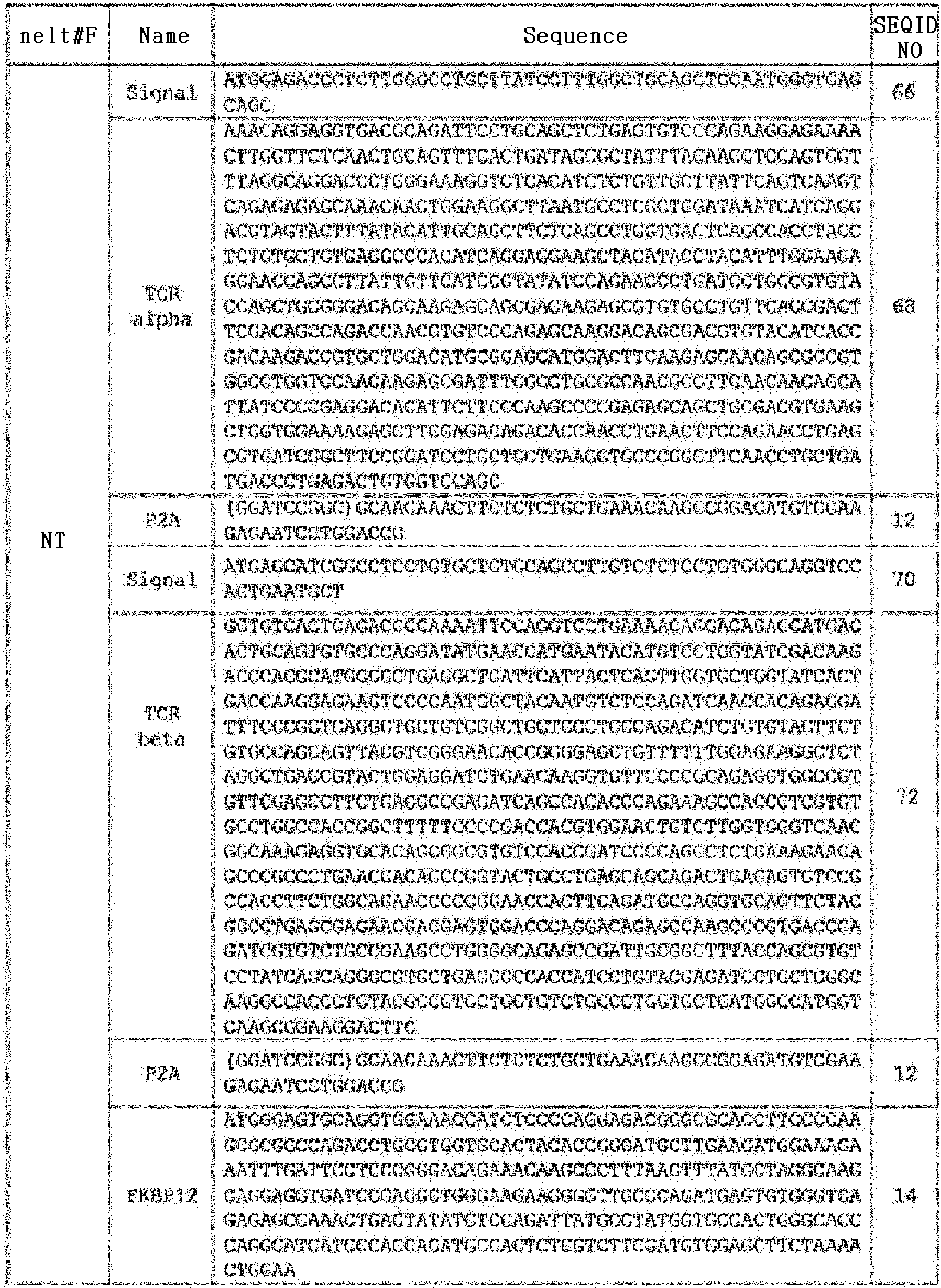

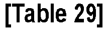

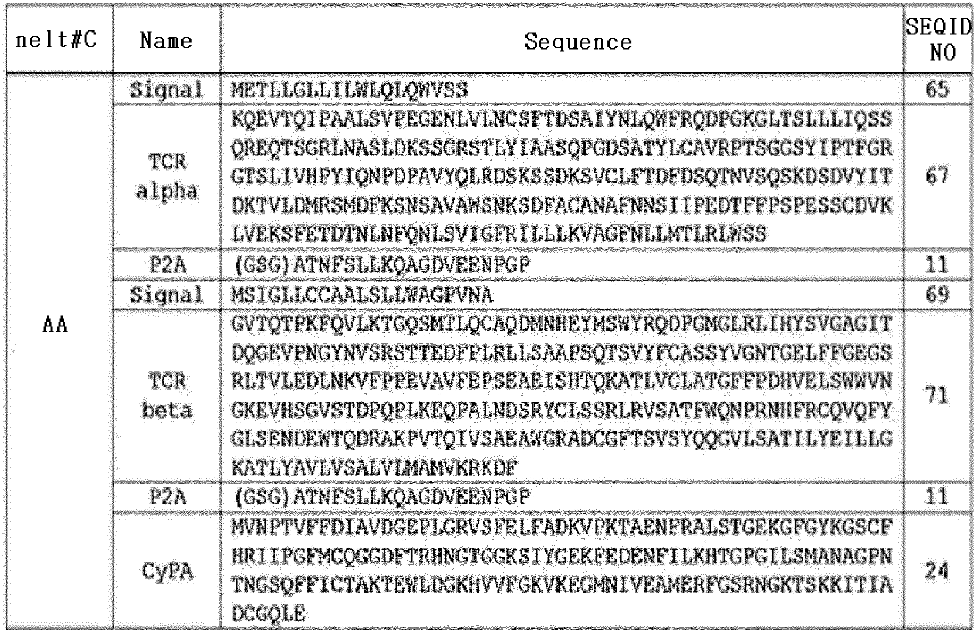

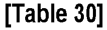

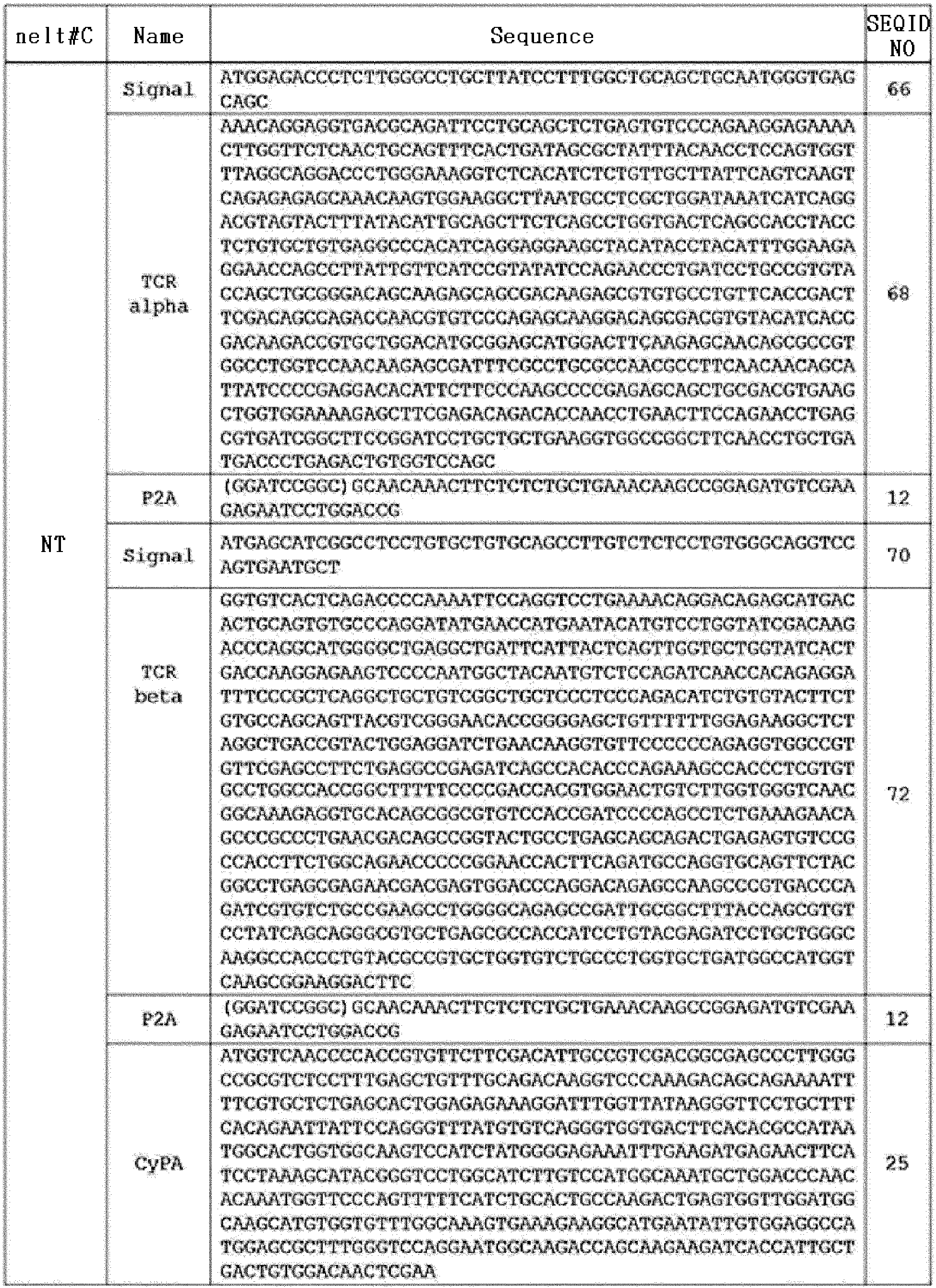

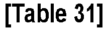

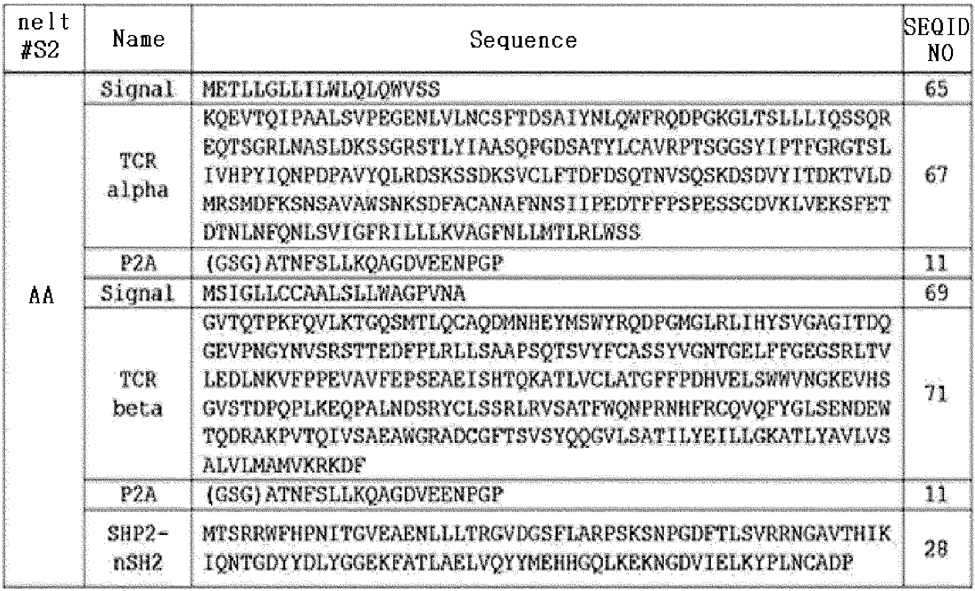

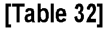

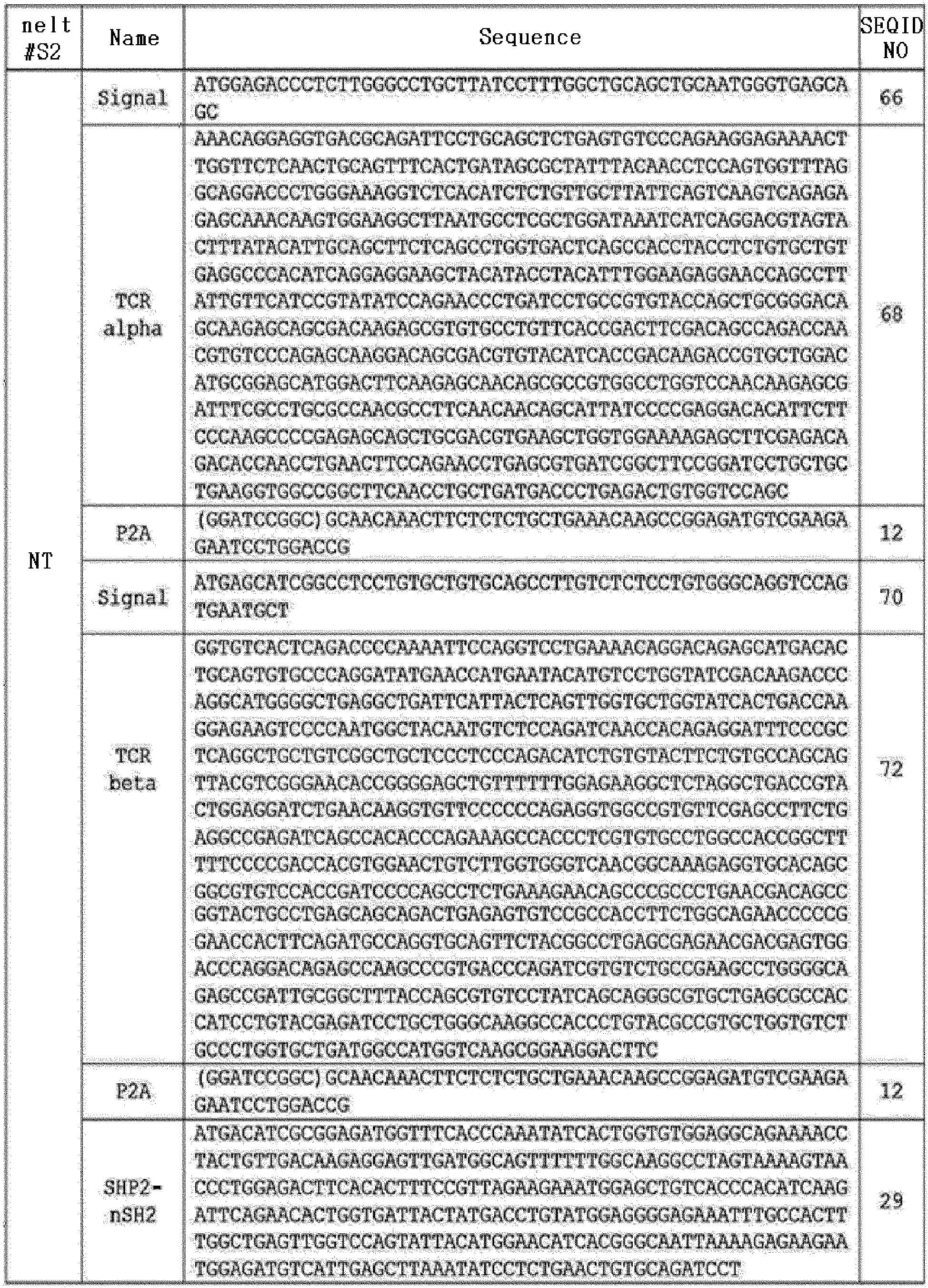

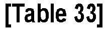

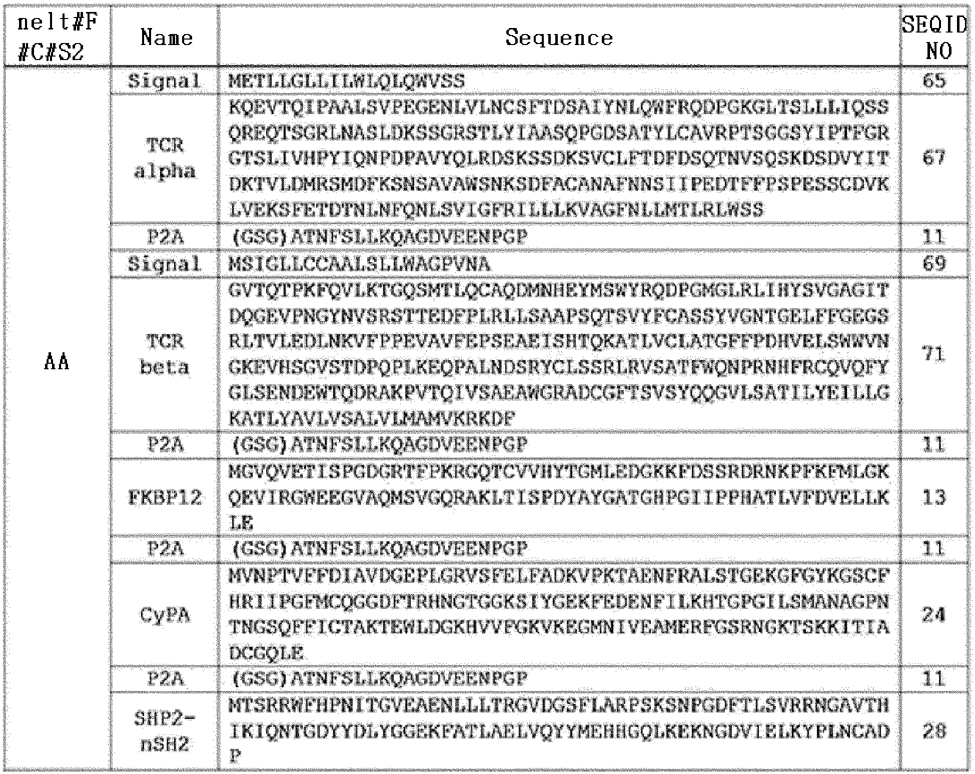

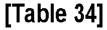

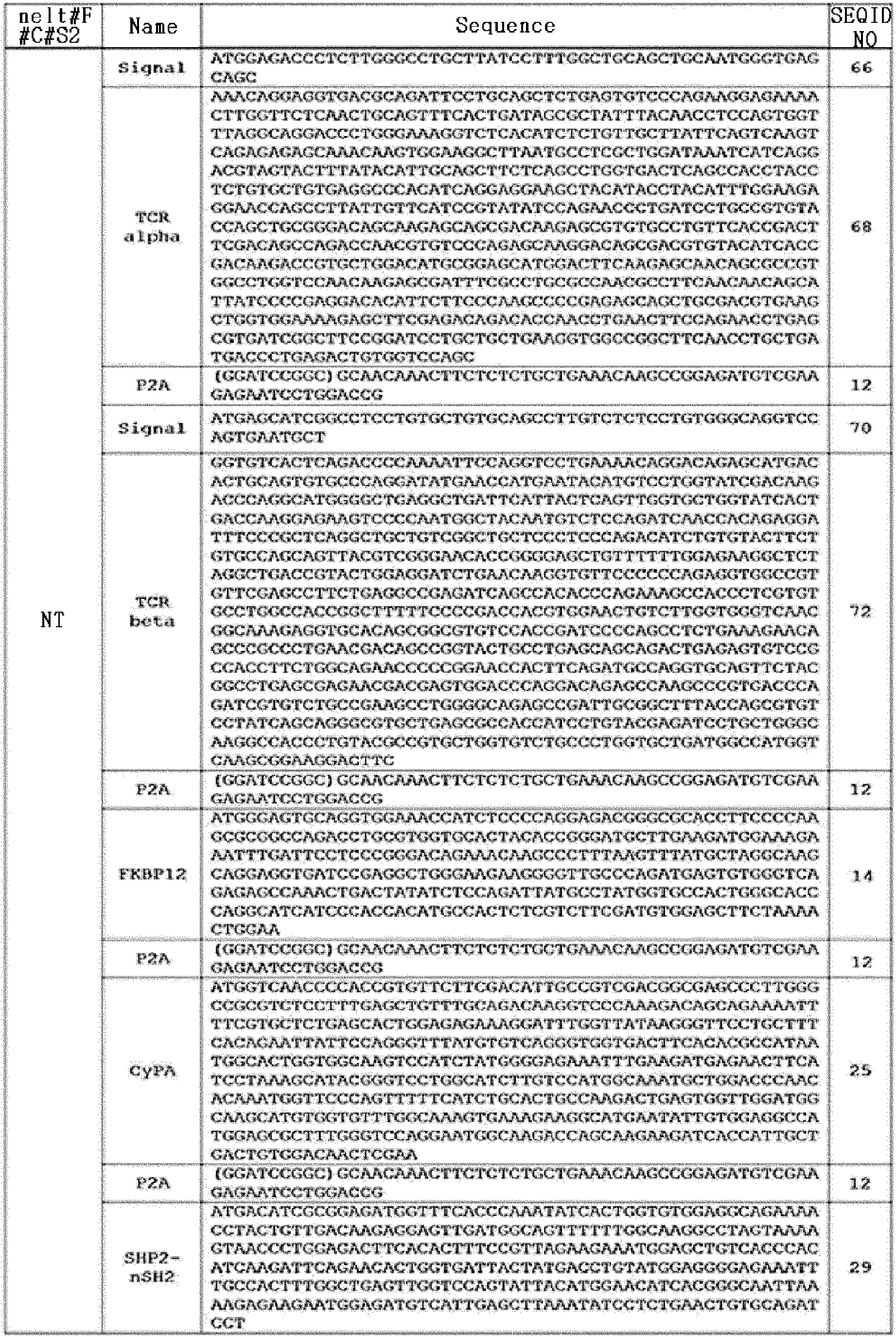

### II. Confirmation of activity of CAR-T into which a signaling pathway modulator is introduced: In vitro

### Experimental Example 1. Confirmation of antigen receptor expression in CAR-T cells

### Experimental Example 1.1. Evaluation of CAR expression through flow cytometry

In order to confirm the expression of CAR on the cell surface expressed in CAR-T cells and antigen expression of the target tumor cells, CAR-T cells were collected 4 days after viral infection and stained with FITC-labeled recombinant human CD19 (Acro, #CD9-HF2H2) to analyze the expression of CD19-specific CAR (Figure 54). In addition, the expression of Her2-specific CAR was also confirmed by staining with Alexa-Fluor 488 labeled AffiniPure F(ab')₂ fragment goat anti-human IgG (H+L) antibody (Jackson ImmunoResearch, #109-546-003) (Figure 69).

Moreover, the expression of the PSMA-specific CAR was confirmed by staining with Biotin labeled AffiniPure F(ab')₂ fragment goat anti-mouse IgG antibody (F(ab')₂ fragment specific antibody (Jackson ImmunoResearch, #115-066-006) and PE labeled streptavidin (BD Pharmingen, #554061) (Figure 77).

In the case of target tumor cells, the expression of CD19 was confirmed by staining with APC labeled anti-human CD19 antibody (BD Pharmingen, #555415) (Figure 58). The expression level of these proteins in the surface of stained cells was measured using CytoFLEX S (Beckman Coulter) flow cytometer (FACS), and analyzed using CytExpert software.

### Experimental Example 1.2. Evaluation of CAR expression using immunofluorescence staining

Eight days after the start of stimulation, the CD19-specific CAR-T cells constructed in Example 4 were stained with Cyanine 3-labeled goat-anti-mouse IgG antibody (Invitrogen, #A10521) (Figure 55), and the Her-specific CAR-T cells constructed in Example 5 were stained with Alexa-Fluor 488-labeled AffiniPure F(ab')₂ fragment goat anti-human IgG (H+L) antibody (Jackson ImmunoResearch, #109-546-003) (Figure 70). In addition, the PSMA-specific CAR-T cells constructed in Example 6 were stained with Cyanine 3-labeled goat-anti-mouse IgG antibody (Invitrogen, #A10521) (Figure 78). The CAR-stained cells were fixed for 20 minutes in eBioscience solution (eBioscience, #00-5123) and permeabilized. Thereafter, the sample was mounted, and the nuclei were stained with DAPI-containing ProLong Gold Antifade Mountant solution (Invitrogen, #P36931).

In another example, 9 days after the start of stimulation, CD19-specific CAR-T cells were co-cultured with Daudi-Fluc-eGFP cells at a 1:1 ratio for 1 hour, and then were stained using Cyanine 3-labeled goat-anti-mouse IgG antibody and DAPI. Cell images were photographed at a 400X-fold ratio using an Optinity KI-2000 microscope equipped with Optinity HDMI 4K C-Mount Camera KCX-80 (Figure 56). Daudi-Fluc-EGFP cells were observed with green fluorescence of EGFP in the cytoplasm.

### Experimental Example 2. Analysis of T cell subsets

Eight days after the start of stimulation, the CAR-T cells constructed in Example 8 were analyzed for T cell subsets using a flow cytometer: they were classified into stem cell-like memory T cells (T memory stem cells, CD45RA⁺CCR7⁺), central memory T cells (CD45RA⁻)CCR7⁺), effector memory T cells (CD45RA⁻CCR7⁻) and effector T cells (CD45RA⁺CCR7⁻) according to the presence or absence of the expression of CD45RA and CCR7. When analyzing CD19-specific CAR(+) cells, FITC-labeled recombinant human CD19 protein (Acro, #CD9-HF2H2) was used to stain the CAR. In addition, when analyzing PSMA-specific CAR(+) cells, FITC-labeled recombinant human PSMA protein (Acro, #PSA-HF244) was used to stain the CAR. In both cases, common T cell subsets were analyzed after staining using specific antibodies as indicated in Table 35 below (Figure 57).

**[Table 35]**

| Target protein | Manufacturer, # Cat No | Fluorescent dye | Species |
|---|---|---|---|
| CD45RA | BD Pharmingen, # 554061 | Alexa Fluor 700 | mouse |
| CCR7 | BD Pharmingen, # 566743 | BV421 | mouse |

### Experimental Example 3. Confirmation of expression of signaling pathway modulators

### Experimental Example 3.1. Evaluation of protein expression through Western blotting

In order to confirm the protein expression of the CAR-T cells constructed in the above example, the CAR-T cells were lyzed with a PROPREP protein extracting solution (iNtRON Biotechnology, #17081) containing a protease inhibitor. The protein concentration was quantified using the BCA Protein Assay kit (Pierce).

After electrophoresis of the proteins in the same amount using SDS-PAGE, they were transferred to the PVDF membrane through the XCell SureLock Mini-Cell Electrophoresis System and XCell II Blot Module (Invitrogen). Thereafter, the membrane was blocked with a blocking solution (5% powdered skim milk solution), and the protein expression was confirmed by sequentially incubating with the primary antibody and the secondary antibody. Goat-anti-mouse IgG HRP (Invitrogen, #62-6520) or goat-anti-rabbit IgG HRP (Abcam, #ab6721) was used as the secondary antibody.

Images for protein expression were photographed using Azure C400 Gel Imaging System, and analyzed with AzureSpot Analysis software (Azure Biosystems) (Figure 59). The primary antibodies used in the experiment are as shown in Table 36 below.

**[Table 36]**

| Primary antibody (target protein) | Manufacturer # Cat No | Species |
|---|---|---|
| human FKBP12 | Santa Cruz #sc-133067 | mouse |
| human Cyclophilin A | Sino Biological # 106317-T36 | rabbit |
| N-terminal SH2 of human SHP2 domain | MyBioSource #MBS1751917 | mouse |
| human GAPDH | Santa Cruz #sc-32233 | mouse |

### Experimental Example 3.2. Evaluation of mRNA expression through real-time polymerase chain reaction

In order to confirm the expression of mRNA in the CAR-T cells constructed in the above Examples, RNA was isolated, 8 days after stimulation, from the CAR-T cells using the ISOLATEII RNA Mini kit (Bioline, #52072), and quantified with using a Nanodrop 1000 spectrophotometer (Thermo Scientific). After cDNA was synthesized using 0.5 µg or 1 µg of RNA, real-time quantitative PCR (Q-PCR) was performed using SensiFAST Probe Hi-ROX One-Step kit (Bioline, #77001).

The GAPDH gene was used as an internal reference. All experiments were repeated twice. The gene expression was analyzed by the ΔΔCt method (Figure 60). The primers and TaqMan probes used in Q-PCR are shown in Table 37 below.

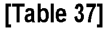

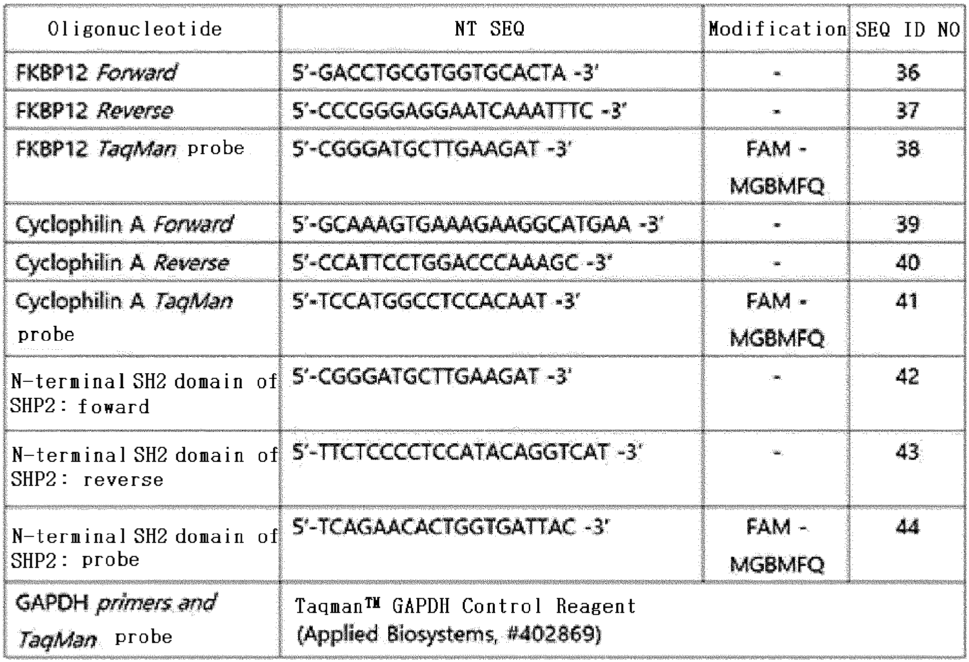

### Experimental Example 4. Confirmation of the activity of CAR-T cells

### Experimental Example 4.1. Target tumor cell line and culture thereof

For *in vitro* functional analysis of CD19-specific CAR-T cells, the target tumor cell line (K562-CD19) was established by stably expressing human CD19 protein in human myeloid leukemia K562 cells (ATCC, #CCL-243) using a lentiviral vector. The K562 parental cell line was used as a negative control.

Moreover, human B lymphoblastic Daudi cells stably expressing firefly luciferase and Emerald green fluorescent protein (GFP, Imanis, #CL158) were also used for *in vitro* functional analysis of CD19-specific CAR-T cells. In addition, human B lymphoblastic Daudi cells stably expressing firefly luciferase were used for *in vivo* anticancer efficacy analysis of CD19-specific CAR-T cells. In addition, human B cell precursor leukemia cell line Nalm6 (ATCC, #CCL-3273) was used for *in vitro* function analysis of CD19-specific CAR-T cells.

The human breast cancer cell line SKBR3 stably expressing firefly luciferase (JCRB, #1627.1) was used for *in vitro* function analysis and *in vivo* anticancer efficacy analysis of Her2-specific CAR-T cells. The human prostate cancer cell line LNCaP (ATCC, #CRL-1740-LUC2) stably expressing firefly luciferase was used for *in vitro* function analysis of PSMA-specific CAT-T cells.

Each cell line of K562, K562-CD19, Nalm6 and Daudi-Fluc-eGFP target tumor cell lines was cultured in a RPMI-1640 medium (Gibco, #11875-085) containing 10% FBS (fetal bovine serum) and antibiotic-antibacterial agent (Gibco, #15240096). SKBR3-Luc cells were cultured in a McCoy's 5a medium (Gibco, #16600-082) containing 10% FBS and antibiotic-antibacterial agent. LnCap-Luc cells were cultured in a RPMI-1640 medium containing 10% FBS, antibiotic-antibacterial agent and 2 µg/mL Blasticidin (Gibco, #A1113903).

### Experimental Example 4.2. Evaluation of the ability of CAR-T cells to secrete cytokines

The CAR-T cells (4×10⁴) constructed in the above Examples were co-cultured with target tumor cells at a 1:1 ratio for 24 hours. Thereafter, a culture medium was collected, and the concentration of various cytokines such as IFNγ, TNFα and IL-2 in the medium was measured using an ELISA (R&D Systems) method. The results are shown as an average value after two repeated experiments (Figures 61 to 63 / Figures 71 to 73).

In addition, when evaluating the release capability of cytokines in the presence of TGF-β1, a medium containing TGF-β1 at the specified concentration (0 ng/mL to 5 ng/mL) was used, and CAR-T cells (1×10⁵) and target tumor cells were co-cultured at a 1:1 ratio for 24 hours. The results are shown as an average value after two repeated experiments (Figures 64 and 65).

In order to correct the difference in the transduction efficiency of CAR-T cells, the number of transduced CAR-T cells and the total number of T cells were adjusted using non-transduced T cells.

### Experimental Example 5. Confirmation of cell killing activity of CAR-T cells

### Experimental Example 5.1. Measurement of cell killing activity of CAR-T cells based on EuTDA

The killing activity against target tumor cells of the CD19-specific CAR-T cells constructed in the above Example was evaluated by measuring EuTDA released for 4 hours with a DELFIA cytotoxicity measurement kit (PerkinElmer, #AD0116). Specifically, the target tumor cells were reacted with a DELFIA BATDA reagent for 15 minutes at 37°C, and then washed three times with a RPMI 1640 medium. Thereafter, the target tumor cells were resuspended in the culture medium so as to be 5×10⁴ cells/mL. The CAR-T cells were co-cultured with the target tumor cells (5×10³) in a 96 well V-bottom plate at a predetermined ratio. Thereafter, the culture medium (20 µL) was transferred to a white bottom plate, mixed with a Europium solution (200 µL), and reacted with gentle agitation for 15 minutes.

TDA released into the medium was evaluated by measuring fluorescence using a SpectraMax iD5 MultiMode microplate reader (Molecular Device). Each experiment was carried out in triplicate, and solubility was calculated through the following equation using fluorescence measurements: $% solubility = \left\{\left(release value of experimental group-background release value\right)/\left(\begin{matrix}maximum \\ release value - background release value\end{matrix}\right)\right\} \times 100 ;$ where, the basic release value is a value measured in spontaneous release of target tumor cells, and the maximum release value is a value measured after completely lyzing the target tumor cells with a DELFIA lysis buffer.

### Experimental Example 5.2. Measurement of cell killing activity of CAR-T cells based on luciferase

The CAR-T cells constructed in the above Example were co-cultured with the target tumor cells (3×10⁴) expressing firefly luciferase in a 96 well U-bottom plate at a predetermined ratio. Each experiment was repeated twice. Specifically, 75 µg/mL D-Luciferin potassium salt (PerkinElmer, #122799) was added, and cultured at 37°C for an optimized time for each target tumor cell. Thereafter, luciferase activity of living cells was measured using a SpectraMax iD5 MultiMode microplate reader (Molecular Device). The capability of CAR(+)-T cells to lyze target tumor cells was evaluated by comparing the difference in the induced luciferase activity value between CAR-T cells and non-transduced T cells at each E:T (effector (CAR-T cell):target (target tumor cell)) ratio. The results are shown as an average value for the two repeated experiments (Figure 68 (top), Figure 75). In each experiment, in order to correct the difference in transuction efficiency, the number of transduced CAR-T cells and the total number of T cells were adjusted using non-transduced T cells.

### Experimental Example 6. Measurement of cell migration

The migration ability of the CAR-T cells constructed in the above Example was measured using a transwell (24 well, Corning, #3422) equipped with a membrane insert of 8 µm pore. Beads were removed from the CAR-T cells that were previously reacted with anti-CD3/anti-CD28 Dynabeads, and the bead-free CAR-T cells were resuspended in a serum-free RPMI1640 medium (containing 0.25% human serum albumin (GreenCross)) containing 0.2 µg/mL recombinant human EGF (Sino Biological, #10605-HNAE). The CAR-T cells were loaded onto the upper chamber of the transwell, whereas the lower chamber was filled with a RPMI1640 medium containing 0.25% human serum albumin (GreenCross). After incubating for 1 hour, the number of cells migrated to the lower chamber was measured using flow cytometry and trypan blue staining method (Figure 66, Figure 74).

In addition, in order to compare and evaluate the intrinsic motility of CAR-T cells after the antigen stimulation, CAR-T cells and target tumor cells were cultured at a 1:1 ratio at 37°C for 48 hours (using a 96 well U-bottom plate), and then cell images were photographed. In this case, the experimental group cultured with CAR-T alone was used as a negative control (Figure 67).

In order to correct the difference in transduction efficiency, the experiment was performed after adjusting the number of transduced CAR-T cells (CAR(+)-T cells) and the total number of T cells using non-transduced T cells. Cytokines were not treated in Medium 1 and Medium 2, and the serum composition of Medium 1 and Medium 2 was different from each other.

### II. Confirmation of the activity of CAR-T into which a signaling pathway modulator is introduced: In vivo

### Experimental Example 7. Evaluation of the anticancer efficacy of CAR-T cells into which a signaling pathway modulator is introduced using a hematologic cancer cell model (I)

Through the following method, the anticancer efficacy of CAR-T cell_19bbz and CAR-T cell_19bbz#F was compared and evaluated by doses (1×10⁶, 3×10⁶, and 1×10⁷). A Daudi cancer cell line into which the firefly luciferase gene (Daudi-Fluc) was introduced was used as a hematologic cancer cell model.

In order to induce a hematologic cancer, 1×10⁶ Daudi-Fluc cells prepared in 100 µL were injected into the left and right tail veins of healthy NPG (NOD-Prkdc^{scid} IL2rg^{null}) (Vital Star, China) female mice (7 weeks old) that had undergone a certain period of acclimation. After transplantation of the Daudi-Fluc cancer cell line, a D-luciferin (PerkinElmer, USA) solution was intraperitoneally injected on the 10^{th} day, and photographed using an IVIS imaging equipment (PerkinElmer, USA) 10 minutes later. Subsequently, after measuring the level of Luciferase expression for each subject with IVIS Lumina Series Software, group separation was performed by calculating the average value. In order to evaluate the anticancer efficacy of each CAR-T cell, 100 µL of CAR-T cells in specified doses (1×10⁶, 3×10⁶, and 1×10⁷) was administered to the left and right veins of the tail of the mice using a 1 mL syringe (BD REF328820, 31G). After the injection of cancer cells, a D-Luciferin solution was intraperitoneally injected in the same manner as described above on Day 14, Day 21, Day 28, Day 35, and Day 42, respectively, and tumor growth was monitored through IVIS imaging (Figure 50). CAR-T cell_19bbz was set as a control.

As a result, as shown in Figure 50, it was confirmed that tumor inhibition was significantly increased only in the group treated with CAR-T_19bbz#F when CAR-T cells were administered at a highest dose.

### Experimental Example 8. Evaluation of the anticancer efficacy of CAR-T cells into which a signaling pathway modulator is introduced using a hematologic cancer cell model (II)

Through the following method, the anticancer efficacy of CAR-T cell_19bbz#F, CAR-T cell_19bbz#C, CAR-T cell_19bbz#M, CAR-T cell_19bbz#N, CAR-T cell_19bbz#S1, CAR-T cell_19bbz#S2, CAR-T cell_19bbz#TC, CAR-T cell_19bbz#RG and CAR-T cell_19bbzT was evaluated. A Daudi cancer cell line into which the firefly luciferase gene (Daudi-Fluc) was introduced was used as a hematologic cancer cell model.

In order to induce a hematologic cancer, 1×10⁶ Daudi-Fluc cells prepared in 100 µL were injected into the left and right tail veins of healthy NPG (NOD-Prkdc^{scid} IL2rg^{null}) (Vital Star, China) female mice (7 weeks old) that had undergone a certain period of acclimation. After transplantation of the Daudi-Fluc cancer cell line, a D-luciferin (PerkinElmer, USA) solution was intraperitoneally injected on the 10^{th} day, and photographed using an IVIS imaging equipment (PerkinElmer, USA) 10 minutes later. Subsequently, after measuring the level of Luciferase expression for each subject with IVIS Lumina Series Software, group separation was performed by calculating the average value.

In order to evaluate the anticancer efficacy of each CAR-T cell after group separation, 1×10⁷ CAR-T cells prepared in 100 µL was administered to the left and right veins of the tail of the mice using a 1 mL syringe (BD REF328820, 31G). After the injection of cancer cells, a D-Luciferin solution was intraperitoneally injected in the same manner as described above on Day 14, Day 17, Day 21, Day 28, and Day 35, respectively, and tumor growth was monitored through IVIS imaging (Figure 7). CAR-T cell_19bbz was set as a control. As a result, it was confirmed that the anticancer effect of CAR-T overexpressing either one of cellular signal factors was superior to that of the control.

### Experimental Example 9. Evaluation of the anticancer efficacy of CAR-T cells into which a signaling pathway modulator is introduced, using a solid cancer model

Through the following method, the anticancer efficacy of CAR-T cell_Hbbz, CAR-T cell_Hbbz#F and CAR-T cell_Hbbz#FCS2 was evaluated. The SKBR3 cancer cell line (SKBR3-Luc) into which the firefly luciferase gene was introduced was used as a solid cancer cell model expressing Her2.

2×10⁶ SKBR3-Luc cells prepared in 200 µL were injected into the abdominal cavity of healthy NSGA (NOD-Prkdc^{scid} IL2rg^{null}) (JA Bio, Korea) female mice (7 weeks old) that had undergone a certain period of acclimation. After transplantation of the SKBR3-Luc cancer cell line, a D-luciferin (PerkinElmer, USA) solution was intraperitoneally injected on the Day 14, and photographed using an IVIS imaging equipment (PerkinElmer, USA) 10 minutes later. Subsequently, after measuring the level of Luciferase expression for each subject with IVIS Lumina Series Software, group separation was performed by calculating the average value.

In order to evaluate the anticancer efficacy of each CAR-T cell after group separation, the CAR T cells (dose: 1×10⁶ CAR(+)-T cells) prepared in 100 µL was administered to the left and right veins of the tail of the mice using a 1 mL syringe (BD REF328820, 31G). After the injection of cancer cells, a D-Luciferin solution was intraperitoneally injected in the same manner as described above on Day 21 (1-week Post-CAR T injection), Day 28 (2 weeks Post-CAR T injection), Day 35 (3 weeks Post-CAR T injection), and tumor growth was monitored through IVIS imaging. Quantitative image data was obtained through Lumina Series Software. The PBS administration group was set as a control.

The results are shown in Figure 76. As shown in the figure, it was confirmed that the CAR-T cells prepared in one embodiment showed the excellent efficacy even for the treatment of solid cancers.

## Claims

1. A polynucleotide encoding a fusion protein that comprises the following (i) to (v):
(i) an antigen binding domain;
(ii) a transmembrane domain;
(iii) an intracellular signaling domain comprising at least one co-stimulatory domain;
(iv) a self-cleavage peptide; and
(v) a signaling pathway modulator(s).

2. The polynucleotide according to claim 1, wherein a spacer is further placed between (i) the antigen binding domain and (ii) the transmembrane domain.

3. The polynucleotide according to claim 1, wherein the signaling pathway modulator is any one selected from the group consisting of a) a protein located in the immunosuppressive signaling pathway, b) an immunophilin, c) a protein involved in the antigen loss-mediated relapse, d) a protein located in the T cell stimulation pathway, e) a protein involved in the inhibition of negative feedback, and f) a combination thereof.

4. The polynucleotide according to claim 1, wherein the signaling pathway modulator operates in the cytoplasm.

5. The polynucleotide according to claim 3, wherein the protein located in the immunosuppressive signaling pathway is a protein located in the TGF-β/SMAD (FKBP12-FK506/rapamycin) signaling pathway or a fragment thereof.

6. The polynucleotide according to claim 5, wherein the protein located in the TGF-β/SMAD (FKBP12-FK506/rapamycin) signaling pathway is any one selected from the group consisting of FKBP12, a MH2 domain at a C-terminal of a SMAD4 protein, and N-SKI.

7. The polynucleotide according to claim 3, wherein the protein located in the immunosuppressive signaling pathway is a protein located in the inhibitory immune checkpoint pathway, or a fragment thereof.

8. The polynucleotide according to claim 7, wherein the protein located in the inhibitory immune checkpoint pathway is an N-SH2 domain of a SHP-1 protein or a fragment thereof, or an N-SH2 domain of a SHP-2 protein, or a fragment thereof.

9. The polynucleotide according to claim 3, wherein the immunophilin is FKBP12 or a fragment thereof, or cyclophilin A or a fragment thereof.

10. The polynucleotide according to claim 3, wherein the protein involved in the antigen loss-mediated relapse is a protein involved in trogocytosis, or a fragment thereof.

11. The polynucleotide according to claim 3, wherein the protein involved in trogocytosis is TC21 or RhoG.

12. The polynucleotide according to claim 3, wherein the protein located in the T cell stimulation pathway is an adaptor of the TCR/Zap70 pathway, or a fragment thereof.

13. The polynucleotide according to claim 12, wherein the adaptor of the TCR/Zap70 pathway is any one selected from the group consisting of NCK1, LAT and NEMO.

14. The polynucleotide according to claim 3, wherein the protein located in the T cell stimulation pathway is a protein located in the TNFR/TLR receptor (TRAF/NF-kB) pathway, or a fragment thereof.

15. The polynucleotide according to claim 3, wherein the protein located in the T cell stimulation pathway is a protein located in the cytokine receptor (JAK-STAT) pathway, or a fragment thereof.

16. The polynucleotide according to claim 3, wherein the protein located in the T cell stimulation pathway is a protein located in the MAP kinase pathway, or a fragment thereof.

17. The polynucleotide according to claim 16, wherein the protein located in the MAP kinase pathway is any one selected from the group consisting of GADD45α, CDC42 and HRAS.

18. The polynucleotide according to claim 3, wherein the protein involved in the inhibition of negative feedback is a protein involved in the inhibition of negative feedback of cytokine signaling pathway, or a fragment thereof.

19. The polynucleotide according to claim 18, wherein the protein involved in the inhibition of negative feedback of cytokine signaling pathway is a C-eterminal domain of a SOCS1 protein.

20. The polynucleotide according to claim 1, wherein the signaling pathway modulator has the following structural formula (I): $N ′ - X - L 1 - Y - C ′$ wherein, in the structural formula (I),
N' is an N-terminal of a fusion protein,
C' is a C-terminal of a fusion protein,
L1 is a self-cleavage peptide, and
each of X and Y may be any one selected from the group consisting of a) a protein located in the immunosuppressive signaling pathway or a fragment thereof; b) an immunophilin or a fragment thereof; c) a protein involved in the antigen loss-mediated relapse or a fragment thereof; d) a protein located in the T cell stimulation pathway or a fragment thereof; and e) a protein involved in the inhibition of negative feedback, or a fragment thereof.

21. The polynucleotide according to claim 1, wherein the signaling pathway modulator has the following structural formula (II): $N ′ - X - L 1 - Y - L 2 - Z - C ′$ wherein, in the structural formula (II),
N' is an N-terminal of a fusion protein,
C' is a C-terminal of a fusion protein,
L1 is a self-cleavage peptide, and
each of X, Y and Z may be any one selected from the group consisting of a) a protein located in the immunosuppressive signaling pathway, or a fragment thereof; b) an immunophilin or a fragment thereof; c) a protein involved in the antigen loss-mediated relapse, or a fragment thereof; d) a protein located in the T cell stimulation pathway, or a fragment thereof; and e) a protein involved in the inhibition of negative feedback, or a fragment thereof.

22. The polynucleotide according to claim 1, wherein the signaling pathway modulator includes a FKBP12 protein or a fragment thereof, cyclophilin A and an N-terminal SH2 domain of a SHP2 protein or a fragment thereof.

23. The polynucleotide according to claim 1, wherein the antigen-binding domain specifically binds to any one antigen selected from the group consisting of alpha folate receptor, 5T4, αvβ6 integrin, BCMA, B7-H3, B7-H6, CAIX, CD16, CD19, CD20, CD22, CD30, CD33, CD44, CD44v6, CD44v7/8, CD70, CD79a, CD79b, CD123, CD138, CD171, CEA, CSPG4, EGFR family including EGFR, and ErbB2 (HER2), EGFRvlll, EGP2, EGP40, EPCAM, EphA2, EpCAM, FAP, fetal AchR, FRα, GD2, GD3, glypican-3 (GPC3), HLA-A1+MAGE1, HLA-A2+MAGE1, HLA-A3+MAGE1, HLA-A1+NY-ESO-1, HLA-A2+NY-ESO-1, HLA-A3+NY-ESO-1, IL-11Rα, IL-13Rα2, lambda, Lewis-Y, kappa, mesothelin, Muc1, Muc16, NCAM, NKG2D ligand, NY-ESO-1, PRAME, PSCA, PSMA, ROR1, SSX, survivin, TAG72, TEMs, VEGFR2, and WT-1.

24. The polynucleotide according to claim 1, wherein the antigen-binding domain specifically binds to CD19.

25. The polynucleotide according to claim 1, wherein the antigen binding domain consists of the amino acid sequence represented by SEQ ID NO: 3.

26. The polynucleotide according to claim 1, wherein the transmembrane domain is derived from any one selected from the group consisting of T-cell receptor, CD3δ, CD3ε, CD3γ, CD3ζ, CD4, CD5, CD8α, CD9, CD16, CD22, CD27, CD28, CD33, CD37, CD45, CD64, CD80, CD86, CD134, CD137, CD152, CD154, AMN, and PD-1.

27. The polynucleotide according to claim 1, wherein the transmembrane domain is derived from CD8α.

28. The polynucleotide according to claim 1, wherein the transmembrane domain consists of the amino acid sequence represented by SEQ ID NO: 5.

29. The polynucleotide according to claim 1, wherein the intracellular signaling domain includes a co-stimulatory domain and a primary signaling domain.

30. The polynucleotide according to claim 29, wherein the co-stimulatory domain is derived from at least one molecule selected from the group consisting of TLR1, TLR2, TLR3, TLR4, TLR5, TLR6, TLR7, TLR8, TLR9, TLR10, CARD11, CD2, CD7, CD27, CD28, CD30, CD40, CD54 (ICAM), CD83, CD134 (OX40), CD137 (4-1BB), CD278 (ICOS), DAP10, LAT, NKD2C, SLP76, TRIM, and ZAP70.

31. The polynucleotide according to claim 24, wherein the co-stimulatory domain is derived from CD137 (4-1BB).

32. The polynucleotide according to claim 29, wherein the co-stimulatory domain consists of the amino acid sequence represented by SEQ ID NO: 7.

33. The polynucleotide according to claim 29, wherein the primary signaling domain is derived from FcRγ, FcRβ, CD3γ, CD3δ, CD3ε, CD3ζ, CD22, CD79a, CD79b, or CD66d.

34. The polynucleotide according to claim 33, wherein the primary signaling domain is derived from CD3ζ.

35. The polynucleotide according to claim 29, wherein the primary signaling domain consists of the amino acid sequence represented by SEQ ID NO: 9.

36. The polynucleotide according to claim 1, wherein the self-cleavage peptide is derived from P2A, E2A, F2A, or T2A.

37. The polynucleotide according to claim 1, wherein the self-cleavage peptide is P2A.

38. An expression vector comprising the polynucleotide of any one of claims 1 to 37.

39. The vector according to claim 38, wherein the vector further comprises a sequence encoding a signal peptide.

40. The vector according to claim 39, wherein the sequence encoding a signal peptide is inserted prior to a sequence encoding an antigen binding domain.

41. A virus comprising the polynucleotide of any one of claims 1 to 37.

42. An immune cell into which the polynucleotide of any one of claims 1 to 37 is introduced.

43. The immune cell according to claim 42, wherein the immune cell is a T cell or a natural killer cell.

44. A polynucleotide including the following (i) to (v):
(i) a polynucleotide encoding an antigen binding domain;
(ii) a polynucleotide encoding a transmembrane domain;
(iii) a polynucleotide encoding an intracellular signaling domain including at least one co-stimulatory domain;
(iv) a polynucleotide encoding an IRES (Internal Ribosome Entry Site); and
(v) a polynucleotide encoding a signaling pathway modulator(s).

45. An expression vector comprising the polynucleotide of claim 44.

46. A virus comprising the polynucleotide of claim 44.

47. An immune cell into which the polynucleotide of claim 44 is introduced.

48. A polynucleotide encoding a fusion protein that comprises the following (i) to (iv):
(i) an antigen binding domain;
(ii) a transmembrane domain;
(iii) an intracellular signaling domain including at least one co-stimulatory domain; and
(iv) a signaling pathway modulator(s).

49. The polynucleotide according to claim 48, wherein a spacer is further comprised between (i) the antigen binding domain and (ii) the transmembrane domain.

50. The polynucleotide according to claim 48, wherein the signaling pathway modulator is a protein located in the T cell stimulation pathway.

51. The polynucleotide according to claim 50, wherein the protein located in the T cell stimulation pathway is a protein located in the TNFR/TLR receptor pathway, or a fragment thereof.

52. The polynucleotide according to claim 51, wherein the protein located in the TNFR/TLR receptor pathway is a TLR4 intracellular domain.

53. The polynucleotide according to claim 50, wherein the protein located in the T cell stimulation pathway is a protein located in the cytokine receptor (JAK-STAT) pathway, or a fragment thereof.

54. The polynucleotide according to claim 51, wherein the protein located in the cytokine receptor (JAK-STAT) pathway is yc.

55. An expression vector comprising the polynucleotide of any one of claims 48 to 54.

56. The vector according to claim 55, further comprising a sequence encoding a signal peptide.

57. The vector according to claim 54, wherein the sequence encoding a signal peptide is inserted prior to a sequence encoding an antigen binding domain.

58. A virus comprising the polynucleotide of any one of claims 48 to 54.

59. An immune cell into which the polynucleotide of any one of claims 48 to 54 is introduced.

60. The immune cell according to claim 59, wherein the immune cell is a T cell or a natural killer cell.

61. A transformed immune cell, **characterized in that** the transformed immune cell expresses a signaling pathway modulator(s) that is externally introduced.

62. The transformed immune cell according to claim 61, wherein the immune cell is a T cell or an NK cell.

63. The transformed immune cell according to claim 61, wherein the immune cell is a CAR-T cell or a TCR T cell.

64. The transformed immune cell according to claim 61, wherein the signaling pathway modulator is at least one selected from the group consisting of a) a protein located in the immunosuppressive signaling pathway, b) a protein involved in the antigen loss-mediated relapse, c) a protein located in the T cell stimulation pathway, d) a protein involved in the inhibition of negative feedback, and e) a combination thereof.

65. The transformed immune cell according to claim 64, wherein the signaling pathway modulator is any one selected from the group consisting of a FKBP12 protein or a fragment thereof; a MH2 domain of at C terminal of a SMAD4 protein or a fragment thereof; N-SKI or a fragment thereof; cyclophilin A (CYPA) or a fragment thereof; a SH2 domain at N-terminal of a SHP-1 protein or a fragment thereof; a SH2 domain at N-terminal of a SHP-2 protein or a fragment thereof; TC21 or a fragment thereof; RhoG or a fragment thereof; NCK1 or a fragment thereof; LAT or a fragment thereof; NEMO or a fragment thereof; a TLR4 cellular domain or a fragment thereof; GADD45a or a fragment thereof; CDC42 or a fragment thereof; HRAS or a fragment thereof; and a C-terminal domain of SOCS1 or a fragment thereof.

66. The transformed immune cell according to claim 63, **characterized in that** the CAR-T cell expresses a fusion protein comprising (i) an antigen binding domain; (ii) a transmembrane domain; and (iii) an intracellular signaling domain comprising at least one co-stimulatory domain.

67. The transformed immune cell according to claim 61, wherein the signaling pathway modulator operates in the cytoplasm.

68. A pharmaceutical composition for treating or preventing cancer, comprising the transformed immune cell of claim 42 or the transformed immune cell of claim 61 as an active ingredient.

69. The pharmaceutical composition according to claim 68, wherein the cancer is any one selected from the group consisting of gastric cancer, liver cancer, lung cancer, colorectal cancer, breast cancer, prostate cancer, ovarian cancer, pancreatic cancer, cervical cancer, thyroid cancer, laryngeal cancer, leukemia, acute myeloid leukemia, brain tumor, neuroblastoma, retinoblastoma, head and neck cancer, salivary gland cancer, lymphoma, kidney cancer, melanoma, multiple myeloma, brain cancer, osteosarcoma, glioblastoma, IgG-opsonized tumor, lymphoma, neuroma, mesothelioma, and esophageal cancer.

70. A method for treating or preventing cancer, comprising:
administering the transformed immune cell of claim 42 or the transformed immune cell of claim 61 to a subject.

71. Use of the transformed immune cell of claim 42 or the transformed immune cell of claim 61 for the treatment of cancer.
